# EUROPEAN PATENT APPLICATION

(11) **EP 4 678 641 A1**
(43) Date of publication of application: **14.01.2026**
(21) Application number: 24741373.5
(22) Date of filing: 12.01.2024
(51) Int. Cl.: C07D 471/04, A61K 31/445, A61K 31/47, A61K 31/519, A61P 35/00, A61P 35/02, A61P 35/04

(54) **QUINOLINONE COMPOUND AND NAPHTHYRIDINONE COMPOUND AND USE THEREOF**

(30) Priority: 13.01.2023 CN 202310062611; 27.07.2023 CN 202310930384
(71) Applicant: Shanghai Helioson Pharmaceutical Co., Ltd., Shanghai 201114 (CN)
(72) Inventor: ZHAO, Liwen, Shanghai 201114 (CN); WU, Jinhua, Shanghai 201114 (CN); ZHOU, Gang, Shanghai 201114 (CN)
(74) Representative: De Clercq & Partners
(86) International application number: PCT/CN2024/072014
(87) International publication number: WO 2024/149378

(57) **Abstract**

The present invention belongs to the technical field of bio-medicines, and particularly relates to a quinolinone compound and a naphthyridinone compound as represented by formula (I) and the use thereof. The quinolinone compound and the naphthyridinone compound can be used as a molecular glue capable of selectively inducing the formation of the PDE3A and SLFN12 complex and the apoptosis of tumor cells, and can be used for treating cancers.

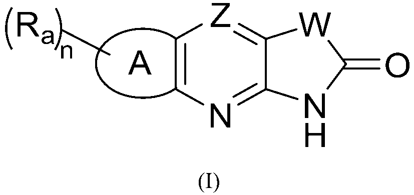

## Description

### FIELD OF TECHNOLOGY

The present application relates to the technical field of bio-medicines, and particularly relates to a molecular glue capable of selectively inducing the formation of a complex of type 3A phosphodiesterase (PDE3A) and schlafen 12 (SLFN12) and the apoptosis of tumor cells, a preparation method therefor, and use thereof in tumor treatment.

### BACKGROUND

Phosphodiesterase (PDE) is a superfamily enzyme system that can mediate the hydrolysis of second messengers, such as cyclic adenosine monophosphate (cAMP) and cyclic guanosine monophosphate (cGMP) within cells (Chen J, Liu N, Huang YP, et al. Nature Comm 2021; 12: 6204), and regulate the intracellular concentrations of cAMP and cGMP. Since second messenger signaling pathways mediated by cAMP and cGMP are involved in regulating various cellular functions, PDE can affect intracellular signaling cascade reactions, and conduct and regulate different physiological processes, such as cell proliferation and apoptosis, inflammatory responses, contraction of cardiomyocytes and smooth muscle cells, permeability of endothelial cells, immune responses, etc., by regulating the activities of cAMP and cGMP (Fertig BA and Baillie GS. J Cardiovasc Dev Dis 2018; 5:8; Nourian YH, Salimian J, Ahmadi A, et al. Biochem Biophys Rep 2023; 34: 101438). According to its hydrolysis specificity for cAMP or cGMP, PDE can be divided into 11 families, wherein PDE4, 7, and 8 are specific to cAMP, PDE5, 6, and 9 are specific to cGMP, while PDE1, 2, 3, 10, and 11 can simultaneously hydrolyze cAMP and cGMP (Ahmad F, Murata T, Simizu K, et al. Oral Dis 2015; 21: e25-e50; Barone I, Giordano C, Bonofiglio D, et al. Oncotarget 2017; 8: 99179-99202).

PDE3 enzyme has two subtypes, including PDE3A and PDE3B (Begum N, Shen WX, Manganiello V. Curr Opin Pharmacol 2011; 11: 725-9), which are respectively located on chromosomes 11 and 12, where PDE3A is mainly distributed in the heart, vascular smooth muscles, platelets, and oocytes, and can effectively regulate the contraction of cardiac muscles and smooth muscles, the aggregation of platelets, and the maturation of oocytes, etc. Although a plurality of PDE3 inhibitors have been used for treating cardiovascular diseases, studies have found that long-term inhibition of the enzymatic activity of PDE3A may cause toxic side effects, such as sudden death of patients with palpitation and heart disease, reduced generation of platelets, and affected maturation of oocytes (Ai YW, He HB, Chen PH, et al. Nature Comm 2020; 11: 3236). Moreover, current PDE3A inhibitors mainly target enzymatic catalytic domains of cAMP/cGMP binding sites. Due to the high homology of the catalytic domains of PDE family, PDE3A inhibitors have different selectivities and greater toxic side effects. For example, the traditional inhibitor anagrelide can simultaneously inhibit PDE3A and PDE2, and may cause many adverse reactions, such as headache, palpitation, diarrhea, and vomiting (Birgegard G, Brorkholm M, Kutti J, et al. Haematologica 2004; 89: 520-7), and milrinone may cause adverse symptoms, such as arrhythmia, angina pectoris, and hypotension. Therefore, improving the safety of PDE3A inhibitors is the top priority in the development of PDE3A drugs.

Recent studies have shown that a plurality of compounds with completely different chemical structures, such as anagrelide, DNMDP, and nauclefine, can all induce the formation of the PDE3A-SLFN12 complex in the form of molecular glues. On the one hand, the formation of the complex stabilizes the intracellular protein level of SLFN12 (under normal conditions, SLFN12 is degraded through a ubiquitination pathway, and the intracellular level is very low). On the other hand, the PDE3A-SLFN12 complex can bind to downstream ribosomal RNA (rRNA) (Li DR, Chen J, Ai YW, et al. Mol Cell 2019; 75: 1103-1116), thereby blocking the binding of SRP (signal recognition particle) to a ribosome. Since the ribosome requires SRP-assisted transport to the endoplasmic reticulum membrane for further synthesis of a membrane-secreted protein, the binding of PDE3A-SLFN12 to rRNA can lead to a decrease in the protein levels of anti-apoptotic proteins Bcl-2 and Mcl-1, thereby making them unable to inhibit the activities of Bax and Bak. The oligomerization of Bax and Bak further promotes the increase of mitochondrial membrane permeability, the release of cytochromes, and the induced apoptosis of tumor cells (Li DR, Chen J, Ai YW, et al. Mol Cell 2019; 75: 1103-1116). Meanwhile, it has also been found that nauclefine does not inhibit the hydrolytic activity of PDE3A to cAMP (Ai YW, He HB, Chen PH, et al. Nature Comm 2020; 11: 3236), and it mainly induces the apoptosis of tumor cells in the manner of the PDE3A-SLFN12 complex, thereby avoiding toxic side risks brought by inhibiting the enzymatic activity of PDE3A.

Based on a PDE3A-SLFN12-dependent apoptosis mechanism, the present application aims to develop a molecular glue compound capable of efficiently mediating the interaction between PDE3A and SLFN12, thereby promoting the apoptosis of tumor cells. The molecular glue of the present application not only has a higher pharmacological and pharmacodynamic activity to reduce risks brought by inhibiting the enzymatic activity of PDE, but also has a higher therapeutic potential in tumors expressing PDE3A and SLFN12.

### SUMMARY

To solve the above technical problems, in one aspect, the present application provides a compound represented by formula (I) below, a pharmaceutically acceptable salt thereof, a deuterated compound thereof, a stereoisomer thereof, a tautomer thereof, or a mixture thereof: 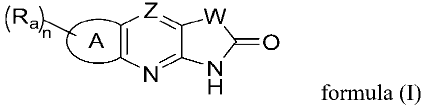 wherein
Z is selected from CR₁ or N;
W is selected from C=O, CR₂R₇, NR₃ or O;
R₁ is selected from H, halogen, hydroxyl, cyano, alkynyl, C₁-C₆ alkyl, C₁-C₆ haloalkyl, C₁-C₆ alkoxy, or C₃-C₆ cycloalkyl;
R₂ and R₇ are each independently selected from H, a deuterium atom, C₁-C₃ alkyl, or C₁-C₃ haloalkyl; or, R₂ and R₇, together with the carbon atoms to which they are attached, form C₃-C₆ cycloalkyl;
R₃ is selected from H, C₁-C₆ alkyl, C₁-C₆ haloalkyl, or C₃-C₆ cycloalkyl;
n is selected from any integer between 0-6, such as 0, 1, 2, 3, 4, 5, or 6;
Rₐ is each independently selected from H, halogen, cyano, alkynyl, optionally substituted C₁-C₆ alkoxy, C₁-C₆ haloalkyl, optionally substituted C₆-C₁₀ aryl, optionally substituted 5-10 membered heteroaryl, optionally substituted C₃-C₉ cycloalkyl, optionally substituted C₄-C₉ cycloalkenyl, or optionally substituted 3-10 membered heterocyclyl, wherein the "optionally substituted" means that the mentioned group is unsubstituted or substituted with one or more substituents independently selected from deuterium, halogen, cyano, amino, C₁-C₆ alkyl (including deuterated C₁-C₆ alkyl), C₁-C₆ haloalkyl, C₃-C₆ cycloalkyl, 3-6 membered heterocyclyl, hydroxyl, C₁-C₆ alkoxy (including deuterated C₁-C₆ alkoxy), C₁-C₆ haloalkoxy, C₆-C₁₀ aryl, 5-10 membered heteroaryl, and C₁-C₆ hydroxyalkyl; and
ring A is selected from C₆-C₁₀ aryl, 5-10 membered heteroaryl, C₄-C₁₀ carbocyclyl, or 4-10 membered heterocyclyl.

In some embodiments, Rₐ is each independently selected from H, halogen, cyano, alkynyl, optionally substituted C₁-C₆ alkoxy, C₁-C₆ haloalkyl, optionally substituted C₆-C₁₀ aryl, optionally substituted 5-10 membered heteroaryl, optionally substituted C₃-C₉ cycloalkyl, optionally substituted C₄-C₉ cycloalkenyl, or optionally substituted 3-10 membered heterocyclyl, wherein the optionally substituted means that the mentioned group is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, C₁-C₆ alkylamino, C₆-C₁₀ aryl, 5-10 membered heteroaryl, and C₁-C₆ hydroxyalkyl.

The present application provides a compound represented by formula (I) below, a pharmaceutically acceptable salt thereof, a deuterated compound thereof, a stereoisomer thereof, a tautomer thereof, or a mixture thereof: 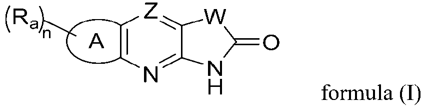 wherein
Z is selected from CR₁ or N;
W is selected from C=O, CR₂R₇, NR₃ or O;
R₁ is selected from H, halogen, hydroxyl, cyano, alkynyl, C₁-C₆ alkyl, C₁-C₆ haloalkyl, C₁-C₆ alkoxy, or C₃-C₆ cycloalkyl;
R₂ and R₇ are each independently selected from H, a deuterium atom, C₁-C₃ alkyl, or C₁-C₃ haloalkyl; or, R₂ and R₇, together with the carbon atoms to which they are attached, form C₃-C₆ cycloalkyl;
R₃ is selected from H, C₁-C₆ alkyl, C₁-C₆ haloalkyl, or C₃-C₆ cycloalkyl;
n is selected from any integer between 0-6, such as 0, 1, 2, 3, 4, 5, or 6;
Rₐ is each independently selected from H, halogen, cyano, alkynyl, optionally substituted C₁-C₆ alkoxy, C₁-C₆ haloalkyl, optionally substituted C₆-C₁₀ aryl, optionally substituted 5-10 membered heteroaryl, optionally substituted C₃-C₉ cycloalkyl, optionally substituted C₄-C₉ cycloalkenyl, or optionally substituted 3-10 membered heterocyclyl; and
ring A is selected from C₆-C₁₀ aryl, 5-10 membered heteroaryl, C₄-C₁₀ carbocyclyl, or 4-10 membered heterocyclyl.

In some preferred embodiments, Z is selected from CH; W is selected from C=O, CH₂, NR₃ or O; and R₃ is selected from H or CH₃.

In some preferred embodiments, Z is selected from N; and W is selected from C=O, NH₂, or NH.

In some more preferred embodiments, Z is selected from CH; and W is selected from NH.

In some embodiments, Z is selected from CR₁ or N, and R₁ is selected from H, halogen, hydroxyl, or C₁-C₃ alkyl. In some embodiments, Z is selected from CH or N.

In some embodiments, W is selected from C=O, CR₂R₇, NR₃ or O, R₂ and R₇ are each independently selected from H, a deuterium atom, or C₁-C₃ alkyl, and R₃ is selected from H, C₁-C₆ alkyl, or C₁-C₆ haloalkyl. In some embodiments, W is selected from C=O, CH₂, NR₃ or O, and R₃ is selected from H or C₁-C₆ alkyl (e.g., C₁-C₅ alkyl, C₁-C₄ alkyl, C₁-C₃ alkyl, or C₁-C₂ alkyl). In some embodiments, W is selected from CH₂ or NH.

In some preferred embodiments, Rₐ is each independently selected from H, halogen, cyano, optionally substituted C₁-C₃ alkoxy, C₁-C₃ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl.

In some more preferred embodiments, Rₐ is each independently selected from H, F, Cl, Br, CN, OCH₃, OCD₃, OCHF₂, OCF₃, CF₃, CHF₂, CH₂F, an optionally substituted benzene ring, or optionally substituted 5-6 membered heteroaryl, wherein the optionally substituted benzene ring or optionally substituted 5-6 membered heteroaryl is unsubstituted or substituted with one or more substituents selected from F, Cl, Br, CN, CHF₂, CF₃, CH₂F, CH₃, CD₃, OCH₃, OCHF₂, OCF₃, OCD₃, or NH₂.

In some embodiments, n is selected from any integer between 1-5 (e.g., 1, 2, 3, 4, or 5), and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl.

In some embodiments, n is 1, and Rₐ is each independently selected from optionally substituted C₆-C₁₀ aryl, optionally substituted 5-10 membered heteroaryl, optionally substituted C₃-C₆ cycloalkyl, or optionally substituted 3-10 membered heterocyclyl; or, n is any integer between 2-5 (e.g., 2, 3, 4, or 5), and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl. Preferably, n is 1, and Rₐ is each independently selected from optionally substituted C₆-C₁₀ aryl, optionally substituted 5-10 membered heteroaryl, optionally substituted C₆ cycloalkyl, or optionally substituted 6-10 membered heterocyclyl; or, n is any integer between 2-5 (e.g., 2, 3, 4, or 5), Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl, and Rₐ is not simultaneously halogen. Alternatively preferably, n is 1, and Rₐ is each independently selected from optionally substituted C₆-C₁₀ aryl, optionally substituted 5-10 membered heteroaryl, optionally substituted C₆ cycloalkyl, or optionally substituted 6-10 membered heterocyclyl; or, n is any integer between 2-5 (e.g., 2, 3, 4, or 5), wherein one Rₐ is selected from optionally substituted C₆-C₁₀ aryl, optionally substituted 5-10 membered heteroaryl, optionally substituted C₃-C₆ cycloalkyl, or optionally substituted 3-10 membered heterocyclyl, and the remaining Rₐ is selected from halogen, optionally substituted C₁-C₆ alkoxy, C₁-C₆ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl.

In some embodiments, n is selected from any integer between 1-3 (e.g., 1, 2, or 3), and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, or optionally substituted 3-10 membered heterocyclyl. In some embodiments, n is 1, and Rₐ is each independently selected from optionally substituted C₆-C₁₀ aryl, optionally substituted 5-10 membered heteroaryl, or optionally substituted 6-10 membered heterocyclyl; or, n is 2 or 3, Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, or optionally substituted 3-10 membered heterocyclyl, and Rₐ is not simultaneously halogen. Alternatively preferably, n is 1, and Rₐ is each independently selected from optionally substituted C₆-C₁₀ aryl, optionally substituted 5-10 membered heteroaryl, or optionally substituted 6-10 membered heterocyclyl; or, n is 2 or 3, wherein one Rₐ is selected from optionally substituted C₆-C₁₀ aryl, optionally substituted 5-10 membered heteroaryl, or optionally substituted 3-10 membered heterocyclyl, and the remaining Rₐ is selected from halogen, optionally substituted C₁-C₆ alkoxy, C₁-C₆ haloalkyl, optionally substituted C₆-C₁₀ aryl, optionally substituted 5-10 membered heteroaryl, or optionally substituted 3-10 membered heterocyclyl.

In some embodiments, n is selected from any integer between 1-3 (e.g., 1, 2, or 3), and Rₐ is each independently selected from halogen, optionally substituted C₁-C₆ alkoxy, C₁-C₆ haloalkyl, optionally substituted C₆-C₁₀ aryl, optionally substituted 5-8 membered heteroaryl, or optionally substituted 4-9 membered heterocyclyl. In some embodiments, n is 1, and Rₐ is each independently selected from optionally substituted C₆-C₁₀ aryl, optionally substituted 5-8 membered heteroaryl, or optionally substituted 6-9 membered heterocyclyl; or, n is 2 or 3, Rₐ is each independently selected from halogen, optionally substituted C₁-C₆ alkoxy, C₁-C₆ haloalkyl, optionally substituted C₆-C₁₀ aryl, optionally substituted 5-8 membered heteroaryl, or optionally substituted 4-9 membered heterocyclyl, and Rₐ is not simultaneously halogen. Alternatively preferably, n is 1, and Rₐ is each independently selected from optionally substituted C₆-C₁₀ aryl, optionally substituted 5-8 membered heteroaryl, or optionally substituted 6-9 membered heterocyclyl; or, n is 2 or 3, wherein one Rₐ is selected from optionally substituted C₆-C₁₀ aryl, optionally substituted 5-8 membered heteroaryl, or optionally substituted 4-9 membered heterocyclyl, and the remaining Rₐ is selected from halogen, optionally substituted C₁-C₆ alkoxy, C₁-C₆ haloalkyl, optionally substituted C₆-C₁₀ aryl, optionally substituted 5-8 membered heteroaryl, or optionally substituted 4-9 membered heterocyclyl.

In some embodiments, n is 1 or 2, and Rₐ is each independently selected from halogen, optionally substituted C₁-C₆ alkoxy, C₁-C₆ haloalkyl, optionally substituted C₆ aryl, optionally substituted 5-6 membered heteroaryl, or optionally substituted 6-8 membered heterocyclyl. Preferably, when n is 2, at least one Rₐ is not halogen, or two Rₐ are different.

In some embodiments, the optionally substituted C₁-C₆ alkoxy, aryl, heteroaryl, or heterocyclyl is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, C₁-C₆ alkylamino, C₆-C₁₀ aryl, 5-10 membered heteroaryl, and C₁-C₆ hydroxyalkyl. In some embodiments, the optionally substituted C₁-C₆ alkoxy, aryl, heteroaryl, or heterocyclyl is unsubstituted or substituted with one or more substituents independently selected from halogen, cyano, amino, C₁-C₆ alkyl, C₁-C₆ haloalkyl, C₁-C₆ alkoxy, C₁-C₆ haloalkoxy, C₃-C₆ cycloalkyl, 3-6 membered heterocyclyl, hydroxyl, C₆-C₁₀ aryl, 5-10 membered heteroaryl, and C₁-C₆ hydroxyalkyl.

In some embodiments, the optionally substituted C₁-C₆ alkoxy is unsubstituted or substituted with halogen (e.g., F, Cl, or Br), and the optionally substituted aryl, heteroaryl, or heterocyclyl is unsubstituted or substituted with one or more (e.g., 1-3 or 1-2) substituents independently selected from halogen, cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), C₁-C₆ haloalkoxy, and C₁-C₆ alkylamino. In some embodiments, the optionally substituted C₁-C₆ alkoxy is unsubstituted or substituted with halogen (e.g., F, Cl, or Br), and the optionally substituted aryl, heteroaryl, or heterocyclyl is unsubstituted or substituted with one or more (e.g., 1-3 or 1-2) substituents independently selected from halogen, cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), and C₁-C₆ haloalkoxy.

In some embodiments, n is 1 or 2, and Rₐ is each independently selected from F, Cl, Br, optionally substituted C₁-C₅ alkoxy, C₁-C₅ haloalkyl, optionally substituted C₆ aryl, optionally substituted 5-6 membered heteroaryl, or optionally substituted 6-8 membered heterocyclyl, wherein the optionally substituted C₁-C₆ alkoxy is unsubstituted or substituted with F, Cl, or Br, and the optionally substituted aryl, heteroaryl, or heterocyclyl is unsubstituted or substituted with one or more (e.g., 1-3 or 1-2) substituents independently selected from F, Cl, Br, cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), C₁-C₆ haloalkoxy, and C₁-C₆ alkylamino. In some embodiments, n is 1 or 2, and Rₐ is each independently selected from F, Cl, Br, optionally substituted C₁-C₅ alkoxy, C₁-C₅ haloalkyl, optionally substituted C₆ aryl, optionally substituted 5-6 membered heteroaryl, or optionally substituted 6-8 membered heterocyclyl, wherein the optionally substituted C₁-C₆ alkoxy is unsubstituted or substituted with F, Cl, or Br, and the optionally substituted aryl, heteroaryl, or heterocyclyl is unsubstituted or substituted with one or more (e.g., 1-3 or 1-2) substituents independently selected from F, Cl, Br, cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), and C₁-C₆ haloalkoxy.

In some embodiments, n is 1 or 2, and Rₐ is selected from F, Cl, Br, optionally substituted C₆ aryl, and optionally substituted 5-6 membered heteroaryl containing 1-2 heteroatoms (e.g., N atoms) selected from N, O, or S, wherein the optionally substituted aryl or heteroaryl is unsubstituted or substituted with one or more (e.g., 1-3 or 1-2) substituents independently selected from F, Cl, Br, cyano, amino, C₁-C₆ alkyl (including deuterated alkyl, e.g., deuterated methyl), C₁-C₆ haloalkyl, and C₁-C₆ alkoxy (including deuterated alkoxy, e.g., deuterated methoxy).

In some embodiments, n is 2, and Rₐ is F, Cl, Br, or optionally substituted C₆ aryl, wherein the optionally substituted aryl is unsubstituted or substituted with one or more (e.g., 1-3, 1-2, or 1) C₁-C₆ alkyl (including deuterated alkyl, e.g., deuterated methyl).

In some preferred embodiments, the ring A is selected from a benzene ring, a naphthalene ring, C₄-C₆ carbocyclyl, or 5-6 membered heterocyclyl.

In some more preferred embodiments, the ring A is selected from 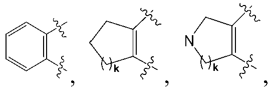 or 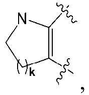 wherein k is selected from 1 or 2.

In some more preferred embodiments, the ring A is selected from 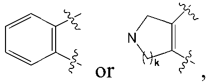 wherein k is selected from 1 or 2.

In some more preferred embodiments, the ring A is selected from

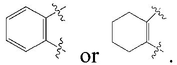

In some embodiments, the ring A is selected from C₆-C₁₀ aryl, 6-10 membered heteroaryl, C₅-C₈ carbocyclyl, or 5-8 membered heterocyclyl.

In some embodiments, the ring A is selected from C₆ aryl, 6-8 membered heteroaryl containing 1-3 (e.g., 1-2) heteroatoms selected from N or O or S, C₅-C₇ carbocyclyl, or 5-7 membered heterocyclyl containing 1-3 (e.g., 1-2) heteroatoms selected from N or O or S.

In some embodiments, the ring A is selected from C₆ aryl, 6-membered heterocyclyl containing 1-2 heteroatoms selected from N or O or S (e.g., containing 1 N atom), or 6-membered heterocyclyl containing 1-2 heteroatoms selected from N or O or S (e.g., containing 1 N atom). In some embodiments, the ring A is C₆ aryl.

In some embodiments, in the above compound of formula (I), the pharmaceutically acceptable salt thereof, the deuterated compound thereof, the stereoisomer thereof, the tautomer thereof, or the mixture thereof,
Z is selected from CR₁ or N, and R₁ is selected from H, halogen, hydroxyl, or C₁-C₃ alkyl;
W is selected from C=O, CR₂R₇, NR₃ or O, R₂ and R₇ are each independently selected from H, a deuterium atom, or C₁-C₃ alkyl, and R₃ is selected from H, C₁-C₆ alkyl, or C₁-C₆ haloalkyl;
n is selected from any integer between 1-5 (e.g., 1, 2, 3, 4, or 5), and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl, wherein the optionally substituted C₁-C₆ alkoxy, aryl, heteroaryl, or heterocyclyl is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, C₁-C₆ alkylamino, C₆-C₁₀ aryl, 5-10 membered heteroaryl, and C₁-C₆ hydroxyalkyl; preferably, n is selected from any integer between 1-3 (e.g., 1, 2, or 3), and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, or optionally substituted 3-10 membered heterocyclyl, wherein the optionally substituted C₁-C₆ alkoxy is unsubstituted or substituted with halogen (e.g., F, Cl, or Br), and the optionally substituted aryl, heteroaryl, or heterocyclyl is unsubstituted or substituted with one or more (e.g., 1-3 or 1-2) substituents independently selected from halogen, cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), C₁-C₆ haloalkoxy, and C₁-C₆ alkylamino; and preferably, n is 1, and Rₐ is selected from optionally substituted C₆-C₁₀ aryl, optionally substituted 5-10 membered heteroaryl, optionally substituted C₃-C₆ cycloalkyl, or optionally substituted 6-10 membered heterocyclyl, or n is any integer between 2-5, wherein one Rₐ is selected from optionally substituted C₆-C₁₀ aryl, optionally substituted 5-10 membered heteroaryl, optionally substituted C₃-C₆ cycloalkyl, or optionally substituted 3-10 membered heterocyclyl; and
the ring A is selected from C₆-C₁₀ aryl, 6-10 membered heteroaryl, C₅-C₈ carbocyclyl, or 5-8 membered heterocyclyl.

In some embodiments, in the above compound of formula (I), the pharmaceutically acceptable salt thereof, the deuterated compound thereof, the stereoisomer thereof, the tautomer thereof, or the mixture thereof,
Z is selected from CH or N;
W is selected from C=O, CH₂, NR₃ or O, and R₃ is selected from H or C₁-C₆ alkyl (e.g., C₁-C₅ alkyl, C₁-C₄ alkyl, C₁-C₃ alkyl, or C₁-C₂ alkyl);
n is 1 or 2, and Rₐ is each independently selected from F, Cl, Br, optionally substituted C₁-C₅ alkoxy, C₁-C₅ haloalkyl, optionally substituted C₆ aryl, optionally substituted 5-6 membered heteroaryl, or optionally substituted 6-8 membered heterocyclyl, wherein the optionally substituted C₁-C₆ alkoxy is unsubstituted or substituted with F, Cl, or Br, and the optionally substituted aryl, heteroaryl, or heterocyclyl is unsubstituted or substituted with one or more (e.g., 1-3 or 1-2) substituents independently selected from F, Cl, Br, cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), C₁-C₆ haloalkoxy, and C₁-C₆ alkylamino; and preferably, when n is 2, at least one Rₐ is not halogen; and
the ring A is selected from C₆ aryl, 6-8 membered heteroaryl containing 1-3 (e.g., 1-2) heteroatoms selected from N or O or S, C₅-C₇ carbocyclyl, or 5-7 membered heterocyclyl containing 1-3 (e.g., 1-2) heteroatoms selected from N or O or S; and preferably, the ring A is selected from C₆ aryl, 6-membered heteroaryl containing 1-3 (e.g., 1-2) heteroatoms selected from N or O or S, C₆ carbocyclyl, or 6-membered heterocyclyl containing 1-3 (e.g., 1-2) heteroatoms selected from N or O or S.

In some embodiments, for the above compound of formula (I), the pharmaceutically acceptable salt thereof, the deuterated compound thereof, the stereoisomer thereof, the tautomer thereof, or the mixture thereof, the compound of formula (I) has a structure of formula (II) or formula (II') or formula (II"): 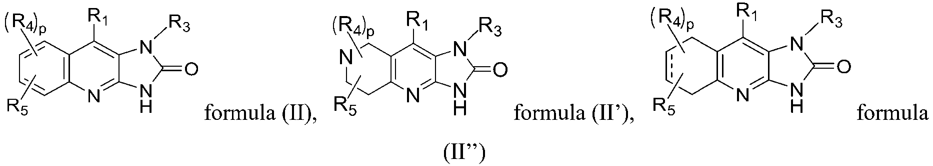 wherein
p is selected from 0, 1, 2, 3, or 4;
R₁ is selected from H, halogen, hydroxyl, cyano, alkynyl, C₁-C₆ alkyl, C₁-C₆ haloalkyl, C₁-C₆ alkoxy, or C₃-C₆ cycloalkyl;
R₃ is selected from H, C₁-C₆ alkyl, C₁-C₆ haloalkyl, or C₃-C₆ cycloalkyl;
R₄ and R₅ are each independently selected from H, halogen, cyano, alkynyl, optionally substituted C₁-C₃ alkoxy, C₁-C₃ haloalkyl, optionally substituted C₆-C₁₀ aryl, optionally substituted 5-10 membered heteroaryl, optionally substituted C₃-C₆ cycloalkyl, optionally substituted C₄-C₆ cycloalkenyl, or optionally substituted 3-10 membered heterocyclyl, wherein the "optionally substituted" means that the mentioned group is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, 6-10 membered aryl (namely, C₆-C₁₀ aryl), 5-10 membered heteroaryl, and C₁-C₆ hydroxyalkyl; and
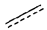 represents a single bond or a double bond (preferably a single bond).

In some alternative embodiments, in the above general formula compound of formula (II) or formula (II') or formula (II"), when p is not 0, one of R₄ and R₅, together with the atoms to which they are attached respectively, are connected to form optionally substituted 4-12 membered heterocyclyl, optionally substituted C₃-C₁₂ cycloalkyl, or optionally substituted 5-12 membered heteroaryl, such as optionally substituted furyl, optionally substituted tetrahydrofuryl, optionally substituted azetidinyl, optionally substituted pyrrolyl, optionally substituted pyrrolidinyl, and optionally substituted pyrazolyl.

In some preferred embodiments, in the above general formula compound of formula (II) or formula (II') or formula (II"), p is selected from 0, 1, 2, or 3; each R₄ is respectively and independently selected from H, halogen, cyano, C₁-C₃ haloalkyl, or optionally substituted C₁-C₃ alkoxy; and R₅ is selected from optionally substituted C₆-C₁₀ aryl, optionally substituted 5-10 membered heteroaryl, cyano, optionally substituted C₃-C₆ cycloalkyl, and optionally substituted 3-10 membered heterocyclyl, wherein the "optionally substituted" means that the mentioned group is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, 6-10 membered aryl, 5-10 membered heteroaryl, and C₁-C₆ hydroxyalkyl.

In some more preferred embodiments, in the above general formula compound of formula (II) or formula (II') or formula (II"), p is selected from 0, 1, 2, or 3;
each R₄ is respectively and independently selected from H, halogen, cyano, C₁-C₃ haloalkyl, or optionally substituted C₁-C₃ alkoxy, preferably, the optionally substituted C₁-C₃ alkoxy is selected from methoxy, ethoxy, -OCH₂F, -OCHF₂, -OCF₃, or -OCH₂CF₃; and
R₅ is selected from optionally substituted C₆-C₁₀ aryl, optionally substituted 5-10 membered heteroaryl, cyano, optionally substituted C₃-C₆ cycloalkyl, and optionally substituted 3-10 membered heterocyclyl, wherein the "optionally substituted" means that the mentioned group is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, 6-10 membered aryl, 5-10 membered heteroaryl, and C₁-C₆ hydroxyalkyl.

In some more preferred embodiments, in the above general formula compound of formula (II) or formula (II') or formula (II"), R₅ is selected from an optionally substituted benzene ring or optionally substituted 5-6 membered heteroaryl, wherein the optionally substituted benzene ring or optionally substituted 5-6 membered heteroaryl is unsubstituted or optionally substituted with one or more substituents selected from F, Cl, Br, CN, CHF₂, CF₃, CH₂F, CH₃, CD₃, OCH₃, OCHF₂, OCF₃, OCD₃, or NH₂.

In some preferred embodiments, in the above general formula compound of formula (II) or formula (II') or formula (II"), R₅ is not

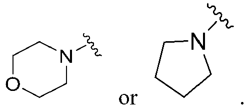

In some preferred embodiments, in the above formula general compound of formula (II) or formula (II') or formula (II"), R₅ is preferably selected from cyano, 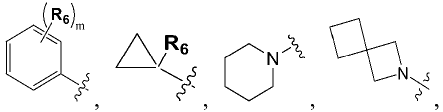 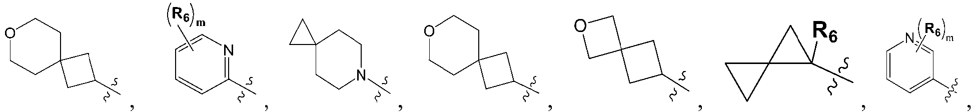 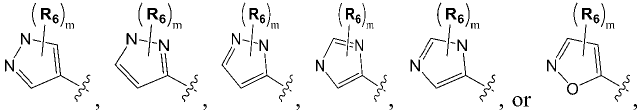 wherein m is selected from 0, 1, 2, or 3; R₆ is each independently selected from H, C₁-C₃ alkyl, C₁-C₃ haloalkyl, halogen, cyano, amino, or optionally substituted C₁-C₃ alkoxy; and the optionally substituted C₁-C₃ alkoxy is selected from -OCH₃, - OCD₃, -OCHD₂, -OCH₂D, ethoxy, -OCH₂F, -OCHF₂, -OCF₃, or -OCH₂CF₃; more preferably, R₅ is preferably selected from

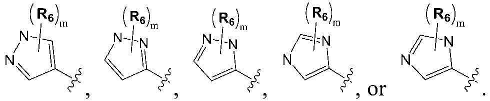

In some preferred embodiments, in the above general formula compound of formula (II) or formula (II') or formula (II"), when R₅ is selected from 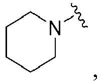 R₄ is not H.

In some embodiments, the present application further provides a compound represented by formula (III) below, a pharmaceutically acceptable salt thereof, a deuterated compound thereof, a stereoisomer thereof, a tautomer thereof, or a mixture thereof: 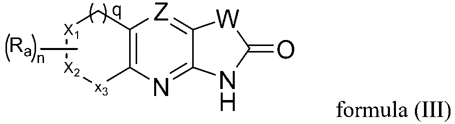 wherein q is selected from 0 or 1; X₁, X₂, and X₃ are each independently selected from C or N; and Z, W, n, and Rₐ are defined as any one of the foregoings.

Any of the above embodiments may be optionally combined to form new technical solutions.

In some embodiments, the present application further provides a compound represented by formula (IV) below, a pharmaceutically acceptable salt thereof, a deuterated compound thereof, a stereoisomer thereof, a tautomer thereof, or a mixture thereof: 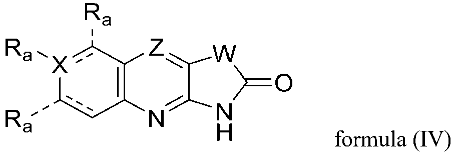 wherein X is selected from C or N, Z, W, and Rₐ are defined as above, 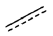 represents a single bond or a double bond, and - - - connected to Rₐ represents that the bond is present or absent.

In some preferred embodiments, X is selected from C, CH or N (e.g., X is C or CH);
Rₐ is each independently selected from halogen (e.g., F, Cl, or Br), substituted or unsubstituted C₁-C₆ alkoxy, C₁-C₆ haloalkyl, substituted or unsubstituted C₆-C₁₀ aryl (e.g., substituted or unsubstituted C₆ aryl), substituted or unsubstituted 5-8 membered heteroaryl (e.g., substituted or unsubstituted 5-6 membered heteroaryl), and substituted or unsubstituted 5-10 membered heterocyclyl (e.g., substituted or unsubstituted 6-8 membered heterocyclyl), wherein the C₁-C₆ alkoxy may be substituted with halogen (e.g., F, Cl, or Br), the aryl, heteroaryl, or heterocyclyl may be substituted with one or more (e.g., 1-3 or 1-2) substituents independently selected from deuterium, halogen (e.g., F, Cl, or Br), cyano, amino, C₁-C₆ alkyl (including deuterated alkyl, e.g., deuterated methyl), C₁-C₆ haloalkyl, C₁-C₆ alkoxy (including deuterated alkoxy, e.g., deuterated methoxy), C₁-C₆ haloalkoxy, and C₁-C₆ alkylamino, and the heteroaryl or heterocyclyl contains 1-3 (e.g., 1-2 or 1) heteroatoms selected from N, O, or S; preferably, Rₐ is selected from F, Cl, Br, substituted or unsubstituted C₁-C₃ alkoxy, C₁-C₃ haloalkyl, substituted or unsubstituted C₆ aryl, substituted or unsubstituted 5-6 membered heteroaryl, and substituted or unsubstituted 6-8 membered heterocyclyl, wherein the C₁-C₆ alkoxy may be substituted with F, Cl, or Br, the aryl, heteroaryl, or heterocyclyl may be substituted with 1-3 (e.g., 1-2) substituents independently selected from F, Cl, Br, cyano, amino, C₁-C₆ alkyl (including deuterated alkyl, e.g., deuterated methyl), C₁-C₆ haloalkyl (e.g., C₁-C₆ fluoroalkyl, C₁-C₆ chloroalkyl, or C₁-C₆ bromoalkyl), C₁-C₆ alkoxy (including deuterated alkoxy, e.g., deuterated methoxy), C₁-C₆ haloalkoxy (e.g., C₁-C₆ fluoroalkoxy, C₁-C₆ chloroalkoxy, or C₁-C₆ bromoalkoxy), and C₁-C₆ alkylamino, and the heteroaryl or heterocyclyl contains 1-3 (e.g., 1-2) heteroatoms selected from N, O, or S (e.g., contains 1-2 heteroatoms selected from N or O); alternatively preferably, Rₐ is selected from substituted or unsubstituted C₆ aryl and substituted or unsubstituted 5-6 membered heteroaryl, wherein the aryl or heteroaryl may be substituted with 1-3 (e.g., 1-2) substituents independently selected from F, Cl, Br, C₁-C₆ alkyl (including deuterated alkyl, e.g., deuterated methyl), C₁-C₆ haloalkyl (e.g., C₁-C₆ fluoroalkyl, C₁-C₆ chloroalkyl, or C₁-C₆ bromoalkyl), and the heteroaryl or heterocyclyl contains 1-3 (e.g., 1-2) heteroatoms selected from N, O, or S (e.g., contains 1-2 heteroatoms selected from N or O); and preferably, when there are two or more Rₐ, at least one of Rₐ is not halogen;
Z is selected from CH or N, W is selected from C=O, CH₂, NR₃ or O, and R₃ is selected from H or C₁-C₆ alkyl (e.g., C₁-C₆ alkyl, C₁-C₄ alkyl, C₁-C₃ alkyl, or C₁-C₂ alkyl); for example, Z is CH, and W is NH;
represents a single bond or a double bond; and - - - connected to Rₐ represents that the bond may be present or absent (preferably absent).

In some preferred embodiments, the present application further provides a compound represented by formula (IV-1) below, a pharmaceutically acceptable salt thereof, a deuterated compound thereof, a stereoisomer thereof, a tautomer thereof, or a mixture thereof: 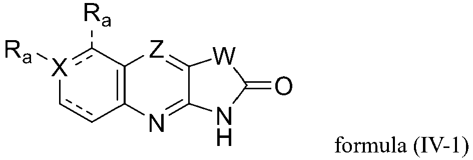 wherein X, Z, W, Rₐ, 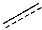 , and - - - are defined as above.

In some preferred embodiments, the present application further provides a compound represented by formula (IV-2) below, a pharmaceutically acceptable salt thereof, a deuterated compound thereof, a stereoisomer thereof, a tautomer thereof, or a mixture thereof: 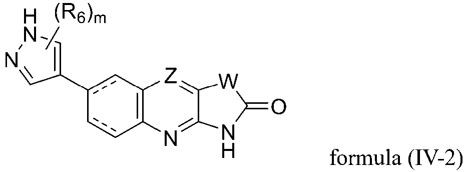 wherein Z, W, and 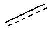 are defined as above, m is selected from 0, 1, 2, or 3, and R₆ is each independently selected from C₁-C₃ alkyl, C₁-C₃ haloalkyl, halogen (e.g., fluorine, chlorine, or bromine), amino, or C₁-C₃ alkoxy, wherein the optionally substituted C₁-C₃ alkoxy is selected from -OCH₃, -OCD₃, - OCHD₂, -OCH₂D, ethoxy, -OCH₂F, -OCHF₂, -OCF₃, or -OCH₂CF₃; preferably, m is 2, and R₆ may be the same or different and is each independently selected from C₁-C₃ alkyl, C₁-C₃ haloalkyl (e.g., C₁-C₃ fluoroalkyl, C₁-C₃ chloroalkyl, or C₁-C₃ bromoalkyl) or halogen (e.g., fluorine, chlorine, or bromine); and more preferably, m is 2, and R₆ is different and is each independently selected from C₁-C₃ haloalkyl (e.g., C₁-C₃ fluoroalkyl, C₁-C₃ chloroalkyl, or C₁-C₃ bromoalkyl), fluorine, chlorine, and bromine. As commonly understood in the art, structural unit 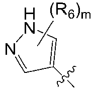 represents that R₆ may be optionally substituted at any position on the pyrazole ring, and for example, the structural unit may be 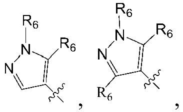 etc.

In some embodiments, for the compound, the pharmaceutically acceptable salt thereof, the deuterated compound thereof, the stereoisomer thereof, the tautomer thereof, or the mixture thereof as described in any one of the foregoings, the compound is selected from, but is not limited to:

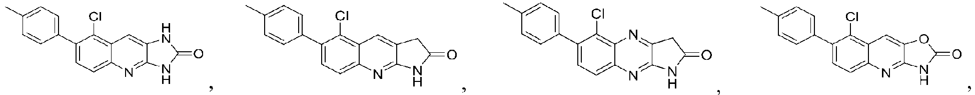

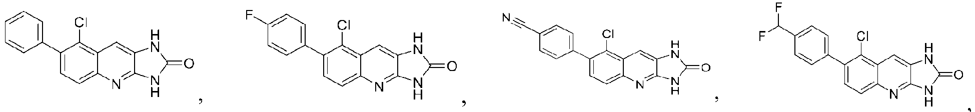

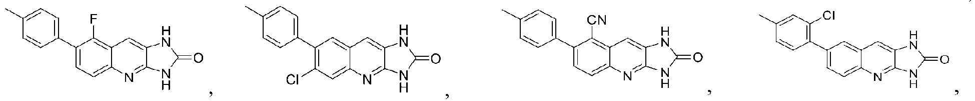

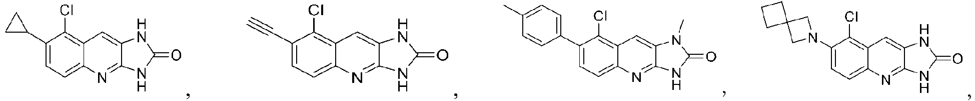

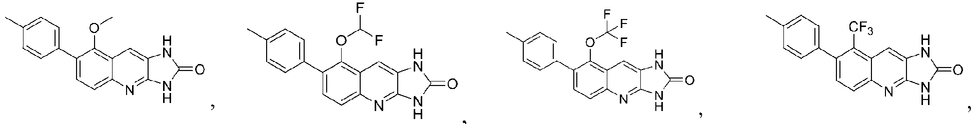

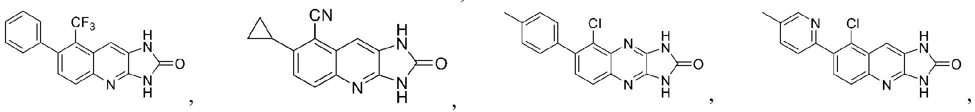

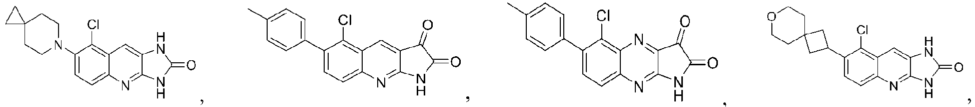

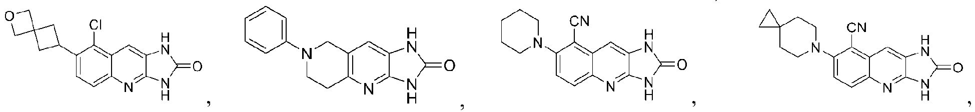

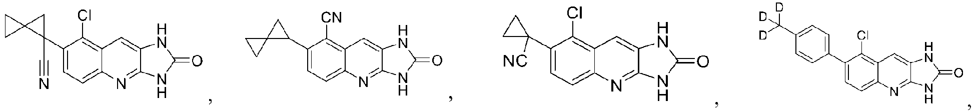

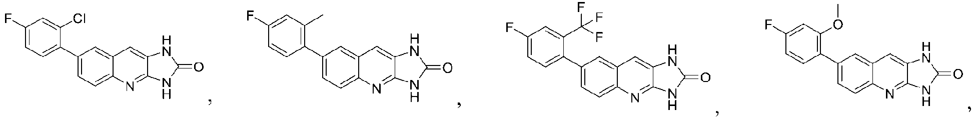

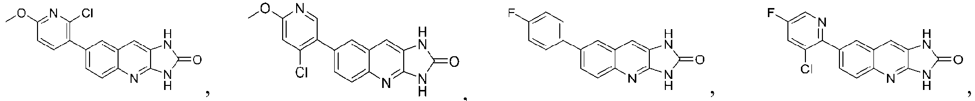

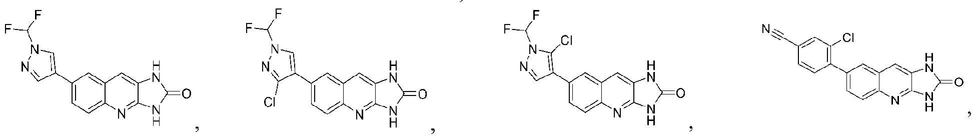

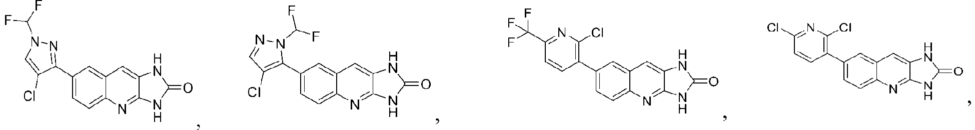

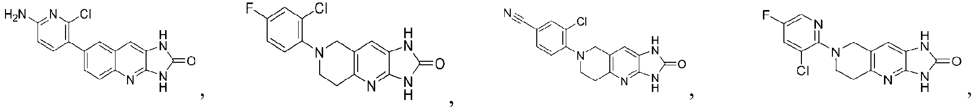

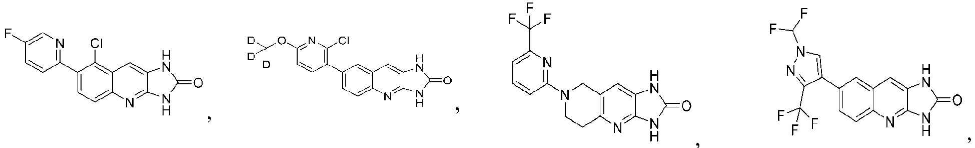

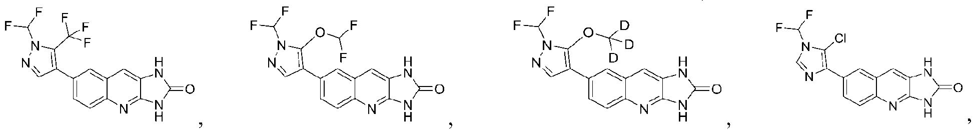

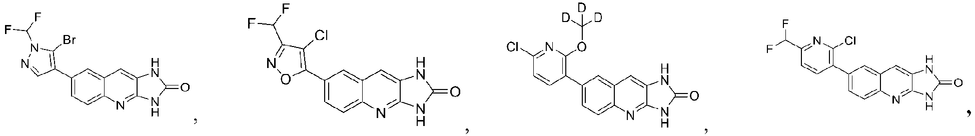

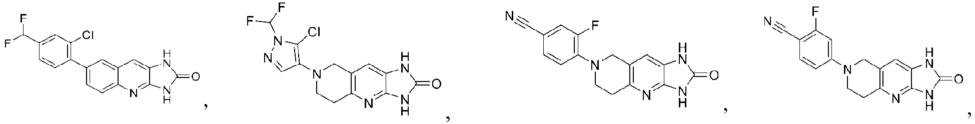

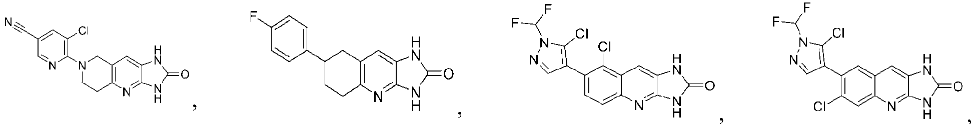

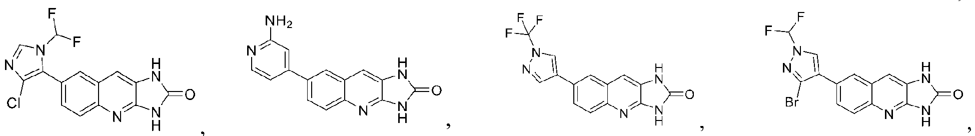

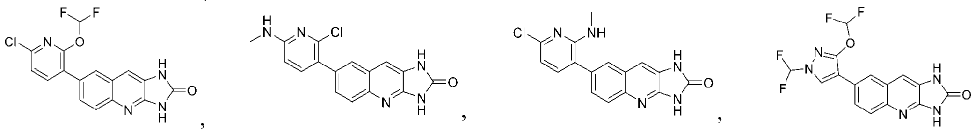

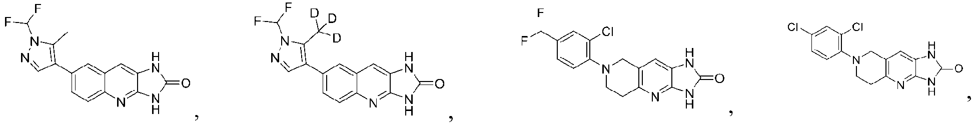

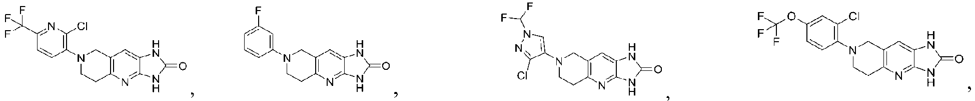

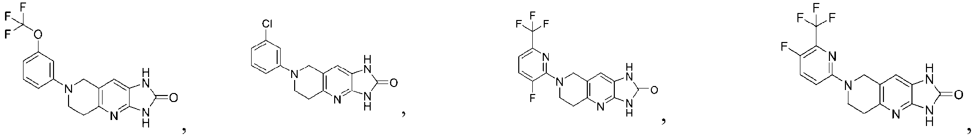

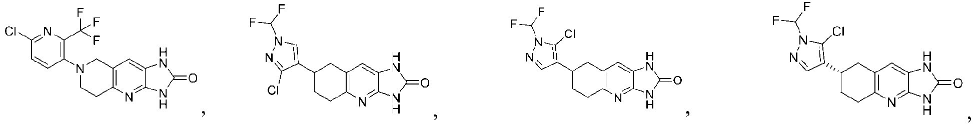

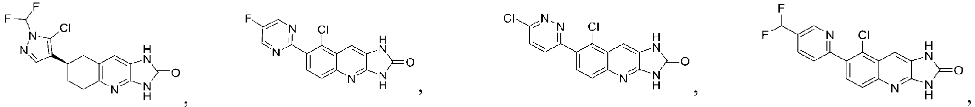

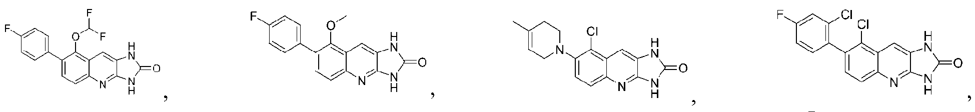

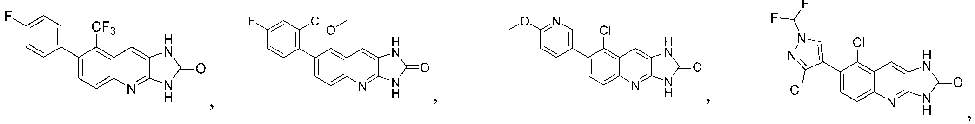

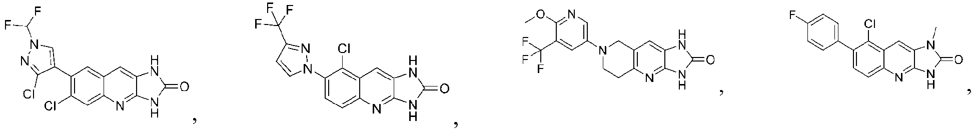

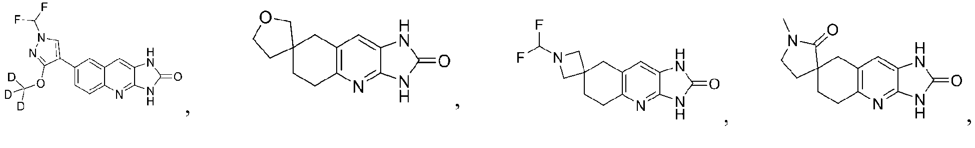

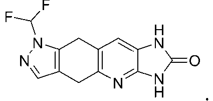

In another aspect, the present application provides use of the compound, the pharmaceutically acceptable salt thereof, the deuterated compound thereof, the stereoisomer thereof, the tautomer thereof, or the mixture thereof as described in any one of the foregoings in preparation of a drug for regulating the interaction between PDE3A and SLFN12. Alternatively, the present application provides the compound, the pharmaceutically acceptable salt thereof, the deuterated compound thereof, the stereoisomer thereof, the tautomer thereof, or the mixture thereof as described in any one of the foregoings for use as a drug for regulating the interaction between PDE3A and SLFN12. Alternatively, the present application provides a method for regulating the interaction between PDE3A and SLFN12, comprising administering to a subject in need thereof the compound, the pharmaceutically acceptable salt thereof, the deuterated compound thereof, the stereoisomer thereof, the tautomer thereof, or the mixture thereof as described in any one of the foregoings. Preferably, the drug is capable of enhancing and/or promoting the interaction between PDE3A and SLFN12.

In some embodiments, the compound, the pharmaceutically acceptable salt thereof, the deuterated compound thereof, the stereoisomer thereof, the tautomer thereof, or the mixture thereof as described in any one of the foregoings is used for preparing a drug for treating and/or preventing a disease, wherein the disease is a hyperproliferative disease. Alternatively, the present application provides the compound, the pharmaceutically acceptable salt thereof, the deuterated compound thereof, the stereoisomer thereof, the tautomer thereof, or the mixture thereof as described in any one of the foregoings for use in treating and/or preventing a disease, wherein the disease is a hyperproliferative disease. Alternatively, the present application provides a method for treating and/or preventing a disease, comprising administering to a subject in need thereof the compound, the pharmaceutically acceptable salt thereof, the deuterated compound thereof, the stereoisomer thereof, the tautomer thereof, or the mixture thereof as described in any one of the foregoings, wherein the disease is a hyperproliferative disease.

In some preferred embodiments, the hyperproliferative disease is a cancer disease.

In some preferred embodiments, the cancer disease includes brain cancer, breast cancer, cervical cancer, acute myeloid leukemia (AML), lung cancer, skin cancer, esophageal cancer, ovarian cancer, pancreatic cancer, prostate cancer, and sarcoma.

The molecular glue developed in the present application, which has no obvious inhibition on the enzymatic activity of PDE3A and is capable of effectively inducing the apoptosis of tumor cells, can maintain high pharmacological and pharmacodynamic activity while also reducing toxic side effects, thus having significant importance for the development of tumor drugs based on PDE3A. Moreover, the compound of the present invention has good inhibitory activity in melanoma cells and has excellent in vivo pharmacodynamic data in animals, thus having good pharmacokinetics.

### DESCRIPTION OF THE EMBODIMENTS

The following is a clear and complete description of the technical solutions in the examples of the present application. Obviously, the examples described are only a part of the examples of the present application, rather than all of the examples. Based on the examples in the present application, all other examples obtained by those of ordinary skill in the art without creative efforts shall fall within the scope of protection of the present application.

Structures of compounds are determined by mass spectrometry (MS) or nuclear magnetic resonance (¹H NMR). A chemical shift (δ) of nuclear magnetic resonance (¹H NMR) is given in the unit of parts per million (ppm). Determination by the nuclear magnetic resonance (¹H NMR) is performed using a Bruker AVANCE-400 nuclear magnetic resonance apparatus with deuterated dimethyl sulfoxide (DMSO) as a determining solvent and tetramethylsilane (TMS) as an internal standard, and the chemical shift is given in the unit of 10⁻⁶ (ppm). Determination by the mass spectrometry (MS) is performed using a FINNIGAN LCQAd (ESI) mass spectrometer (manufacturer: Therm, model: Finnigan LCQ advantage MAX).

Yantai Huanghai HSGF254 or Qingdao GF254 silica gel plate is used as a silica gel for thin-layer chromatography. Yantai Huanghai silica gel, 200-300 mesh, is generally used as a carrier for column chromatography.

### Definitions and general terms

Unless otherwise defined, all technical and scientific terms used herein have the meanings commonly understood by those skilled in the art to which the present application belongs. As used herein, unless otherwise specified, the following terms have the meanings assigned to them below.

The term "substitution" in the present application means that one or more (e.g., one, two, three, or four) hydrogens on a specified atom are replaced by a selection from specified groups, provided that a normal valence of the specified atom is not exceeded in a current instance and the substitution forms a stable compound. Combinations of substituents and/or variables are permitted only when such combinations form stable compounds. It should be understood that, as required, other atoms, such as hydrogen atoms or substituents as described herein, may be present to satisfy valencies of the atoms.

The term "independently" or "respectively" means that when more than one substituent is selected from many possible substituents, those substituents may be the same or different.

Whenever a group is described as "optional", "optionally substituted", or "substituted or unsubstituted", the group may be unsubstituted or substituted with one or more indicated substituents, and that is to say, "optionally substituted" indicates "substituted or unsubstituted". If no substituent is specified, it means that the group may be optionally substituted with one or more groups selected from but not limited to deuterium, alkyl, haloalkyl, cycloalkyl, heterocycloalkyl, alkenyl, alkynyl, aryl, heteroaryl, heterocyclyl, halogen, hydroxyl, hydroxyalkyl, alkoxy, haloalkoxy, cyano, nitro, amino, and aminohydroxyl. Preferably, the "optionally substituted" means being substituted with substituents independently selected from deuterium, C₁-C₆ alkyl, C₁-C₆ haloalkyl, C₁-C₆ haloalkoxy, C₃-C₆ cycloalkyl, F, Cl, Br, hydroxyl, C₁-C₆ alkoxy, cyano, amino, C₆-C₁₀ aryl, 3-6 membered heterocyclyl, 5-10 membered heteroaryl, and C₁-C₆ hydroxyalkyl, wherein the optionally substituted substituents also include isotopic forms in which any hydrogen atoms in the groups are substituted with deuterium. Specific examples for listing include, but are not limited to, methyl, CD₃, ethyl, propyl, CF₃, CHF₂, CH₂F, cyclopropyl, F, Cl, cyano, methoxy, ethoxy, OCHF₂, OCF₃, OCD₃, amino, phenyl, naphthyl, pyridyl, pyrazinyl, etc.

The term "Cₘ-Cₙ" in the present application means that the number of carbon atoms is at least m and at most n, including both endpoints m and n; and m and n are integers not equal to 0, wherein m < n.

The term "alkyl" appearing separately or in combination in the present application refers to a linear or branched saturated hydrocarbon group composed only of carbon atoms and hydrogen atoms (including deuterium atoms), including but not limited to C₁-C₆ alkyl, C₁-C₅ alkyl, C₁-C₄ alkyl, C₁-C₃ alkyl, C₁-C₂ alkyl, and C₁ alkyl (including deuterated C₁-C₆ alkyl, deuterated C₁-C₅ alkyl, deuterated C₁-C₄ alkyl, deuterated C₁-C₃ alkyl, deuterated C₁-C₂ alkyl, and deuterated C₁ alkyl). As non-limiting examples of the alkyl, the following linear or branched saturated hydrocarbon groups may be listed: methyl, ethyl, n-propyl, isopropyl, n-butyl, isobutyl, sec-butyl, tert-butyl, n-pentyl and other seven isomers thereof, and n-hexyl and other sixteen isomers thereof. For example, "C₁-C₆ alkyl" includes methyl, CD₃, ethyl, propyl, butyl, pentyl, hexyl, and all isomers thereof. The C₁-C₆ alkyl may be substituted with an optional substituent.

The term "haloalkyl" in the present application refers to an alkyl in which one or more hydrogen atoms are substituted with halogen, for example, substituted with F or Cl. For example, non-limiting examples of "C₁-C₆ haloalkyl" include, but are not limited to, -CF₃, -CHF₂, -CHCF₂, -CH₂CH₂F, and -CH₂CF₃.

The term "alkoxy" appearing separately or in combination in the present application refers to an "-O-alkyl" group, including deuterated alkoxy. The alkoxy herein includes, but is not limited to, C₁-C₆ alkoxy, C₁-C₅ alkoxy, C₁-C₄ alkoxy, C₁-C₃ alkoxy, C₁-C₂ alkoxy, and C₁ alkoxy (including deuterated C₁-C₆ alkoxy, deuterated C₁-C₅ alkoxy, deuterated C₁-C₄ alkoxy, deuterated C₁-C₃ alkoxy, deuterated C₁-C₂ alkoxy, and deuterated C₁ alkoxy). For example, the term "C₁-C₃ alkoxy" refers to a saturated linear or branched hydrocarbon moiety that has at least 1 and at most 3 carbon atoms and is bonded by connecting oxygen to atoms. Specific exemplary examples include, but are not limited to, methoxy, ethoxy, n-propoxy, isopropoxy, n-butoxy, sec-butoxy, and tert-butoxy (including deuterated methoxy, deuterated ethoxy, deuterated n-propoxy, deuterated isopropoxy, deuterated n-butoxy, deuterated sec-butoxy, and deuterated tert-butoxy), etc. The "alkoxy" may be substituted with an optional substituent. For example, non-limiting examples of substitution with F and Cl include -OCF₃, -OCF₂, etc.

The term "carbocyclyl" appearing separately or in combination in the present application refers to a monocyclic, bicyclic, or tricyclic hydrocarbon ring in which all ring members are carbon atoms, wherein any one of them may be saturated (i.e., cycloalkyl) or partially unsaturated (non-aromatic, e.g., cycloalkenyl, cyclodienyl, etc.). Non-limiting examples of such carbocyclyl include, but are not limited to, cyclopropyl, cyclobutyl, cyclobutenyl, cyclopentyl, cyclopentenyl, cyclohexyl, cycloheptenyl, cycloheptyl, cyclooctyl, cyclooctenyl, cyclooctendienyl, etc. As shown above, fused rings and bridged rings are also included in the definition of carbon rings. When the term "carbocyclyl" is used, it includes "cycloalkyl", "cycloalkenyl", and "partially unsaturated cycloalkyl (non-aromatic)".

The term "cycloalkyl" appearing separately or in combination in the present application refers to a saturated monocyclic or polycyclic (e.g., spiro, fused, or bridged) hydrocarbon ring. For example, the term "C₃-C₆ cycloalkyl" refers to a saturated monocyclic ring or polycyclic ring having 3 to 6 ring-forming carbon atoms, and its non-limiting examples include cyclopropyl, cyclobutyl, cyclopentyl, cyclohexyl, cycloheptyl, or cyclooctyl, which may be optionally substituted with one or more (e.g., 1 to 3) suitable substituents, for example, methyl-substituted cyclopropyl, etc.

The term "cycloalkenyl" appearing separately or in combination in the present application refers to a monocyclic ring or polycyclic ring that is completely formed by carbon atoms and has one or more carbon-carbon double bonds (non-aromatic) therein. For example, the term "C₄-C₉ cycloalkenyl" refers to a cycloalkenyl having 4 to 9 (e.g., 5, 6, 7, or 8) carbon atoms. Specific non-limiting examples include, but are not limited to, cyclobutenyl, cyclopentenyl, cyclohexenyl, cyclohexadienyl, cycloheptrienyl, etc.

The term "heterocyclyl" appearing separately or in combination in the present application refers to a monocyclic, bicyclic, or tricyclic group composed of carbon atoms and one or more heteroatoms, wherein the cyclic group may be saturated, partially unsaturated (non-aromatic), or completely unsaturated (aromatic), wherein at least one ring has at least one heteroatom (O, S, or N), and the ring containing heteroatoms preferably has 1, 2, or 3 heteroatoms selected from O, S, and N. For example, "4-9 membered heterocyclyl" refers to a 4-9 membered ring composed of carbon atoms and heteroatoms. Specific non-limiting examples of the "heterocyclyl" include, but are not limited to, aziridine, ethylene oxide, azetidine, oxetane, tetrahydrofuran, tetrahydrothiophene, tetrahydropyrrole, piperidine, pyrroline, oxazolidine, piperazine, dioxolane, dioxane, morpholine, tetrahydropyran, 1,2-dihydroazete, 1,2-dihydrooxete, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, etc. When the term "heterocyclyl" is used, it includes "heteroaryl".

The term "heteroaryl" appearing separately or in combination in the present application refers to a monovalent group having an aromatic monocyclic ring or a fused polycyclic ring, wherein at least one (e.g., 1, 2, 3, or 4) ring atom is a heteroatom selected from N, O, and S, and the remaining ring atoms are C. Non-limiting examples include, but are not limited to, 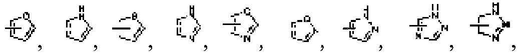 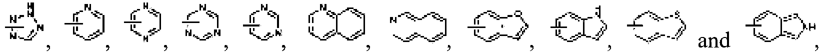 etc. For example, non-limiting examples of 5-10 membered heteroaryl include, but are not limited to, furyl, thienyl, pyrrolyl, pyrazolyl, oxazolyl, thiazolyl, imidazolyl, pyrazinyl, pyridazinyl, pyridyl, or pyrimidinyl, etc.

The term "aryl" appearing separately or in combination in the present application refers to an aromatic hydrocarbon group having multiple carbon atoms. The aryl is usually a monocyclic, bicyclic, or tricyclic aryl having multiple carbon atoms. Furthermore, the term "aryl" used herein refers to an aromatic substituent that may be a single aromatic ring or multiple aromatic rings fused together. Non-limiting examples include phenyl, naphthyl, or tetrahydronaphthyl.

The term "alkynyl" in the present application refers to a linear or branched monovalent hydrocarbon group having one or more carbon-carbon triple bonds, and for example, may have 2 to 8 carbon atoms, such as C₂-C₆ alkynyl and C₂-C₅ alkynyl. Specific non-limiting examples include, but are not limited to, acetenyl, propargyl, 1-propynyl, 1-butynyl, pentynyl, hexynyl, etc.

The term "halogen" in the present application refers to F, Cl, Br, or I. The term "hydroxyl" in the present application refers to an -OH group.

The term "amino" in the present application refers to an -NH₂ group, which may be substituted or unsubstituted. When referring to a substituted amino, it is -N(R")ₙ, wherein R" is independently an optional and suitable substituent (e.g., any substituent as defined above).

The term "cyano" in the present application refers to a -CN group. The term "nitro" in the present application refers to an -NO₂ group.

The alkyl, alkoxy, cycloalkyl, aryl, heteroaryl, cycloalkenyl, heterocyclyl, and carbocyclyl in the present application may all be optionally substituted with one or more suitable substituents.

The term "alkylamino" in the present application refers to a group in which a hydrogen atom of an amino is substituted with an alkyl (e.g., C₁-C₆ alkyl, C₁-C₃ alkyl, methyl, ethyl, etc.).

Unless otherwise specified, "hetero" and "heteroatom" herein refer to atoms other than carbon or hydrogen, including O, S, and N, but not limited thereto. Herein, the heteroaryl and heterocyclyl may contain 1 or more (e.g., 1-3 or 1-2) heteroatoms.

The term "nitrogen protection" in the present application refers to connecting a reaction flask to a nitrogen balloon with a volume of 1 L.

Unless otherwise specified in the present application, solutions referred to in reactions of the present application are aqueous solutions.

The term "room temperature" in the present application refers to the temperature between 10°C and 25°C.

When referring to a compound in the present application, all isomeric forms of such a structure, such as stereoisomers (including enantiomers and diastereomers), geometric isomers (or conformational isomers), and tautomeric forms are included. Among them, the term "stereoisomer" (also referred to as "optical isomer") refers to a stable isomer that has a vertical asymmetric plane due to at least one chiral factor (including a chiral center, a chiral axis, a chiral plane, etc.) and enables the rotation of plane-polarized light. The term "tautomer" (also referred to as "tautomeric form") refers to structural isomers with different energies that may be interconverted via a low-energy barrier. For example, proton tautomers (also referred to as proton transfer tautomers) include (but are not limited to) interconversions via proton migration, such as keto-enol isomerization, imine-enamine isomerization, amide-imidol isomerization, etc. The term "cis-trans isomer" refers to a stereoisomer formed by the different positions of atoms (or groups) located on either side of a double bond or ring system relative to a reference plane; in the cis-isomer, the atoms (or groups) are located on the same side of the double bond or the ring system, while in the trans-isomer, the atoms (or groups) are located on the opposite sides of the double bond or the ring system.

Furthermore, the compounds of the present application may also include non-natural proportions of atomic isotope forms at one or more atoms that constitute these compounds, that is, isotopic forms of hydrogen in D or T, or isotopes of any atoms, such as all isotopic forms of C and N. Specifically, radioactive isotopes, such as tritium (³H), iodine-125 (¹²⁵I), or carbon-14 (¹⁴C) may be used to radiolabel the compounds. All isotopic variants in the compounds of optional structures in the present application, whether radioactive or not, are encompassed within the scope of protection of the present application. Particularly, the present application includes deuterated forms of hydrogens at any positions in the following compounds of general formulae, including hydrogens on substituents:

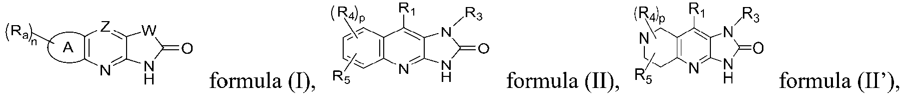

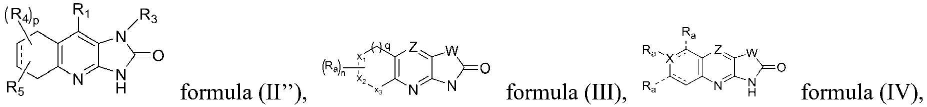

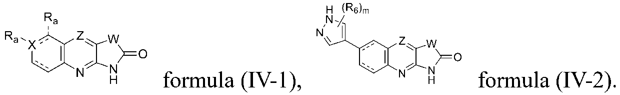

The terms "include", "comprise", "contain", and their equivalents should be understood as open and non-exclusive, that is, "including but not limited to" means that in addition to listed elements, components, and steps, other unspecified elements, components, and steps may also be encompassed. Unless otherwise specified, all numbers used herein to denote amounts of ingredients, measurement values, or reaction conditions should be understood to be modified by the term "about" in all cases. When associated with a percentage, the term "about" may indicate, for example, ±1%, preferably ±0.5%, and more preferably ±0.1%. Unless otherwise specified clearly in the context, singular terms herein encompass plural referents, and vice versa. Similarly, unless otherwise specified clearly in the context, the word "or" herein is intended to include "and".

### Intermediate 1a. Synthesis of 7-bromo-8-chloro-1,3-bis((2-(trimethylsilyl)ethoxy)methyl)-1,3-dihydro-2H-imidazo[4,5-b]quinolin-2-one

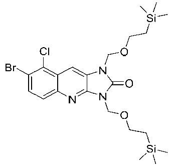

### 1) Synthesis of 5-(6-amino-3-bromo-2-chlorophenylmethylene)imidazolin-2,4-dione

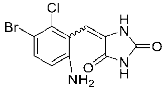

Sodium methoxide (932 mg, 17.24 mmol) was added into a solution of 6-amino-3-bromo-2-chlorobenzaldehyde (2 g, 8.53 mmol, synthesized by a method provided in WO2019167000A1) in ethanol (20 mL) and stirred for half an hour, and then diethyl (2,5-dioxoimidazolidin-4-yl)phosphonate (2.03 g, 8.62 mmol, CAS: 95378-36-2) was added and continuously stirred for 4 hours. The reaction was not treated, and the crude product was directly used in the next step. LC-MS: m/z [M+H]⁺ = 316/318.

### 2) Synthesis of 7-bromo-8-chloro-1,3-dihydro-2H-imidazo[4,5-b]quinolin-2-one

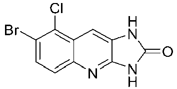

Acetic acid (5 mL) was directly added into the reaction solution obtained in the previous step and stirred at 25°C for 2 hours until a yellow solid was precipitated. The reaction solution was filtered. The filter cake was washed 3 times with ethanol (10 mL), collected, and concentrated under reduced pressure to obtain the title compound as a yellow solid (2.4 g of a crude product). LC-MS: m/z [M+H]⁺ = 298/300.

### 3) Synthesis of 7-bromo-8-chloro-1,3-bis((2-(trimethylsilyl)ethoxy)methyl)-1,3-dihydro-2H-imidazo[4,5-b]quinolin-2-one

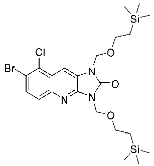

Sodium hydride (1.29 g, 32 mmol, 60%w/w in mineral oil) was added into a solution of 7-bromo-8-chloro-1,3-dihydro-2H-imidazo[4,5-b]quinolin-2-one (2.4 g, 8 mmol) in N,N-dimethylformamide (20 mL), the mixture was stirred at 0°C for 1 hour, and then 2-(trimethylsilyl)ethoxymethyl chlorine (3.36 g, 20.1 mmol) was added and stirred at 25°C for 2 hours. The reaction solution was poured into water (100 mL) for quenching and then extracted with ethyl acetate (50 mL x 2). The organic phases were combined, dried over anhydrous sodium sulfate, filtered, concentrated under reduced pressure, and purified by column chromatography (petroleum ether/ethyl acetate = 100:1-10:1) to obtain the title compound as a yellow solid (200 mg, three-step yield: 4%). LC-MS: m/z [M+H]⁺ = 558/560.

### Intermediate 1. Synthesis of 7-bromo-8-chloro-1,3-dihydro-2H-imidazo[4,5-b]quinolin-2-one

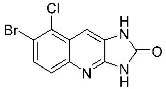

7-bromo-8-chloro-1,3-bis((2-(trimethylsilyl)ethoxy)methyl)-1,3-dihydro-2H-imidazo[4,5-b]quinolin-2-one (200 mg, 0.62 mmol, intermediate 1a) was dissolved in trifluoroacetic acid (3 mL) and stirred under microwave heating at 100°C for 1 hour. The reaction solution was added with dichloroethane (10 ml x 2), concentrated, and purified by reversed-phase chromatography (5%-95% acetonitrile and water) to obtain the title compound as a yellow solid (60 mg, yield: 56%). ¹H NMR (400 MHz, DMSO-*d*₆) δ 7.81 (d, J = 8 Hz, 1H), 7.75-7.71 (m, 2H); LC-MS: m/z [M+H]⁺ = 298/300.

### Intermediate 2. Synthesis of diethyl (1-(4-methoxybenzyl)-2,5-dioxopyrrolidin-3-yl)phosphonate

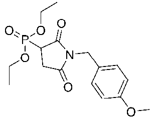

### 1) Synthesis of 1-(4-methoxybenzyl)-1H-pyrrole-2,5-dione

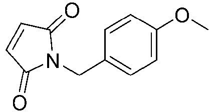

Furan-2,5-dione (5.0 g, 51.02 mmol) and p-methoxybenzylamine (7.7 g, 56.12 mmol) were dissolved in acetic acid (50 mL), and the reaction solution was heated to 110°C and left overnight. The reaction solution was cooled to room temperature and concentrated. A saturated sodium bicarbonate solution (100 mL) and ethyl acetate (100 mL) were added into the residue. The organic phase was separated, dried over anhydrous sodium sulfate, concentrated, and purified by column chromatography (petroleum ether/ethyl acetate = 3:1) to obtain the title compound as a white solid (3.0 g, yield: 27%). LC-MS: m/z [M+H]⁺ = 218.

### 2) Synthesis of diethyl (1-(4-methoxybenzyl)-2,5-dioxopyrrolidin-3-yl)phosphonate

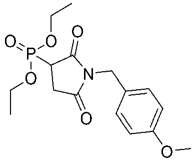

1-(4-methoxybenzyl)-1H-pyrrole-2,5-dione (1 g, 4.58 mmol) was dissolved in acetic acid (10 ml), triethyl phosphite (1.14 g, 6.87 mmol) was slowly added, and the reaction solution was heated to 100°C and stirred for 1 hour. The reaction solution was cooled to room temperature and concentrated to remove most of the acetic acid. The residue was poured into a saturated sodium bicarbonate solution (50 mL) and extracted with ethyl acetate (50 mL x 2). The organic phases were combined, dried over anhydrous sodium sulfate, and concentrated to obtain the title compound as a white oil (1.2 g of a crude product). LC-MS: m/z [M+H]⁺ = 356.

### Intermediate 3. Synthesis of 7-bromo-1,3-dihydro-2H-imidazo[4,5-b]quinolin-2-one

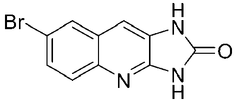

### 1) Synthesis of 6-bromo-3-nitroquinolin-2(1H)-one

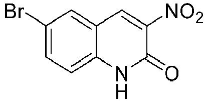

2-amino-5-bromobenzaldehyde (10.00 g, 50 mmol, CAS: 29124-57-0) and anhydrous ethanol (120 mL) were added into a 250 mL single-necked flask, then ethyl 2-nitroacetate (13.3 g, 100 mmol, CAS: 626-35-7) and piperidine (2.13 g, 25 mmol) were added into the above solution, and the reaction mixture was heated to 80°C and stirred under reflux overnight. The reaction solution was cooled to room temperature and filtered, and the filter cake was washed 2 times with isopropyl ether (20 mL) and then dried to obtain the title compound as a yellow solid (10.00 g, yield: 74%). LC-MS: m/z [M+H]⁺ = 269/271.

### 2) Synthesis of 6-bromo-2-chloro-3-nitro-1,2-dihydroquinoline

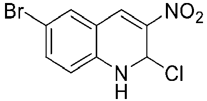

6-bromo-3-nitroquinolin-2(1H)-one (10.00 g, 37.2 mmol) was dissolved in phosphorus oxychloride (120 mL), and the mixture was allowed to react at 110°C overnight. The reaction solution was cooled to room temperature and then slowly added into ice water (300 mL) for quenching the reaction, the resulting suspension was filtered, and the filter cake was washed 2 times with water (20 mL) and then dried to obtain the title compound as a gray solid (9.7 g, yield: 90%). LC-MS: m/z [M+H]⁺ = 289/291.

### 3) Synthesis of 6-bromo-3-nitroquinolin-2-amine

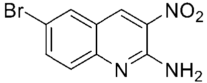

6-bromo-2-chloro-3-nitro-1,2-dihydroquinoline (9.7 g, 33.5 mmol) was added into a 200 mL sealed tank, a 2 M solution of ammonia in methanol (110 mL) was added, and the mixture was allowed to react at 120°C overnight. The reaction solution was cooled to room temperature and filtered, and the filter cake was washed 2 times with cyclohexane (20 mL) and then dried to obtain the title compound as a red solid (7.8 g, yield: 87%). LC-MS: m/z [M+H]⁺ = 268/270.

### 4) Synthesis of 6-bromoquinolin-2,3-diamine

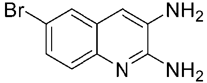

6-bromo-3-nitroquinolin-2-amine (7.8 g, 29.1 mol) was dissolved in methanol (120 mL), a Raney nickel catalyst (0.78 g, CAS: 7440-02-0) was added, hydrogen displacement was performed 3 times, and the reaction solution was stirred overnight at room temperature in a hydrogen atmosphere. The reaction solution was filtered through Celite, and the filtrate was directly concentrated to obtain the title compound as a red solid (6.8 g, yield: 98%). LC-MS: m/z [M+H]⁺ = 238/240.

### 5) Synthesis of 7-bromo-1,3-dihydro-2H-imidazo[4,5-b]quinolin-2-one

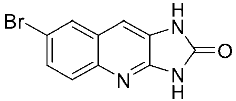

6-bromoquinolin-2,3-diamine (6.8 g, 28.6 mmol) was dissolved in acetonitrile (2 L), then N,N'-disuccinimidyl carbonate (7.4 g, 28.9 mmol) was added, and the reaction solution was stirred overnight at room temperature. The reaction solution was concentrated to remove most of the solvent and then diluted with water (200 mL), the mixture was stirred at room temperature for half an hour, and the solid was collected by filtration and then dried to obtain the title compound as a grayish-white solid (5 g, yield: 66%). LC-MS: m/z [M+H]⁺ = 264/266.

Referring to the table below, except that a raw material listed in the "Raw material" column was used to replace the corresponding raw material, the following intermediate 25 was prepared according to the preparation method of the intermediate 3.

| Number | Name | Structure | LC-MS (m/z [M+H]⁺) | Raw material |
|---|---|---|---|---|
| Intermediate 25 | 7-bromo-8-(trifluoromethyl)-1,3-dihydro -2H-imidazo[4,5-b]quinolin-2-one | 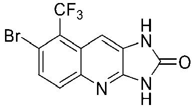 | 332/334 | Intermediate 24 |

### Intermediate 4. Synthesis of 7-bromo-1,3-bis((2-(trimethylsilyl)ethoxy)methyl)-1,3-dihydro-2H-imidazo[4,5-b]quinolin-2-one

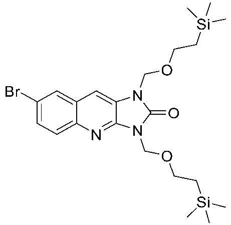

Sodium hydride (91 mg, 3.8 mmol, 60%w/w in mineral oil) was added into a solution of 7-bromo-1,3-dihydro-2H-imidazo[4,5-b]quinolin-2-one (500 mg, 1.9 mmol, intermediate 3) in N,N-dimethylformamide (10 mL), the mixture was stirred at 0°C for 1 hour, then 2-(trimethylsilyl)ethoxymethyl chlorine (790 mg, 4.7 mmol) was added, and the reaction solution was stirred overnight at room temperature. The reaction solution was poured into water (20 mL) for quenching and extracted twice with ethyl acetate (20 mL). The organic phases were combined, washed twice with water (30 mL), washed twice with brine (50 mL), dried over anhydrous sodium sulfate, filtered, concentrated under reduced pressure, and purified by column chromatography (petroleum ether/ethyl acetate = 5:1) to obtain the title compound as a white solid (337 mg, yield: 34%). LC-MS: m/z [M+H]⁺ = 524/526.

Referring to the table below, except that a raw material listed in the "Raw material" column was used to replace the corresponding raw material, the following intermediate 28 was prepared according to the preparation method of the intermediate 4.

| Number | Name | Structure | LC-MS (m/z [M+H]⁺) | Raw material |
|---|---|---|---|---|
| Intermediate 28 | 8-chloro-7-iodo-1,3-bis((2-(trimethylsilyl)ethoxy) methyl)-1,3-dihydro-2H-imidazo[4,5-b]quinoline -2-one | 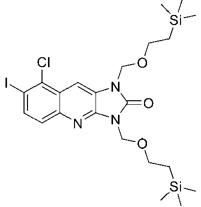 | 606 | Intermediate 27 |

### Intermediate 5. Synthesis of 7-(4,4,5,5-tetramethyl-1,3,2-dioxaborolan-2-yl)-1,3-bis((2-(trimethylsilyl)ethoxy)methyl)-1,3-dihydro-2H-imidazo[4,5-b]quinolin-2-one

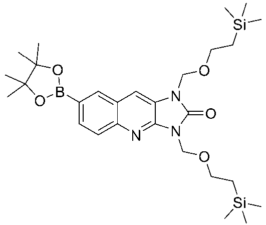

7-bromo-1,3-bis((2-(trimethylsilyl)ethoxy)methyl)-1,3-dihydro-2H-imidazo[4,5-b]quinolin-2-one (100 mg, 0.19 mmol, intermediate 4), bis(pinacolato)diboron (97 mg, 0.38 mmol, CAS: 73183-34-3), [1,1'-bis(diphenylphosphino)ferrocene]dichloropalladium (14 mg, 0.019 mmol, CAS: 72287-26-4), and potassium acetate (56 mg, 0.57 mmol) were dissolved in 1,4-dioxane (3 mL), nitrogen replacement was performed 3 times, and the reaction solution was heated and stirred overnight at 85°C in a nitrogen atmosphere. The reaction solution was cooled to room temperature, diluted with water (20 mL), and extracted twice with ethyl acetate (20 mL). The organic phases were combined, washed twice with water (20 mL), washed twice with a saturated salt solution (20 mL), dried over anhydrous sodium sulfate, concentrated under reduced pressure, and purified by column chromatography (petroleum ether/ethyl acetate = 5:1) to obtain the title compound as a white solid (150 mg, yield: 138%). LC-MS: m/z [M+H]⁺ = 572.

Referring to the table below, except that raw materials listed in the "Raw material" column were used to replace the corresponding raw material, the following intermediates 9 and 26 were prepared according to the preparation method of the intermediate 5.

| Number | Name | Structure | LC-MS (m/z [M+H]⁺) | Raw material |
|---|---|---|---|---|
| Intermediate 9 | 8-chloro-7-(4,4,5,5-tetramethyl-1,3,2-dioxaborolan -2-yl)-1,3-bis((2-(trimethylsilyl)ethoxy)methyl)-1,3 -dihydro-2H-imidazo[4,5-b]quinolin-2-one | 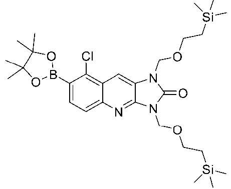 | 606 | Intermediate 1a |
| Intermediate 26 | 6-chloro-7-(4,4,5,5-tetramethyl-1,3,2-dioxaborolan -2-yl)-1,3-bis((2-(trimethylsilyl)ethoxy)methyl)-1,3 -dihydro-2H-imidazo[4,5-b]quinolin-2-one | 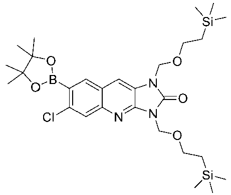 | 606 | Intermediate 21 |

### Intermediate 6. Synthesis of a mixture of 3-chloro-1-(difluoromethyl)-4-iodo-1H-pyrazole and 5-chloro-1-(difluoromethyl)-4-iodo-1H-pyrazole

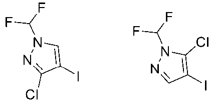

### 1) Synthesis of 3-chloro-4-iodo-1H-pyrazole

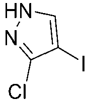

N-iodosuccinimide (5.49 g, 24.39 mmol, CAS: 516-12-1) was added into a solution of 3-chloro-1H-pyrazole (2.5 g, 24.39 mmol, CAS: 14339-33-4) in acetic acid (20 mL), and the mixture was stirred at 25°C for 16 hours. The reaction solution was filtered. The filter cake was collected, concentrated under reduced pressure, and purified by column chromatography (petroleum ether/ethyl acetate = 1:1) to obtain the title compound as a light yellow solid (1.28 g, yield: 23%). LC-MS: m/z [M+H]⁺ = 229.

### 2) Synthesis of a mixture of 3-chloro-1-(difluoromethyl)-4-iodo-1H-pyrazole and 5-chloro-1-(difluoromethyl)-4-iodo-1H-pyrazole

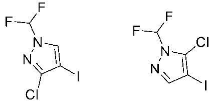

3-chloro-4-iodo-1H-pyrazole (1.22 g, 5.34 mmol) was added into a mixed solution of potassium hydroxide (4 g, 71.29 mmol) in acetonitrile (20 mL) and water (20 mL), then bromodifluoromethane diethyl phosphate (2.85 g, 10.68 mmol, CAS: 65094-22-6) was added into the reaction solution, and the reaction mixture was stirred at 60°C for 5 hours. The reaction solution was cooled to room temperature and extracted 3 times with ethyl acetate (15 mL). The organic phases were combined, dried over anhydrous sodium sulfate, filtered, concentrated under reduced pressure, and purified by column chromatography (petroleum ether/ethyl acetate = 2:1) to obtain a colorless oily mixture (1 g, yield: 67%, the mixture of 3-chloro-1-(difluoromethyl)-4-iodo-1H-pyrazole and 5-chloro-1-(difluoromethyl)-4-iodo-1H-pyrazole, which was directly used in the next step without further purification). LC-MS: m/z [M+H]⁺ = 279.

Referring to the table below, except that raw materials listed in the "Raw material" column were used to replace the corresponding raw material, the following intermediates 12 and 14 were prepared according to the preparation method of the intermediate 6.

| Number | Name | Structure | LC-MS (m/z [M+H]⁺) | Raw material |
|---|---|---|---|---|
| Intermediate 12 | Mixture of 5-bromo-1-(difluoromethyl)-4-iodo-1H-pyrazole and 3-bromo-1-(difluoromethyl)-4-iodo-1H-pyrazole | 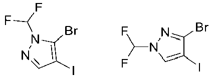 | 323/325 | 5-bromo-1H-pyrazole (CAS: 14521-80-3) |
| Intermediate 14 | Mixture of 5-chloro-1-(difluoromethyl)-4-iodo-1H-imidazole and 4-chloro-1-(difluoromethyl)-5-iodo-1H-imidazole | 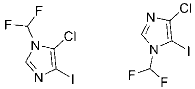 | 279 | 4-chloroimidazole (CAS: 15965-31-8) |

### Intermediate 7. Synthesis of a mixture of 3-bromo-4-chloro-1-(difluoromethyl)-1H-pyrazole and 5-bromo-4-chloro-1-(difluoromethyl)-1H-pyrazole

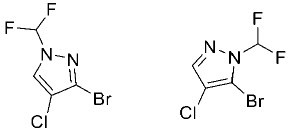

5-bromo-4-chloro-1H-pyrazole (250 mg, 1.38 mmol, CAS: 27258-12-4) was added into a mixed solution of potassium hydroxide (200 mg, 3.56 mmol) in acetonitrile (1 mL) and water (1 mL), then bromodifluoromethane diethyl phosphate (736 mg, 2.76 mmol, CAS: 65094-22-6) was added into the reaction solution, and the reaction mixture was stirred at 60°C for 5 hours. The reaction solution was cooled to room temperature and extracted 3 times with dichloromethane (2 mL). The organic phases were combined, dried over anhydrous sodium sulfate, filtered, concentrated under reduced pressure, and purified by column chromatography (petroleum ether/ethyl acetate = 10:1) to obtain a colorless oily mixture (84 mg, yield: 26%, the mixture of 3-bromo-4-chloro-1-(difluoromethyl)-1H-pyrazole and 5-bromo-4-chloro-1-(difluoromethyl)-1H-pyrazole, which was directly used in the next step without further purification).

### Intermediate 8. Synthesis of N²,N³,N³-tri(4-methoxybenzyl)-5,6,7,8-tetrahydro-1,6-naphthyridin-2,3-diamine

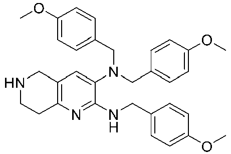

### 1) Synthesis of tert-butyl 3-nitro-7,8-dihydro-1,6-naphthyridin-6(5H)-carboxylate

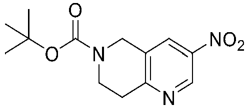

A 7 M solution of ammonia in methanol (37.09 mL, 259.64 mmol) was added into a solution of 1-methyl-3,5-dinitro-1,2-dihydropyridin-2-one (4.7 g, 23.60 mmol, CAS: 14150-94-8) and N-tert-butoxycarbonyl-4-piperidone (4.70 g, 23.60 mmol) in methanol (90 mL), and the mixture was stirred at 60°C for 5 hours. The reaction solution was cooled to room temperature, concentrated under reduced pressure, and purified by column chromatography (petroleum ether/ethyl acetate = 20:1-5:1) to obtain the title compound as a white solid (4.0 g, yield: 61%). LC-MS: m/z [M+H]⁺ = 280.

### 2) Synthesis of tert-butyl 3-amino-7,8-dihydro-1,6-naphthyridin-6(5H)-carboxylate

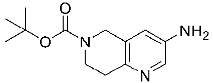

A 10% palladium on carbon catalyst (0.3 g, CAS: 7440-05-3) was added into a solution of tert-butyl 3-nitro-7,8-dihydro-1,6-naphthyridin-6(5H)-carboxylate (2 g, 7.16 mmol) in methanol (50 mL). The mixture was degassed, subjected to hydrogen replacement 3 times, and stirred at 25°C for 18 hours in a hydrogen atmosphere. The reaction solution was filtered through Celite, the filter cake was washed 1 time with methanol (20 mL), and the filtrate was concentrated under reduced pressure to obtain the title compound as a colorless oil (1.78 g, yield: 100%). LC-MS: m/z [M+H]⁺ = 250.

### 3) Synthesis of tert-butyl 3-amino-2-bromo-7,8-dihydro-1,6-naphthyridin-6(5H)-carboxylate

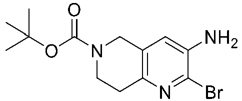

At 0°C, a solution of N-bromosuccinimide (1.27 g, 7.14 mmol) in N,N-dimethylformamide (10 mL) was added dropwise into a solution of tert-butyl 3-amino-7,8-dihydro-1,6-naphthyridin-6(5H)-carboxylate (1.78 g, 7.14 mmol) in N,N-dimethylformamide (15 mL), and after the dropping was completed, the reaction mixture was heated to 25°C and stirred for 18 hours. The mixture was poured into water (150 mL) and extracted 3 times with ethyl acetate (100 mL). The combined organic phases were washed 1 time with saline solution (50 mL), dried over anhydrous sodium sulfate, filtered, concentrated under reduced pressure, and purified by column chromatography (petroleum ether/ethyl acetate = 10:1-3:1) to obtain the title compound as a white solid (1.8 g, yield: 77%). LC-MS: m/z [M+H]⁺ = 328/330.

### 4) Synthesis of tert-butyl 3-(bis(4-methoxybenzyl)amino)-2-bromo-7,8-dihydro-1,6-naphthyridin-6(5H)-carboxylate

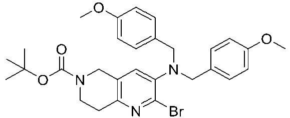

P-methoxybenzyl chloride (1.825 mL, 13.46 mmol, d = 1.155 g/mL) was added into a solution of tert-butyl 3-amino-2-bromo-7,8-dihydro-1,6-naphthyridin-6(5H)-carboxylate (2 g, 6.09 mmol) and potassium tert-butoxide (1.709 g, 15.23 mmol) in N,N-dimethylformamide (30 mL), and the mixture was stirred at 80°C for 18 hours. The mixture was poured into water (80 mL) and extracted 3 times with ethyl acetate (60 mL). The combined organic phases were washed with saline solution (30 mL), dried over anhydrous sodium sulfate, filtered, concentrated under reduced pressure, and purified by column chromatography (petroleum ether/ethyl acetate = 10:1-4:1) to obtain the title compound as a yellow solid (3.4 g, yield: 98%). LC-MS: m/z [M+H]⁺ = 568/570.

### 5) Synthesis of tert-butyl 3-(bis(4-methoxybenzyl)amino)-2-((4-methylbenzyl)amino) -7,8-dihydro-1,6-naphthyridin-6(5H)-carboxylate

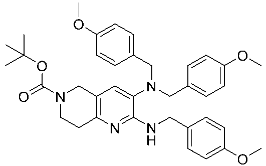

Tert-butyl 3-(bis(4-methoxybenzyl)amino)-2-bromo-7,8-dihydro-1,6-naphthyridin-6(5H)-carboxylate (3.4 g, 5.98 mmol), 4-methoxybenzylamine (1.23 g, 8.97 mmol), cesium carbonate (3.9 g, 11.96 mmol), palladium acetate (134.26 mg, 0.60 mmol, CAS: 3375-31-3), and 2,2'-bis(diphenylphosphino)-1,1'-binaphthyl (372.37 mg, 0.60 mmol, CAS: 98327-87-8) were added into 1,4-dioxane (60 mL). The mixture was degassed, subjected to nitrogen replacement 3 times, and stirred at 100°C for 18 hours in a nitrogen atmosphere. The reaction solution was filtered through Celite, the filter cake was washed 3 times with ethyl acetate (20 mL), and the filtrate was concentrated under reduced pressure and purified by column chromatography (petroleum ether/ethyl acetate = 10:1-4:1) to obtain the title compound as a yellow oil (2.5 g, yield: 67%). LC-MS: m/z [M+H]⁺ = 625.

### 6) Synthesis of N²,N³,N³-tri(4-methoxybenzyl)-5,6,7,8-tetrahydro-1,6-naphthyridin-2,3-diamine

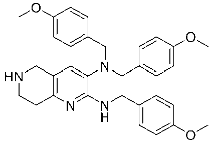

Zinc bromide (1405.68 mg, 6.24 mmol) was added into a solution of tert-butyl 3-(bis(4-methoxybenzyl)amino)-2-((4-methylbenzyl)amino)-7,8-dihydro-1,6-naphthyridin-6(5H)-carboxylate (780 mg, 1.25 mmol) in dichloromethane (35 mL), and the mixture was stirred at 25°C for 18 hours. The reaction solution was filtered through Celite and washed 3 times with dichloromethane (20 mL), and the filtrate was concentrated under reduced pressure and purified by preparative liquid chromatography (1% formic acid) to obtain the title compound as a yellow solid (547 mg, yield: 83%). LC-MS: m/z [M+H]⁺ = 525.

### Intermediate 10. Synthesis of a mixture of 3-bromo-2-chloro-6-(methoxy-d3)pyridine and 3-bromo-6-chloro-2-(methoxy-d3)pyridine

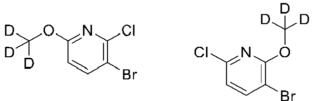

Under nitrogen protection and at 0°C, sodium hydride (0.21 g, 5.29 mmol, 60% dispersed in mineral oil) was added into deuterated methanol (2 mL, 49.35 mmol, d = 0.888 g/mL, CAS: 811-98-3), and the mixture was stirred at room temperature for 0.5 hour. A solution of 3-bromo-2,6-dichloropyridine (1 g, 4.41 mmol, CAS: 866755-20-6) in deuterated methanol (6 mL) was added dropwise into the above mixture, and stirred at 60°C for 16 hours. The reaction solution was quenched with a saturated ammonium chloride solution (15 mL) and then extracted 3 times with ethyl acetate (10 mL). The organic layer was dried over anhydrous sodium sulfate, filtered, and concentrated under reduced pressure to obtain a crude product. The crude product was purified by column chromatography (petroleum ether/ethyl acetate = 5:1) to obtain the title mixture (950 mg, white solid). LC-MS: m/z [M+H]⁺ = 225/227.

### Intermediate 11. Synthesis of a mixture of 1-(difluoromethyl)-4-iodo-5-(trifluoromethyl)-1H-pyrazole and 1-(difluoromethyl)-4-iodo-3-(trifluoromethyl)-1H-pyrazole

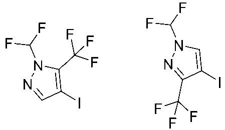

At 0°C, bromodifluoromethane diethyl phosphate (1450 mg, 5.43 mmol, CAS: 65094-22-6) was added into a mixed solution of 4-iodo-3-(trifluoromethyl)-1H-pyrazole (1050 mg, 4.01 mmol, CAS: 866638-72-4), potassium hydroxide (800 mg, 14.26 mmol), water (10 mL), and acetonitrile (12 mL) to react at 60°C for 15 hours. The reaction solution was concentrated and purified by column chromatography (petroleum ether/ethyl acetate = 15:1) to obtain the title mixture as a yellow viscous liquid (200 mg). LC-MS: m/z [M+H]⁺ = 313.

### Intermediate 13. Synthesis of 5-bromo-4-chloro-3-(difluoromethyl)isoxazole

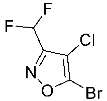

### 1) Synthesis of ethyl 5-amino-4-chloroisoxazole-3-carboxylate

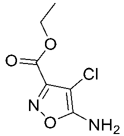

N-chlorosuccinimide (4.49 g, 33.62 mmol, CAS: 128-09-6) was added into a solution of ethyl 5-aminoisoxazole-3-carboxylate (5 g, 32.02 mmol, CAS: 1629161-40-5) in N,N-dimethylformamide (50 mL), and the mixture was stirred at 25°C for 16 hours. The reaction solution was diluted with water (500 mL) and extracted 3 times with ethyl acetate (50 mL). The organic phases were dried over anhydrous sodium sulfate, filtered, concentrated under reduced pressure, and purified by column chromatography (petroleum ether/ethyl acetate = 1:1) to obtain the title compound as a light yellow solid (5.77 g, yield: 94.6%). LC-MS: m/z [M+H]⁺ = 191.

### 2) Synthesis of ethyl 5-bromo-4-chloroisoxazole-3-carboxylate

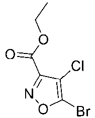

Tert-butyl nitrite (2.52 mL, 20.9 mmol, d = 0.867 g/mL) was added dropwise into a solution of ethyl 5-amino-4-chloroisoxazole-3-carboxylate (2 g, 10.49 mmol) and copper bromide (2.34 g, 10.49 mmol) in acetonitrile (20 mL), and the mixture was stirred at 25°C for 2 hours. The reaction solution was diluted with water (50 mL) and extracted 3 times with ethyl acetate (10 mL). The organic phases were dried over anhydrous sodium sulfate, filtered, concentrated under reduced pressure, and purified by column chromatography (petroleum ether/ethyl acetate = 1:1) to obtain the title compound as a light yellow oil (1.4 g, yield: 52%). LC-MS: m/z [M+H]⁺ = 254/256.

### 3) Synthesis of (5-bromo-4-chloroisoxazol-3-yl)methanol

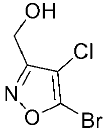

At 0°C, sodium borohydride (1.33 g, 35.19 mmol) was added into a solution of ethyl 5-bromo-4-chloroisoxazole-3-carboxylate (2.985 g, 11.73 mmol) in methanol (20 ml), and the mixture was stirred at 50°C for 2 hours. The reaction solution was added with a saturated aqueous solution of ammonium chloride (50 mL) for quenching the reaction and then extracted 3 times with ethyl acetate (10 mL). The organic phases were dried over anhydrous sodium sulfate, filtered, and concentrated under reduced pressure to obtain the title compound as a colorless oil (2 g, yield: 80%). LC-MS: m/z [M+H]⁺ = 212/214.

### 4) Synthesis 5-bromo-4-chloroisoxazole-3-carboxaldehyde

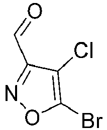

Dess-Martin periodinane (4.55 g, 10.72 mmol, CAS: 87413-09-0) was added into a solution of (5-bromo-4-chloroisoxazol-3-yl)methanol in dichloromethane (25 mL), and the mixture was stirred at 25°C for 4 hours. The reaction was quenched with a saturated aqueous solution of sodium thiosulfate (20 mL), and the mixture was extracted 3 times with dichloromethane (10 mL). The organic phases were dried over anhydrous sodium sulfate, filtered, concentrated under reduced pressure, and purified by column chromatography (petroleum ether/ethyl acetate = 1:100) to obtain the title compound as a light yellow solid (1.1 g, yield: 54%). LC-MS: m/z [M+H]⁺ = 210/212.

### 5) Synthesis of 5-bromo-4-chloro-3-(difluoromethyl)isoxazole

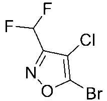

At -30°C and under nitrogen protection, diethylaminosulfur trifluoride (DAST, 1.57 mL, 11.88 mmol, d = 1.22 g/mL, CAS: 38078-09-0) was added dropwise into a solution of 5-bromo-4-chloroisoxazole-3-carboxaldehyde (500 mg, 2.38 mmol) in dichloromethane (10 mL), and the mixture was stirred at 25°C for 18 hours. The reaction was quenched with a saturated aqueous solution of sodium bicarbonate (10 mL), and the mixture was extracted 3 times with dichloromethane (5 mL). The organic phases were dried over anhydrous sodium sulfate, filtered, concentrated under reduced pressure, and purified by column chromatography (petroleum ether/ethyl acetate = 2:1) to obtain the title compound as a light yellow solid (120 mg, yield: 22%). ¹H NMR (400 MHz, DMSO-*d*₆) δ 7.40 (t, J = 51.6 Hz, 1H); LC-MS: m/z [M+H]⁺ = 232/234.

### Intermediate 15. Synthesis of 3-bromo-6-chloro-2-(difluoromethoxy)pyridine

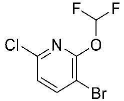

### 1) Synthesis of 6-(difluoromethoxy)pyridin-2-amine

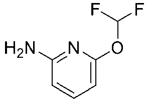

Sodium 2-chloro-2,2-difluoroacetate (8306.94 mg, 54.49 mmol, CAS: 1895-39-2) was added into a solution of 6-aminopyridin-2-one (3000 mg, 27.24 mmol, CAS: 5154-00-7) and potassium carbonate (5647.48 mg, 40.86 mmol) in N,N-dimethylformamide (45 mL), and the mixture was stirred at 100°C for 3 hours under nitrogen protection. The reaction solution was cooled to room temperature, then poured into water (200 mL), and extracted 3 times with ethyl acetate (50 mL). The organic phases were combined, washed 1 time with brine (100 mL), dried over anhydrous sodium sulfate, filtered, concentrated under reduced pressure, and purified by column chromatography (petroleum ether/ethyl acetate = 10:1) to obtain the title compound as a colorless oil (3.50 g, yield: 80%). LC-MS: m/z [M+H]⁺ = 161.

### 2) Synthesis of 5-bromo-6-(difluoromethoxy)pyridin-2-amine

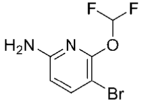

A solution of N-bromosuccinimide (2.22 g, 12.49 mmol) in N,N-dimethylformamide (10 mL) was added into a solution of 6-(difluoromethoxy)pyridin-2-amine (2 g, 12.49 mmol) in N,N-dimethylformamide (10 mL), and the mixture was stirred at 25°C for 18 hours. The reaction solution was poured into a saturated sodium chloride solution (300 mL) and extracted 3 times with ethyl acetate (150 mL). The organic phases were combined, washed 3 times with brine (100 mL), dried over anhydrous sodium sulfate, filtered, concentrated under reduced pressure, and purified by column chromatography (petroleum ether/ethyl acetate = 5:1) to obtain the title compound as a yellow solid (2090 mg, yield: 70%). LC-MS: m/z [M+H]⁺ = 239/241.

### 3) Synthesis of 3-bromo-6-chloro-2-(difluoromethoxy)pyridine

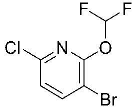

Under nitrogen protection, tert-butyl nitrite (3.51 mL, 29.29 mmol, d = 0.867 g/mL, CAS: 540-80-7) was added into a solution of 5-bromo-6-(difluoromethoxy)pyridin-2-amine (700 mg, 2.93 mmol) and benzyltriethylammonium chloride (3001.87 mg, 13.18 mmol, CAS: 56-37-1) in dichloromethane (30 mL), and the mixture was stirred at 25°C for 3 hours. The reaction was quenched with a saturated aqueous solution of sodium thiosulfate (100 mL), and the mixture was extracted 3 times with ethyl acetate (50 mL). The organic phases were combined, washed 1 time with brine (30 mL), dried over anhydrous sodium sulfate, filtered, concentrated under reduced pressure, and purified by column chromatography (petroleum ether/ethyl acetate = 20:1) to obtain the title compound as a colorless oil (470 mg, yield: 62%).

### Intermediate 16a. Synthesis of 5-bromo-6-chloro-N-methylpyridin-2-amine

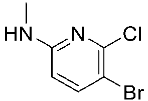

A 2 M solution of methylamine in tetrahydrofuran (11 mL, 22.3 mmol) was added into a solution of 3-bromo-2,6-dichloropyridine (1000 mg, 4.46 mmol, CAS: 866755-20-6) in tetrahydrofuran (10 mL), and the mixture was stirred overnight at 70°C. The reaction solution was directly concentrated under reduced pressure and purified by column chromatography (petroleum ether/ethyl acetate = 3:1) to obtain the title compound as a white solid (500 mg, yield: 51%). LC-MS: m/z [M+H]⁺ = 221/223.

### Intermediate 16b. Synthesis of a mixture of 3-bromo-6-chloro-N-methylpyridin-2-amine

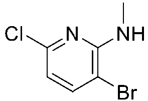

3-bromo-6-chloropyridin-2-amine (1000 mg, 4.85 mmol, CAS: 442127-50-6) and methyl trifluoromethanesulfonate (1171 mg, 7.27 mmol, CAS: 333-27-7) were dissolved in hexafluoroisopropanol (10 mL, CAS: 920-66-1), the mixture was stirred overnight at 25°C, and the reaction solution was added with water (20 mL) and extracted 2 times with ethyl acetate (20 mL). The organic phases were combined, dried over anhydrous sodium sulfate, filtered, concentrated under reduced pressure, and purified by column chromatography (petroleum ether/ethyl acetate = 3:1) to obtain the title compound as a white solid (200 mg, yield: 18%). LC-MS: m/z [M+H]⁺ = 221/223.

### Intermediate 17a. Synthesis of 5-(difluoromethoxy)-1-(difluoromethyl)-4-iodo-1H-pyrazole

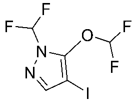

### Intermediate 17b. Synthesis of 3-(difluoromethoxy)-1-(difluoromethyl)-4-iodo-1H-pyrazole

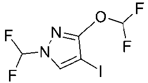

### 1) Synthesis of tert-butyl 3-oxo-2,3-dihydro-1H-pyrazole-1-formate

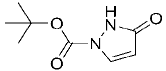

Di-tert-butyl dicarbonate (14.00 mL, 65.41 mmol, CAS: 24424-99-5) was added into a solution of 1,2-dihydro-3H-pyrazol-3-one (5 g, 59.47 mmol, CAS: 137-45-1) and triethylamine (9.89 mL, 71.36 mmol, d = 0.728 g/mL) in dichloromethane (50 mL), and the mixture was stirred at 25°C for 5 hours. The reaction solution was directly concentrated and purified by column chromatography (petroleum ether/ethyl acetate = 10:1-2:1) to obtain the title compound as a yellow solid (8.8 g, yield: 51%).

### 2) Synthesis of 3-(difluoromethoxy)-1H-pyrazole

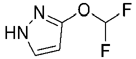

Tert-butyl 3-oxo-2,3-dihydro-1H-pyrazole-1-formate (7.8 g, 42.35 mmol) and potassium hydroxide (7.13 g, 127.04 mmol) were added into acetonitrile (80 mL) and water (80 mL), then bromodifluoromethane diethyl phosphate (22.61 g, 84.70 mmol, CAS: 65094-22-6) was added, and the mixture was stirred at 60°C for 5 hours. The reaction solution was poured into brine (300 mL) and extracted 3 times with ethyl acetate (200 mL). The organic phases were combined, dried over anhydrous sodium sulfate, filtered, concentrated under reduced pressure, and purified by column chromatography (petroleum ether/dichloromethane = 10:1-1:1) to obtain the title compound as a colorless oil (1.95 g, yield: 34%). LC-MS: m/z [M+H]⁺ = 135.

### 3) Synthesis of 3-(difluoromethoxy)-4-iodo-1H-pyrazole

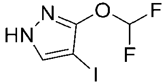

3-(difluoromethoxy)-1H-pyrazole (2.04 g, 15.21 mmol) was dissolved in N,N-dimethylformamide (80 mL), then a solution of N-iodosuccinimide (3.42 g, 15.21 mmol, CAS: 516-12-1) in N,N-dimethylformamide (10 mL) was added, and the mixture was stirred at 25°C for 18 hours. The reaction solution was poured into brine (1000 mL) and extracted 3 times with ethyl acetate (500 mL). The organic phases were combined, dried over anhydrous sodium sulfate, filtered, concentrated under reduced pressure, and purified by column chromatography (petroleum ether/dichloromethane= 10:1-2:1) to obtain the title compound as a yellow solid (3.5 g, yield: 88%). LC-MS: m/z [M+H]⁺ = 261.

### 4) Synthesis of 5-(difluoromethoxy)-1-(difluoromethyl)-4-iodo-1H-pyrazole and 3-(difluoromethoxy)-1-(difluoromethyl)-4-iodo-1H-pyrazole

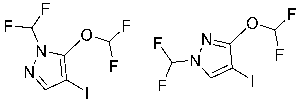

3-(difluoromethoxy)-4-iodo-1H-pyrazole (1 g, 3.85 mmol) and potassium hydroxide (2.16 g, 38.46 mmol) were added into acetonitrile (20 mL) and water (20 mL), then bromodifluoromethane diethyl phosphate (2.05 g, 7.69 mmol, CAS: 65094-22-6) was added, and the mixture was stirred at 60°C for 18 hours. The reaction solution was poured into water (100 mL) and extracted 3 times with ethyl acetate (50 mL). The organic phases were combined, dried over anhydrous sodium sulfate, filtered, concentrated under reduced pressure, and purified by column chromatography (petroleum ether/ethyl acetate = 30:1) to obtain 5-(difluoromethoxy)-1-(difluoromethyl)-4-iodo-1H-pyrazole as a colorless oil (117 mg, intermediate 17a) and 3-(difluoromethoxy)-1-(difluoromethyl)-4-iodo-1H-pyrazole as a colorless oil (277 mg, intermediate 17b), respectively. LC-MS: m/z [M+H]⁺ = 311.

### Intermediate 18. Synthesis of (methyl-d3)boronic acid

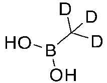

At -70°C to -60°C, methyl-d3-magnesium iodide (8 mL, 8.00 mmol, 1 M ethyl ether solution) was slowly added into a solution (15 mL) of trimethyl borate (1.63 mL, 14.40 mmol, CAS: 121-43-7) in tetrahydrofuran. The reaction solution was stirred at -70°C for 1 hour under nitrogen protection, slowly heated to room temperature, and then stirred at room temperature for 1 hour. An aqueous solution of hydrochloric acid (5 mL, 1 N) was slowly added dropwise to quench the reaction, and ethyl acetate (50 mL) was added. The organic phase was washed 1 time with brine (20 mL), dried over anhydrous sodium sulfate, filtered, and concentrated to obtain the title compound as a yellow oil (400 mg, crude product), which was directly used in the next reaction.

### Intermediate 19. Synthesis of 1-bromo-2-chloro-4-(difluoromethyl)benzene

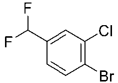

4-bromo-3-chlorobenzaldehyde (2 g, 9.26 mmol, CAS: 120077-69-2) was dissolved in dichloromethane (20 mL), diethylaminosulphur trifluoride (DAST, 2.98 g, 18.52 mmol, CAS: 38078-09-0) was slowly added at 0°C, and after the addition was completed, the mixture was heated to 25°C and stirred overnight. The reaction solution was added with water (100 mL) to quench the reaction and then extracted 2 times with dichloromethane (60 mL). The organic phases were combined, dried over anhydrous sodium sulfate, filtered, concentrated under reduced pressure, and purified by column chromatography (petroleum ether/dichloromethane = 10:1) to obtain the title compound as a yellow oil (2.0 g, yield: 91%).

### Intermediate 20. Synthesis of 7-bromo-8-fluoro-1,3-bis((2-(trimethylsilyl)ethoxy)methyl)-1,3-dihydro-2H-imidazo[4,5-blquinolin-2-one

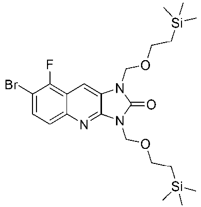

### 1) Synthesis of 2-amino-6-fluorobenzaldehyde

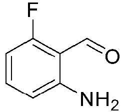

Active manganese dioxide (6467.47 mg, 74.3 mmol) was added into a solution of (2-amino-6-fluorophenyl)methanol (2100 mg, 14.88 mmol, CAS: 221285-25-2) in dichloromethane (30 mL), and the mixture was stirred overnight at 25°C. The reaction solution was filtered, and the filtrate was concentrated under reduced pressure to obtain the title compound as a light yellow solid (2000 mg, yield: 97%). LC-MS: m/z [M+H]⁺ = 140.

### 2) Synthesis of 6-amino-3-bromo-2-fluorobenzaldehyde

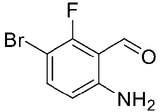

At 0°C, N-bromosuccinimide (2.51 g, 14.09 mmol) was added into a solution of 2-amino-6-fluorobenzaldehyde (1.96 g, 14.09 mmol) in N,N-dimethylformamide (20 mL), and the mixture was stirred at 25°C for 2 hours. The reaction solution was poured into ice water (50 mL), the precipitated solid was collected by filtration, and the filter cake was washed 3 times with water (10 mL) and then dried to obtain the title compound as a light yellow solid (2.71 g, yield: 88%). LC-MS: m/z [M+H]⁺ = 218/220.

### 3) Synthesis of 5-(6-amino-3-bromo-2-fluorobenzylidene)imidazolin-2,4-dione

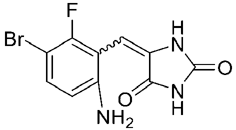

Sodium methoxide (3.30 mL, 17.75 mmol, 30 wt% methanol solution) was added dropwise into a solution of diethyl (2,5-dioximidazolin-4-yl)phosphonate (4.19 g, 17.75 mmol, CAS: 95378-36-2) in ethanol (10 mL), and the mixture was stirred at 25°C for 20 minutes. Then, 6-amino-3-bromo-2-fluorobenzaldehyde (2.58 g, 11.83 mmol) in ethanol was added dropwise, and after the dropping was completed, the reaction solution was stirred at 25°C for 1 hour. The reaction solution was not treated and was directly used in the next step. LC-MS: m/z [M+H]⁺ = 300/302.

### 4) Synthesis of 7-bromo-8-fluoro-1,3-dihydro-2H-imidazo[4,5-b]quinolin-2-one

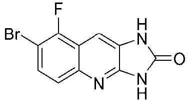

Acetic acid (15 mL) was directly added into the reaction solution in the previous step and stirred at 25°C for 2 hours until a yellow solid was precipitated. The reaction solution was filtered. The filter cake was washed 3 times with ethanol (10 mL), collected, and concentrated under reduced pressure to obtain the title compound as a yellow solid (2.77 g of a crude product). LC-MS: m/z [M+H]⁺ = 282/284.

### 5) Synthesis of 7-bromo-8-fluoro-1,3-bis((2-(trimethylsilyl)ethoxy)methyl)-1,3-dihydro-2H-imidazo[4,5-b]quinolin-2-one

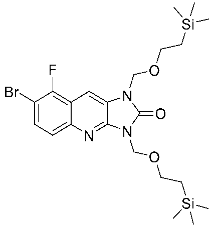

Sodium hydride (1.19 g, 30 mmol, 60%w/w in mineral oil) was added into a solution of 7-bromo-8-fluoro-1,3-dihydro-2H-imidazo[4,5-b]quinolin-2-one (2.1 g, 7.44 mmol , intermediate 20) in N,N-dimethylformamide (30 mL), the mixture was stirred at 25°C for 1 hour, then 2-(trimethylsilyl)ethoxymethyl chloride (3.30 mL, 18.61 mmol, d = 0.942 g/mL) was added, and the reaction solution was stirred at 25°C for 2 hours. The reaction solution was poured into a saturated ammonium chloride solution (50 mL) for quenching and then extracted with ethyl acetate (15 mL x 3). The organic phases were combined, dried over anhydrous sodium sulfate, filtered, concentrated under reduced pressure, and purified by column chromatography (petroleum ether/ethyl acetate = 10:1) to obtain the title compound as a yellow solid (711 mg, yield: 18%). LC-MS: m/z [M+H]⁺ = 542/544.

Referring to the table below, except that a raw material listed in the "Raw material" column was used to replace the corresponding raw material, the following intermediate 21 was prepared according to the preparation method of the intermediate 20.

| Number | Name | Structure | LC-MS (m/z [M+H]⁺) | Raw material |
|---|---|---|---|---|
| Intermediate 21 | 7-bromo-6-chloro-1,3-bis((2-(trimethylsilyl) ethoxy)methyl)-1,3-dihydro-2H-imidazo[4,5-b] quinolin-2-one | 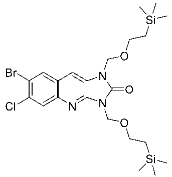 | 558/560 | 2-amino-5-bromo -4-chlorobenzaldehyde (CAS: 1036757-11-5) |

### Intermediate 22. Synthesis of N-bis(tert-butoxycarbonyl)-2-aminoacetonitrile

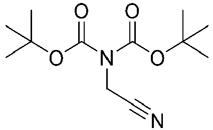

Bromoacetonitrile (16.56 g, 138.08 mmol, CAS: 590-17-0) was added into a mixed solution of bis(tert-butoxycarbonyl)amine (25.00 g, 115.07 mmol, CAS: 51779-32-9), sodium hydroxide (13.81 g, 345.21 mmol), and tetrabutylammonium bromide (3.71 g, 11.51 mmol) in tetrahydrofuran (200 mL) and water (200 mL), and the mixture was stirred at 45°C for 2 hours. The organic phase was separated, and the aqueous phase was extracted 1 time with ethyl acetate (300 mL). The organic phases were combined, washed 3 times with brine (300 mL), dried over anhydrous sodium sulfate, filtered, and concentrated under reduced pressure to obtain the title compound as a black solid (26.4 g, yield: 95%).

### Intermediate 23. Synthesis of methyl 6-amino-3-bromo-2-methoxybenzoate

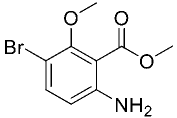

### 1) Synthesis of 5-methoxy-2H-benzo[d][1,3]oxazin-2,4(1H)-dione

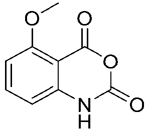

At 0°C, a solution of trisphosgene (8.78 mL, 52.64 mmol, 6 M toluene solution) in toluene (125 mL) was slowly added into a solution of 2-amino-6-methoxybenzoic acid (22 g, 131.61 mmol, CAS: 53600-33-2) and sodium hydroxide (14.48 g, 361.93 mmol) in water (500 mL), and the reaction mixture was heated to 25°C and stirred for 18 hours. The precipitated solid was collected by filtration, and the filter cake was washed 1 time with water (100 mL) and then dried to obtain the title compound as a white solid (16.5 g, yield: 65%). LC-MS: m/z [M+H]⁺ = 192.

### 2) Synthesis of 6-bromo-5-methoxy-2H-benzo[d][1,3]oxazin-2,4(1H)-dione

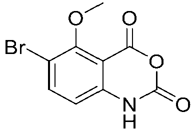

At 0°C, a solution of N-bromosuccinimide (16.72 g, 93.96 mmol) in N,N-dimethylformamide (60 mL) was slowly added dropwise into a solution of 5-methoxy-2H-benzo[d][1,3]oxazin-2,4(1H)-dione (16.5 g, 85.42 mmol) in N,N-dimethylformamide (60 mL) and dichloromethane (360 mL), and the reaction mixture was heated to 25°C and stirred for 18 hours. The dichloromethane solvent was removed by concentration under reduced pressure, the residue was poured into water (150 mL), the precipitated solid was collected by filtration, and the filter cake was washed 1 time with water (30 mL) and dried to obtain the title compound as a white solid (37 g of a crude product). LC-MS: m/z [M+H]⁺ = 270/272.

### 3) Synthesis of methyl 6-amino-3-bromo-2-methoxybenzoate

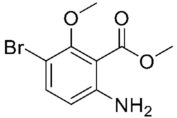

A solution of 6-bromo-5-methoxy-2H-benzo[d][1,3]oxazin-2,4(1H)-dione (37.2 g, 136.74 mmol) in methanol (300 mL) was stirred at 70°C for 18 hours. The reaction solution was directly concentrated under reduced pressure and purified by column chromatography (petroleum ether/ethyl acetate = 100:1-10:1) to obtain the title compound as a white solid (5.69 g, yield: 16%). LC-MS: m/z [M+H]⁺ = 260/262.

### Intermediate 24. Synthesis of 6-amino-3-bromo-2-(trifluoromethyl)benzaldehyde

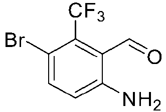

### 1) Synthesis of (2-amino-6-(trifluoromethyl)phenyl)methanol

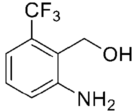

2-amino-6-(trifluoromethyl)benzoic acid (5 g, 24.4 mmol) was dissolved in anhydrous tetrahydrofuran (60 mL), lithium aluminum hydride (0.93g, 24.4 mmol) was added at 0°C under nitrogen protection, and the mixture was allowed to react at 0°C for 4 hours. The reaction solution was quenched with sodium sulfate decahydrate and extracted 3 times with dichloromethane (50 mL). The organic phases were combined, concentrated under reduced pressure, and purified by column chromatography (petroleum ether/ethyl acetate = 10:1) to obtain the title compound as a white solid (2 g, yield: 43%). LC-MS: m/z [M+H]⁺ = 192.

### 2) Synthesis of 2-amino-6-(trifluoromethyl)benzaldehyde

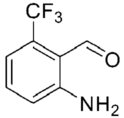

Active manganese dioxide (4.5 g, 52.3 mmol) was added into a solution of (2-amino-6-(trifluoromethyl)phenyl)methanol (2 g, 10.5 mmol) in dichloromethane (60 mL), and the mixture was stirred overnight at 25°C. The reaction solution was filtered, and the filtrate was concentrated under reduced pressure to obtain the title compound as a colorless oil (1.5 g, yield: 76%). LC-MS: m/z [M+H]⁺ = 190.

### 3) Synthesis of 6-amino-3-bromo-2-(trifluoromethyl)benzaldehyde

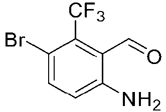

At 0°C, N-bromosuccinimide (1.4 g, 7.9 mmol) was added into a solution of 2-amino-6-(trifluoromethyl)benzaldehyde (1.5 g, 7.9 mmol) in N,N-dimethylformamide (20 mL), and the mixture was stirred overnight at 25°C. The reaction solution was poured into ice water (50 mL), the precipitated solid was collected by filtration, and the filter cake was washed 3 times with water (10 mL) and then dried to obtain the title compound as a yellow solid (1.0 g, yield: 47%). LC-MS: m/z [M+H]⁺ = 268/270.

### Intermediate 27. Synthesis of 8-chloro-7-iodo-1,3-dihydro-2H-imidazo[4,5-b]quinolin-2-one

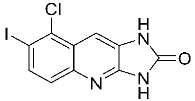

### 1) Synthesis of 6-amino-2-chloro-3-iodobenzaldehyde

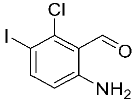

At room temperature, N-iodosuccinimide (14.46 g, 64.28 mmol, CAS: 516-12-1) was added in batches into a solution of 2-chloro-6-aminobenzaldehyde (10 g, 64.28 mmol, CAS: 35490-90-5) in acetic acid (80 mL), and the reaction mixture was stirred at room temperature for 2 hours. The reaction solution was filtered. The filtrate was collected, concentrated under reduced pressure, and dried to obtain the title compound as a yellow solid (10.9 g, yield: 60%). LC-MS: m/z [M+H]⁺ = 282/284.

### 2) Synthesis of tert-butyl (2-amino-5-chloro-6-iodoquinolin-3-yl)carbamate

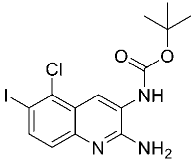

At room temperature, a 1 M solution of potassium tert-butoxide in tetrahydrofuran (50.34 mL, 50.34 mmol) was added into a solution of 6-amino-2-chloro-3-iodobenzaldehyde (10.9 g, 38.72 mmol) and N-bis(tert-butoxycarbonyl)-2-aminoacetonitrile (12.9 g, 50.34 mmol, intermediate 22) in tetrahydrofuran (200 mL), and the mixture was stirred overnight at room temperature under nitrogen protection. The reaction solution was filtered, and the filtrate was concentrated under reduced pressure and purified by column chromatography (petroleum ether/ethyl acetate = 5:1-1:1) to obtain the title compound as a yellow solid (1.7 g, yield: 10%). LC-MS: m/z [M+H]⁺ = 420/422.

### 3) Synthesis of 8-chloro-7-iodo-1,3-dihydro-2H-imidazo[4,5-blciuinolin-2-one

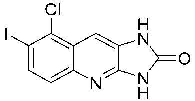

Potassium tert-butoxide (721.95 mg, 6.43 mmol) was added into a solution of tert-butyl (2-amino-5-chloro-6-iodoquinolin-3-yl)carbamate (900 mg, 2.14 mmol) in tetrahydrofuran (25 mL), and the mixture was stirred at 60°C for 16 hours. The reaction solution was adjusted with 1 N hydrochloric acid to make pH = 6 and then filtered. The filter cake was washed 3 times with water (15 mL), collected, concentrated under reduced pressure, and dried to obtain the title compound as a yellow solid (569 mg, yield: 77%). LC-MS: m/z [M+H]⁺ = 346.

### Intermediate 29. Synthesis of 6-bromo-5-chloro-1,3-dihydro-2H-imidazo[4,5-b]quinoxalin-2-one

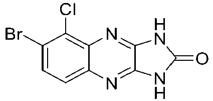

### 1) Synthesis of 4-bromo-3-chlorobenzene-1,2-diamine

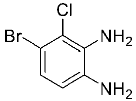

At room temperature, iron powder (2.22 g, 39.77 mmol) was added into ethanol (20 mL) and water (10 mL) of 3-bromo-2-chloro-6-nitroaniline (2000 mg, 7.95 mmol, CAS: 2091859-73-1) and ammonium chloride (4.25 g, 79.54 mmol), and the mixture was stirred at 80°C for 2 hours under nitrogen protection. The reaction solution was filtered, the filtrate was concentrated under reduced pressure, and the residue was dissolved in ethyl acetate (100 mL), washed 1 time with brine (30 mL), dried over anhydrous sodium sulfate, filtered, and concentrated under reduced pressure to obtain the title compound as a yellow solid (1300 mg, yield: 74%).

### 2) Synthesis of 6-bromo-5-chloro-1,4-dihydroquinoxalin-2,3-dione

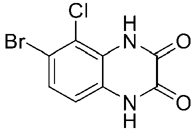

Under ice bath conditions, 4-bromo-3-chlorobenzene-1,2-diamine (700 mg, 3.18 mmol) and dimethyl oxalate (563 mg, 4.77 mmol) were slowly added into 4 M hydrochloric acid (2 mL), respectively. The mixture was stirred at room temperature for 5 minutes and then stirred at 80°C for 16 hours under nitrogen protection. The reaction solution was cooled to room temperature, and the brown filter cake obtained by filtration was washed 3 times with water (1 mL) and then dried. The dried solid was triturated with ethyl acetate (1 mL) and collected by filtration, and the filter cake was rinsed with ethyl acetate (0.5 mL) and then dried to obtain the title compound as a brown solid (800 mg, yield: 92%). LC-MS: m/z [M+H]⁺ = 275/277.

### 3) Synthesis of 6-bromo-2,3,5-trichloroquinoxaline

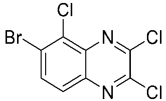

A suspension of 6-bromo-5-chloro-1,4-dihydroquinoxalin-2,3-dione (750 mg, 2.72 mmol) in phosphorus oxychloride (120 mL) was stirred overnight at 110°C under nitrogen protection. The reaction solution was directly concentrated under reduced pressure to obtain the title compound as a brown solid (850 mg, yield: 100%).

### 4) Synthesis of 6-bromo-5-chloroquinoxalin-2,3-diamine

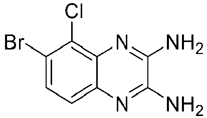

6-bromo-2,3,5-trichloroquinoxaline (800 mg, 2.56 mmol), ammonia water (5 mL), and tetrahydrofuran (10 mL) were added into a sealed tank, and the mixture was allowed to react overnight at 80°C. The reaction solution was cooled to room temperature, diluted with ethyl acetate (50 mL), washed 1 time with brine (30 mL), dried over anhydrous sodium sulfate, filtered, and concentrated under reduced pressure. Purification was performed by column chromatography (petroleum ether/ethyl acetate = 9:1-1:9) to obtain the title compound as a brown solid (140 mg, yield: 20%). LC-MS: m/z [M+H]⁺ = 273/275.

### 5) Synthesis of 6-bromo-5-chloro-1,3-dihydro-2H-imidazo[4,5-b]quinoxalin-2-one

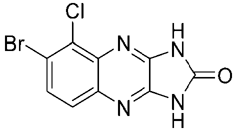

At room temperature, N,N'-carbonyldiimidazole (143 mg, 0.88 mmol) was added into a solution of 6-bromo-5-chloroquinoxalin-2,3-diamine (120 mg, 0.44 mmol) and N,N-diisopropylethylamine (0.29 mL, 1.76 mmol, d = 0.742 g/mL) in tetrahydrofuran (6 mL), and the mixture was stirred overnight at 60°C under nitrogen protection. The reaction solution was directly concentrated under reduced pressure, dissolved in N,N-dimethylformamide (3 mL), and then slowly added dropwise into water (30 mL). The precipitated solid was collected by filtration and then dried to obtain the title compound as a brown solid (120 mg, yield: 73%). ¹H NMR (400 MHz, DMSO-*d*₆) δ 12.19 (s, 2H), 7.83 (d, J = 8.9 Hz, 1H), 7.72 (d, J = 8.9 Hz, 1H); LC-MS: m/z [M+H]⁺ = 299/301.

### Intermediate 30. Synthesis of 1-(difluoromethyl)-4-iodo-3-(methoxy-d3)-1H-pyrazole

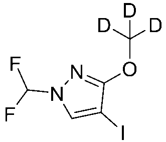

### 1) Synthesis of 1-acetyl-1,2-dihydro-3H-pyrazol-3-one

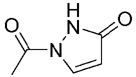

At room temperature, acetic anhydride (5.85 mL, 62.44 mmol) was slowly added into a solution of 1,2-dihydro-3H-pyrazol-3-one (5000 mg, 59.47 mmol, CAS: 137-45-1) in pyridine (35 mL), and the mixture was stirred at 90°C for 4 hours under nitrogen protection. The reaction solution was cooled to room temperature and concentrated under reduced pressure, and the residue was triturated with methyl tert-butyl ether (40 mL) to obtain the title compound as a white solid (6500 mg, yield: 87%). LC-MS: m/z [M+H]⁺ = 127.

### 2) Synthesis of 1-(3-(methoxy-d3)-1H-pyrazol-1-yl)ethan-1-one

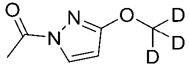

At room temperature, deuterated iodomethane (3.33 mL, 53.52 mmol, d = 2.330 g/mL) was added into a solution of 1-acetyl-1,2-dihydro-3H-pyrazol-3-one (4500 mg, 35.68 mmol) and potassium carbonate (7397.11 mg, 53.52 mmol) in N,N-dimethylformamide (40 mL), and the mixture was stirred at room temperature for 2 hours. The reaction solution was diluted with ethyl acetate (400 mL) and sequentially washed 1 time with water (150 mL) and 1 time with brine (150 mL). The organic phase was dried over anhydrous sodium sulfate, filtered, concentrated under reduced pressure, and purified by column chromatography (petroleum ether/ethyl acetate =50:1-10:1) to obtain the title compound as a colorless oil (2900 mg, yield: 57%). LC-MS: m/z [M+H]⁺ = 144.

### 3) Synthesis of 3-(methoxy-d3)-1H-pyrazole

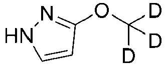

1-(3-(methoxy-d3)-1H-pyrazol-1-yl)ethan-1-one (2900 mg, 20.26 mmol) was slowly added into a mixed solution of potassium hydroxide (2841.56 mg, 50.64 mmol) in ethanol (30 ml) and water (15 ml), and the mixture was stirred at room temperature for 2 hours. The reaction solution was directly concentrated. The residue was dissolved in ethyl acetate (150 mL) and washed 1 time with brine (30 mL). The organic phase was dried over anhydrous sodium sulfate, filtered, and concentrated under reduced pressure to obtain the title compound as a colorless liquid (2000 mg, yield: 98%). LC-MS: m/z [M+H]⁺ = 102.

### 4) Synthesis of 4-iodo-3-(methoxy-d3)-1H-pyrazole

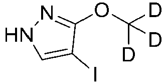

At 0-10°C, N-iodosuccinimide (4449.96 mg, 19.78 mmol) was added in batches into a solution of 3-(methoxy-d3)-1H-pyrazole (2000 mg, 19.78 mmol) in N,N-dimethylformamide (30 mL), and the mixture was stirred at room temperature for 2 hours under nitrogen protection. The reaction solution was poured into water (200 mL) and extracted 3 times with ethyl acetate (60 mL). The organic phases were combined, washed 1 time with brine (50 mL), dried over anhydrous sodium sulfate, filtered, concentrated under reduced pressure, and purified by column chromatography (petroleum ether/ethyl acetate = 10:1-5:1) to obtain the title compound as a colorless oil (3000 mg, yield: 67%). LC-MS: m/z [M+H]⁺ = 228.

### 5) Synthesis of 1-(difluoromethyl)-4-iodo-3-(methoxy-d3)-1H-pyrazole

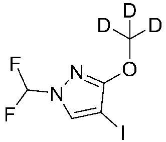

At 0°C, bromodifluoromethane diethyl phosphate (1450 mg, 5.43 mmol, CAS: 65094-22-6) was added into a mixed solution of 4-iodo-3-(methoxy-d3)-1H-pyrazole (1100 mg, 4.85 mmol), potassium hydroxide (800 mg, 14.26 mmol), water (10 mL), and acetonitrile (12 mL), and the mixture was allowed to react at 60°C for 15 hours. The reaction solution was cooled to room temperature and extracted 3 times with ethyl acetate (40 mL). The organic phases were combined, dried over anhydrous sodium sulfate, filtered, concentrated under reduced pressure, and purified by column chromatography (petroleum ether/dichloromethane = 3:1) to obtain the title compound as a yellow viscous liquid (240 mg, yield: 18.5%). LC-MS: m/z [M+H]⁺ = 278.

### Example 1 8-chloro-7-(p-tolyl)-1,3-dihydro-2H-imidazo[4,5-b]quinolin-2-one

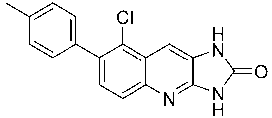

7-bromo-8-chloro-1,3-dihydro-2H-imidazo[4,5-b]quinolin-2-one (60 mg, 0.2 mmol, intermediate 1), cesium carbonate (128 mg, 0.4 mmol), and 4-methylphenylboronic acid (22.4 mg, 0.2 mmol) were dissolved in 1,4-dioxane (2 mL) and water (0.5 mL), dichloro[1,1'-bis(di-tert-butylphosphino)ferrocene]palladium (II) (8 mg, 0.02 mmol, CAS: 95408-45-0) was added, and the mixture was subjected to nitrogen replacement 3 times and stirred at 100°C for 12 hours under nitrogen protection. The reaction solution was filtered through Celite, the filter cake was washed with dichloromethane (10 mL × 3), and the filtrate was concentrated under reduced pressure and purified by reversed-phase chromatography (5%-95% acetonitrile and water) to obtain the title compound as a white solid (15 mg, yield: 24%). ¹H NMR (400 MHz, DMSO-*d*₆) δ 11.76 (s, 1H), 11.25 (s, 1H), 7.84 (d, J = 8 Hz, 1H), 7.82 (s, 1H), 7.51 (d, J = 8 Hz, 1H), 7.39 (d, J = 8 Hz, 2H), 7.31 (d, J = 8 Hz, 2H), 2.39 (s, 3H); LC-MS: m/z [M+H]⁺ = 310.

### Example 2 5-chloro-6-(p-tolyl)-1,3-dihydropyrrolo[2,3-blquinolin-2-one

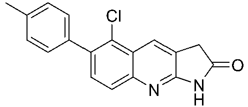

### 1) Synthesis of 4-amino-2-chloro-4'-methyl-[1,1'-biphenyl]-3-carbaldehyde

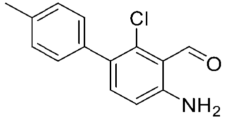

6-amino-3-bromo-2-chlorobenzaldehyde (500 mg, 2.12 mmol, synthesized according to a method provided in WO2019167000A1), p-methylphenylboronic acid (288 mg, 2.12 mmol), and sodium carbonate (449 mg, 4.24 mmol) were dissolved in 1,4-dioxane (8 mL) and water (2 mL), [1,1'-bis(diphenylphosphino)ferrocene]dichloropalladium (154 mg, 0.21 mmol) was added, nitrogen replacement was performed 3 times, and the reaction solution was heated to 90°C and left overnight. The reaction solution was cooled to room temperature, diluted with water (50 mL), and extracted twice with ethyl acetate (30 mL). The organic phases were combined, dried over anhydrous sodium sulfate, concentrated under reduced pressure, and purified by column chromatography (petroleum ether/ethyl acetate = 1:1) to obtain the title compound as a yellow solid (250 mg, yield: 48%). LC-MS: m/z [M+H]⁺ = 246.

### 2) Synthesis of 3-((4-amino-2-chloro-4'-methyl-[1,1'-biphenyl]-3-yl)methylene)-1-(4-methoxybenzyl)pyrrolidin-2,5-dione

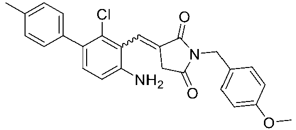

Diethyl (1-(4-methoxybenzyl)-2,5-dioxopyrrolidin-3-yl)phosphonate (652 mg, 1.84 mmol, intermediate 2) was dissolved in methanol (5 mL), sodium methoxide (151 mg, 2.76 mmol) was added, and the reaction solution was stirred at room temperature for 30 minutes. Then, 4-amino-2-chloro-4'-methyl-[1,1'-biphenyl]-3-carbaldehyde (220 mg, 0.92 mmol) was added, and the mixture was heated to 50°C and stirred overnight. The reaction solution was cooled to room temperature and then directly used in the next reaction without post-treatment. LC-MS: m/z [M+H]⁺ = 447.

### 3) Synthesis of 5-chloro-1-(4-methoxybenzyl)-6-(p-tolyl)-1,3-dihydro-2H-pyrrolo[2,3-b]quinolin-2-one

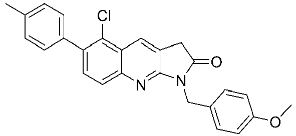

Acetic acid (5 mL) was added into the above reaction solution, the reaction mixture was stirred at room temperature for 2 hours, and the reaction solution was directly concentrated. A saturated sodium bicarbonate solution (20 mL) and ethyl acetate (20 mL) were added into the residue, the organic phase was separated, and the aqueous phase was extracted once with ethyl acetate (20 mL). The organic phases were combined, dried over anhydrous sodium sulfate, concentrated, and purified by column chromatography (petroleum ether/ethyl acetate = 2/1) to obtain the title compound as a white solid (60 mg, two-step yield: 15%). LC-MS: m/z [M+H]⁺ = 429.

### 4) Synthesis of 5-chloro-6-(p-tolyl)-1,3-dihydropyrrolo[2,3-b]quinolin-2-one

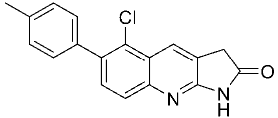

5-chloro-1-(4-methoxybenzyl)-6-(p-tolyl)-1,3-dihydro-2H-pyrrolo[2,3-b]quinolin-2-one (60 mg, 0.14 mmol) was dissolved in trifluoroacetic acid (5 mL), and the mixture was heated under a microwave to 150°C and stirred to carry out a reaction for 1 hour. The reaction solution was cooled to room temperature, then directly concentrated, dissolved in dimethyl sulfoxide (2 mL), and purified by preparative liquid chromatography (formic acid system) to obtain the title compound as a yellow solid (9 mg, yield: 21%). ¹H NMR (400 MHz, DMSO-d6) δ 11.43 (s, 1H), 8.36 (s, 1H), 7.80 (d, J = 8.6 Hz, 1H), 7.61 (d, J = 8.6 Hz, 1H), 7.40 (d, J = 8.0 Hz, 2H), 7.31 (d, J = 8.0 Hz, 2H), 3.77 (d, J = 1.6 Hz, 2H), 2.39 (s, 3H); LC-MS: m/z [M+H]⁺ = 309.

### Example 3 7-(2-chloro-4-fluorophenyl)-1,3-dihydro-2H-imidazo[4,5-b]quinolin-2-one

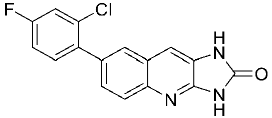

7-bromo-1,3-dihydro-2H-imidazo[4,5-b]quinolin-2-one (300.00 mg, 1.14 mmol, intermediate 3), 2-chloro-4-fluorophenoboronic acid (495.2 mg, 2.8 mmol, CAS: 313545-72-1), methanesulfonato(2-dicyclohexylphosphino-2',4',6'-tri-isopropyl-1,1'-biphenyl)(2'-amino-1,1'-biphenyl-2-yl) palladium (II) (48.1 mg, 0.06 mmol, CAS: 1445085-55-1), 2-dicyclohexylphosphino-2',4',6'-triisopropylbiphenyl (54.2 mg, 0.11 mmol, CAS: 564483-18-7), and potassium carbonate (392 mg, 2.8 mmol) were dissolved in a mixed solvent of 1,4-dioxane (3.2 mL) and water (0.8 mL), nitrogen replacement was performed 3 times, and the reaction solution was heated and stirred overnight at 85°C in a nitrogen atmosphere. The reaction solution was directly concentrated, purified by column chromatography (dichloromethane/methanol = 50:1) to obtain a white powder (131 mg), and then triturated with methyl tert-butyl ether (3 mL) to obtain the title compound as a white solid (44 mg, yield: 12%). ¹H NMR (400 MHz, DMSO-*d*₆) δ 11.59 (s, 1H), 11.11 (s, 1H), 7.93 (d, *J* = 1.6 Hz, 1H), 7.85 (d, *J=* 8.4 Hz, 1H), 7.67 (s, 1H), 7.60 (dd, *J* = 8.8, 2.4 Hz, 1H), 7.56 (dd, *J=* 8.4, 2.0 Hz, 2H), 7.35 (td, *J* = 8.4, 2.4 Hz, 1H); LC-MS: m/z [M+H]⁺ = 314.

### Example 4 7-(4-fluoro-2-methylphenyl)-1,3-dihydro-2H-imidazo[4,5-b]quinolin-2-one

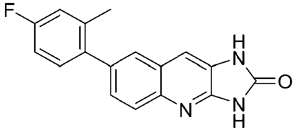

7-bromo-1,3-dihydro-2H-imidazo[4,5-b]quinolin-2-one (100 mg, 0.38 mmol, intermediate 3), 4-fluoro-2-methylphenylboronic acid (120 mg, 0.78 mmol, CAS: 139911-29-8), a complex of [1,1'-bis(diphenylphosphino)ferrocene]dichloropalladium and dichloromethane (32 mg, 0.039 mmol, CAS: 95464-05-4), and cesium carbonate (390 mg, 1.2 mmol) were dissolved in a mixed solvent of 1,4-dioxane (2 mL) and water (0.4 mL), nitrogen replacement was performed 3 times, and the reaction solution was heated and stirred overnight at 95°C in a nitrogen atmosphere. The reaction solution was cooled to room temperature, diluted with water (20 mL), and extracted twice with ethyl acetate (20 mL). The organic phases were combined, washed twice with water (30 mL), washed twice with brine (50 mL), dried over anhydrous sodium sulfate, concentrated under reduced pressure, and purified by preparative liquid chromatography (formic acid system) to obtain the title compound as a white solid (25 mg, yield: 22.5%). ¹H NMR (400 MHz, DMSO-*d*₆) δ 11.54 (s, 1H), 11.07 (s, 1H), 7.87 - 7.77 (m, 2H), 7.64 (s, 1H), 7.50 - 7.46 (m, 1H), 7.35-7.31 (m, 1H), 7.25 - 7.00 (m, 2H), 2.28 (s, 3H); LC-MS: m/z [M+H]⁺ = 294.

### Example 5 7-(4-fluoro-2-(trifluoromethylphenyl))-1,3-dihydro-2H-imidazo[4,5-b]quinolin-2-one

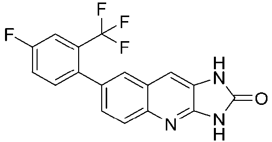

7-bromo-1,3-dihydro-2H-imidazo[4,5-b]quinolin-2-one (150 mg, 0.57 mmol, intermediate 3), 4-fluoro-2-trifluoromethylphenylboronic acid (236 mg, 1.14 mmol, CAS: 182344-16-7), methanesulfonato(2-dicyclohexylphosphino-2',4',6'-tri-isopropyl-1,1'-biphenyl)(2'-amino-1,1'-biphenyl-2-yl) palladium (II) (14.47 mg, 0.017 mmol, CAS: 1445085-55-1), 2-dicyclohexylphosphino-2',4',6'-triisopropylbiphenyl (16.21 mg, 0.034 mmol, CAS: 564483-18-7), and potassium carbonate (157 mg, 1,14 mmol) were dissolved in a mixed solvent of 1,4-dioxane (2 mL) and water (0.5 mL), nitrogen replacement was performed 3 times, and the reaction solution was heated and stirred at 80°C for 16 hours in a nitrogen atmosphere. The reaction solution was directly concentrated and purified by column chromatography (dichloromethane/methanol = 50:1-30:1) to obtain a solid, and then the solid was triturated with methanol (1 mL) to obtain the title compound as a white solid (74 mg, yield: 37%). ¹H NMR (400 MHz, DMSO-*d*₆) δ 11.60 (s, 1H), 11.12 (s, 1H), 7.85 - 7.81 (m, 2H), 7.78-7.75 (m, 1H), 7.68 - 7.61 (m, 2H), 7.58 - 7.54 (m, 1H), 7.45 - 7.42 (m, 1H); LC-MS: m/z [M+H]⁺ = 348.

### Example 6 7-(4-fluoro-2-methoxyphenyl)-1,3-dihydro-2H-imidazo[4,5-b]quinolin-2-one

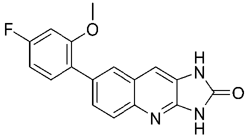

7-bromo-1,3-dihydro-2H-imidazo[4,5-b]quinolin-2-one (150 mg, 0.57 mmol, intermediate 3), 4-fluoro-2-methoxyphenylboronic acid (193 mg, 1.14 mmol, CAS: 179899-07-1), a complex of [1,1'-bis(diphenylphosphino)ferrocene]dichloropalladium and dichloromethane (41 mg, 0.05 mmol, CAS: 95464-05-4), and potassium carbonate (235 mg, 1.7 mmol) were dissolved in a mixed solvent of 1,4-dioxane (1.6 mL) and water (0.4 mL), nitrogen replacement was performed 3 times, and the reaction solution was heated and stirred overnight at 90°C in a nitrogen atmosphere. The reaction solution was cooled to room temperature, diluted with water (30 mL), and extracted twice with ethyl acetate (30 mL). The organic phases were combined, washed twice with water (30 mL), washed twice with brine (30 mL), dried over anhydrous sodium sulfate, concentrated under reduced pressure, and purified by column chromatography (dichloromethane/methanol = 10:1) to obtain the title compound as a white solid (50 mg, yield: 28%). ¹H NMR (400 MHz, DMSO-*d*₆) δ 11.52 (s, 1H), 11.05 (s, 1H), 7.92 (s, 1H), 7.78 (d, J = 8.4 Hz, 1H), 7.63 - 7.58 (m, 2H), 7.41 (t, J = 7.2 Hz, 1H), 7.06 (d, J = 10.8 Hz, 1H), 6.89 (t, J = 7.2 Hz, 1H), 3.81 (s, 3H); LC-MS: m/z [M+H]⁺ = 310.

### Example 7 7-(2-chloro-6-methoxypyridin-3-yl)-1,3-dihydro-2H-imidazo[4.5-b]quinolin-2-one

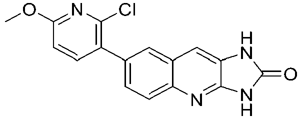

### 1) Synthesis of 7-(2-chloro-6-methoxypyridin-3-yl)-1,3-bis((2-(trimethylsilyl)ethoxy)methyl)-1,3-dihydro-2H-imidazo[4,5-b]quinolin-2-one

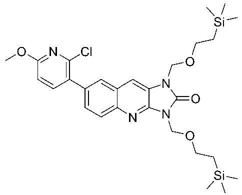

7-bromo-1,3-bis((2-(trimethylsilyl)ethoxy)methyl)-1,3-dihydro-2H-imidazo[4,5-b]quinolin-2-one (156 mg, 0.3 mmol, intermediate 4), 2-chloro-6-methoxypyridin-3-boronic acid (236 mg, 1.26 mmol, CAS: 1072946-25-8), [1,1'-bis(diphenylphosphino)ferrocene]dichloropalladium (40 mg, 0.055 mmol, CAS: 72287-26-4), and potassium carbonate (115 mg, 0.83 mmol) were dissolved in a mixed solvent of 1,4-dioxane (1 mL) and water (0.2 mL), nitrogen replacement was performed 3 times, and the reaction solution was stirred at 90°C for 2 hours in a nitrogen atmosphere. The reaction solution was cooled to room temperature, diluted with water (30 mL), and extracted twice with ethyl acetate (30 mL). The organic phases were combined, washed twice with water (30 mL), washed twice with brine (30 mL), dried over anhydrous sodium sulfate, concentrated under reduced pressure, and separated by column chromatography (petroleum ether/ethyl acetate = 5:1) to obtain the title compound as a colorless and transparent liquid (172 mg, yield: 98%). LC-MS: m/z [M+H]⁺ = 585.

### 2) Synthesis of 7-(2-chloro-6-methoxypyridin-3-yl)-1,3-dihydro-2H-imidazo[4,5-b]quinolin-2-one

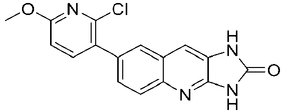

7-(2-chloro-6-methoxypyridin-3-yl)-1,3-bis((2-(trimethylsilyl)ethoxy)methyl)-1,3-dihydro-2H-imidazo [4,5-b]quinolin-2-one (172 mg, 0.29 mmol) was dissolved in trifluoroacetic acid (3 ml), and the mixture was allowed to react overnight at 90°C. The reaction solution was quenched with water (5 mL), concentrated under reduced pressure, and separated by column chromatography (dichloromethane/methanol = 50:1-30:1) to obtain a solid, and then the solid was triturated with methanol (1 mL) to obtain the title compound as a white solid (33 mg, yield: 34%). ¹H NMR (400 MHz, DMSO-d6) δ 11.59 (s, 1H), 11.11 (s, 1H), 7.95 (d, J = 2.0 Hz, 1H), 7.89 (d, J = 8.4 Hz, 1H), 7.85 (d, J = 8.4 Hz, 1H), 7.66 (s, 1H), 7.59 (dd, J = 8.8, 2.0 Hz, 1H), 6.99 (d, J = 8.4 Hz, 1H), 3.93 (s, 3H); LC-MS: m/z [M+H]⁺ = 325.

### Example 8 7-(4-chloro-6-methoxypyridin-3-yl)-1,3-dihydro-2H-imidazo[4,5-b]quinolin-2-one

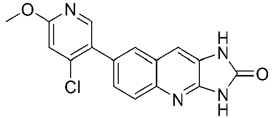

### 1) Synthesis of 7-(4-chloro-6-methoxypyridin-3-yl)-1,3-bis((2-(trimethylsilyl)ethoxy)methyl)-1,3-dihydro-2H-imidazo[4,5-b]quinolin-2-one

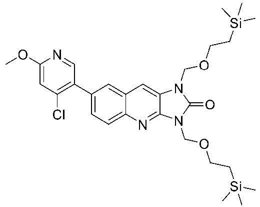

7-(4,4,5,5-tetramethyl-1,3,2-dioxaborolan-2-yl)-1,3-bis((2-(trimethylsilyl)ethoxy)methyl)-1,3-dihydro-2H-imidazo[4,5-b]quinolin-2-one (130 mg, 0.23 mmol, intermediate 5), 5-bromo-4-chloro-2-methoxypyridine (51 mg, 0.23 mmol, CAS: 851607-27-7), [1,1'-bis(diphenylphosphino)ferrocene]dichloropalladium (17 mg, 0.023 mmol, CAS: 72287-26-4), and potassium carbonate (78 mg, 0.56mmol) were dissolved in a mixed solvent of 1,4-dioxane (3 mL) and water (0.6 mL), nitrogen replacement was performed 3 times, and the reaction solution was stirred overnight at 90°C in a nitrogen atmosphere. The reaction solution was cooled to room temperature, diluted with water (20 mL), and extracted twice with ethyl acetate (20 mL). The organic phases were combined, washed twice with water (20 mL), washed twice with brine (20 mL), dried over anhydrous sodium sulfate, concentrated under reduced pressure, and separated by column chromatography (petroleum ether/ethyl acetate = 5:1) to obtain the title compound as a white solid (59 mg, yield: 44%). LC-MS: m/z [M+H]⁺ = 587.

### 2) Synthesis of 7-(4-chloro-6-methoxypyridin-3-yl)-1,3-dihydro-2H-imidazo[4,5-b]quinolin-2-one

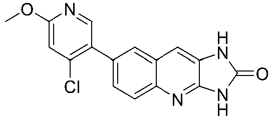

7-(4-chloro-6-methoxypyridin-3-yl)-1,3-bis((2-(trimethylsilyl)ethoxy)methyl)-1,3-dihydro-2H-imidazo [4,5-b]quinolin-2-one (49 mg, 0.08 mmol) was dissolved in trifluoroacetic acid (3.5 mL), and the mixture was allowed to react overnight at 90°C. The reaction solution was cooled to room temperature, diluted with water (20 mL), and extracted twice with ethyl acetate (20 mL). The organic phases were combined, washed twice with water (20 mL), washed twice with brine (20 mL), dried over anhydrous sodium sulfate, concentrated under reduced pressure, and separated by column chromatography (dichloromethane/methanol = 10:1) to obtain the title compound as a white solid (15 mg, yield: 55%). ¹H NMR (400 MHz, DMSO-d6) δ 11.60 (s, 1H), 11.12 (s, 1H), 8.30 (d, *J* = 7.2 Hz, 1H), 7.99 - 7.83 (m, 2H), 7.69 - 7.55 (m, 1H), 7.18 (d, *J* = 4.4 Hz, 1H), 6.54 (t, *J* = 7.2 Hz, 1H), 3.94 (s, 3H); LC-MS: m/z [M+H]⁺ = 327.

### Example 9 7-(4-fluorophenyl)-1,3-dihydro-2H-imidazo[4,5-b]quinolin-2-one

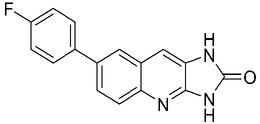

7-bromo-1,3-dihydro-2H-imidazo[4,5-b]quinolin-2-one (100 mg, 0.38 mmol, intermediate 3), 4-fluorobenzeneboronic acid (110 mg, 0.71 mmol, CAS: 1765-93-1), a complex of [1,1'-bis(diphenylphosphino)ferrocene]dichloropalladium and dichloromethane (32 mg, 0.039 mmol, CAS: 95464-05-4), and cesium carbonate (390 mg, 1.2 mmol) were dissolved in a mixed solvent of 1,4-dioxane (2 mL) and water (0.4 mL), nitrogen replacement was performed 3 times, and the reaction solution was heated and stirred overnight at 95°C in a nitrogen atmosphere. The reaction solution was cooled to room temperature, diluted with water (20 mL), and extracted twice with ethyl acetate (20 mL). The organic phases were combined, washed twice with water (30 mL), washed twice with brine (50 mL), dried over anhydrous sodium sulfate, concentrated under reduced pressure, and purified by preparative liquid chromatography (formic acid system) to obtain the title compound as a white solid (6 mg, yield: 6%). ¹H NMR (400 MHz, DMSO-*d*₆) δ 11.55 (s, 1H), 11.07 (s, 1H), 8.18 (s, 1H), 7.75 (brs, 4H), 7.66 (s, 1H), 7.34 (brs, 2H); LC-MS: m/z [M+H]⁺ = 280.

### Example 10 7-(3-chloro-5-fluoropyridin-2-yl)-1,3-dihydro-2H-imidazo[4,5-b]quinolin-2-one

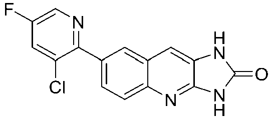

The title compound was synthesized by replacing 5-bromo-4-chloro-2-methoxypyridine (CAS: 851607-27-7) with 2-bromo-3-chloro-5-fluoropyridine (CAS: 1214326-94-9) using a method similar to that in Example 8. ¹H NMR (400 MHz, DMSO-d6) δ 11.62 (s, 1H), 11.13 (s, 1H), 8.74 (d, *J =* 2.4 Hz, 1H), 8.26 (dd, *J* = 8.8, 2.8 Hz, 1H), 8.20 (d, *J* = 2.0 Hz, 1H), 7.87 (d, *J =* 8.4 Hz, 1H), 7.78 (dd, *J =* 8.4, 2.0 Hz, 1H), 7.72 (s, 1H); LC-MS: m/z [M+H]⁺ = 315.

### Example 11 7-(1-(difluoromethyl)-1H-pyrazol-4-yl)-1,3-dihydro-2H-imidazo[4,5-b]quinolin-2-one

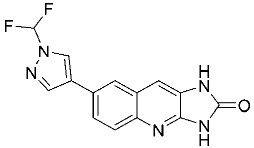

The title compound was synthesized by replacing 4-fluorobenzeneboronic acid (CAS: 1765-93-1) with (1-(difluoromethyl)-1H-pyrazol-4-yl)boronic acid (CAS: 1312693-57-4) and replacing the complex of [1,1'-bis(diphenylphosphino)ferrocene]dichloropalladium and dichloromethane (CAS: 95464-05-4) with a catalyst [1,1'-bis(di-tert-butylphosphino)ferrocene]dichloropalladium (CAS: 95408-45-0) using a method similar to that in Example 9. ¹H NMR (400 MHz, DMSO-d6) δ 11.56 (s, 1H), 11.06 (s, 1H), 8.78 (s, 1H), 8.35 (s, 1H), 8.21 (d, *J* = 2.0 Hz, 1H), 8.04 - 7.72 (m, 3H), 7.55 (s, 1H); LC-MS: m/z [M+H]⁺ = 302.

### Example 12 7-(3-chloro-1-(difluoromethyl)-1H-pyrazol-4-yl)-1,3-dihydro-2H-imidazo[4,5-b]quinolin-2-one

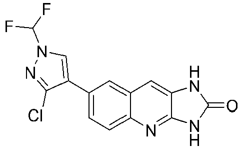

### Example 13 7-(5-chloro-1-(difluoromethyl)-1H-pyrazol-4-yl)-1,3-dihydro-2H-imidazo[4,5-b]quinolin-2-one

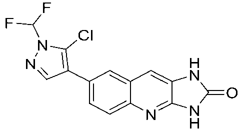

The 2 title compounds were synthesized by replacing 5-bromo-4-chloro-2-methoxypyridine (CAS: 851607-27-7) with a mixture of 3-chloro-1-(difluoromethyl)-4-iodo-1H-pyrazole and 5-chloro-1-(difluoromethyl)-4-iodo-1H-pyrazole (intermediate 6) using a method similar to that in Example 8, and finally purified by preparative high performance liquid chromatography (24%-54%(v/v) acetonitrile and water, 0.1% formic acid), respectively. Example 12: ¹H NMR (400 MHz, DMSO-d6) δ 11.59 (s, 1H), 11.13 (s, 1H), 8.76 (s, 1H), 8.16 (d, J = 2.0 Hz, 1H), 8.02 - 7.70 (m, 3H), 7.65 (s, 1H); LC-MS: m/z [M+H]⁺ = 336. Example 13: ¹H NMR (400 MHz, DMSO-d6) δ 11.52 (s, 1H), 11.23 (s, 1H), 8.37 (s, 1H), 8.20 (d, J = 2.0 Hz, 1H), 8.18 - 7.83 (m, 2H), 7.80 (dd, J = 8.4, 2.0 Hz, 1H), 7.65 (s, 1H); LC-MS: m/z [M+H]⁺ = 336.

### Example 14 7-(2-chloro-4-cyanophenyl)-1,3-dihydro-2H-imidazo[4,5-b]quinolin-2-one

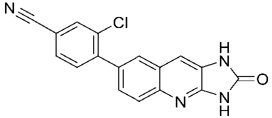

The title compound was synthesized by replacing 5-bromo-4-chloro-2-methoxypyridine (CAS: 851607-27-7) with 2-chloro-4-cyanophenylboronic acid (CAS: 677743-50-9) using a method similar to that in Example 8. ¹H NMR (400 MHz, DMSO-d6) δ 11.63 (s, 1H), 11.16 (s, 1H), 8.23 (s, 1H), 8.10 - 7.80 (m, 3H), 7.75 - 7.60 (m, 3H); LC-MS: m/z [M+H]⁺ = 319.

### Example 15 7-(4-chloro-1-(difluoromethyl)-1H-pyrazol-3-yl)-1,3-dihydro-2H-imidazo[4,5-b]quinolin-2-one

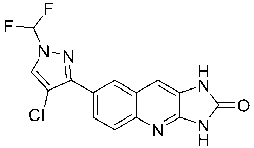

### Example 16 7-(4-chloro-1-(difluoromethyl)-1H-pyrazol-5-yl)-1,3-dihydro-2H-imidazo[4,5-b]quinolin-2-one

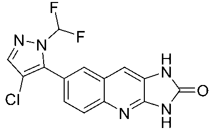

The 2 title compounds were synthesized by replacing 5-bromo-4-chloro-2-methoxypyridine (CAS: 851607-27-7) with a mixture of 3-bromo-4-chloro-1-(difluoromethyl)-1H-pyrazole and 5-bromo-4-chloro-1-(difluoromethyl)-1H-pyrazole (intermediate 7) using a method similar to that in Example 8, and finally purified by preparative high performance liquid chromatography (32%-62%(v/v) acetonitrile and water, 10 mM ammonium bicarbonate), respectively. Example 15: ¹H NMR (400 MHz, DMSO-d6) δ 11.40 (s, 2H), 8.74 (s, 1H), 8.40 (d, *J =* 2.0 Hz, 1H), 8.04 - 7.71 (m, 4H); LC-MS: m/z [M+H]⁺ = 336. Example 16: ¹H NMR (400 MHz, DMSO-d6) δ 11.71 (s, 1H), 11.22 (s, 1H), 8.13 (s, 1H), 8.05 (d, *J* = 2.0 Hz, 1H), 7.94 (d, *J =* 8.7 Hz, 1H), 7.84 - 7.54 (m, 3H); LC-MS: m/z [M+H]⁺ = 336.

### Example 17 7-(2-chloro-6-(trifluoromethyl)pyridin-3-yl)-1,3-dihydro-2H-imidazo[4,5-b]quinolin-2-one

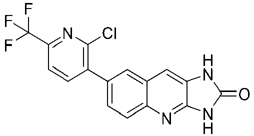

The title compound was synthesized by replacing 4-fluorobenzeneboronic acid (CAS: 1765-93-1) with (2-chloro-6-(trifluoromethyl)pyridin-3-yl)boronic acid (CAS: 205240-63-7) and replacing the complex of [1,1'-bis(diphenylphosphino)ferrocene]dichloropalladium and dichloromethane (CAS: 95464-05-4) with a catalyst [1,1'-bis(di-tert-butylphosphino)ferrocene]dichloropalladium (CAS: 95408-45-0) using a method similar to that in Example 9. ¹H NMR (400 MHz, DMSO-d6) δ 11.66 (s, 1H), 11.18 (s, 1H), 8.27 (d, J = 7.8 Hz, 1H), 8.12 - 8.06 (m, 2H), 7.90 (d, J = 8.6 Hz, 1H), 7.71 - 7.65 (m, 2H); LC-MS: m/z [M+H]⁺ = 365.

### Example 18 7-(2,6-dichloropyridin-3-yl)-1,3-dihydro-2H-imidazo[4,5-b]quinolin-2-one

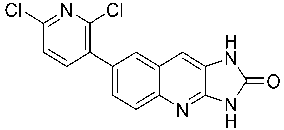

The title compound was synthesized by replacing 5-bromo-4-chloro-2-methoxypyridine (CAS: 851607-27-7) with 3-bromo-2,6-dichloropyridine (CAS: 866755-20-6) using a method similar to that in Example 8. ¹H NMR (400 MHz, DMSO-d6) δ 8.05 (d, J = 8.0 Hz, 1H), 8.01 (d, J = 2.0 Hz, 1H), 7.88 (d, J = 8.4 Hz, 1H), 7.71 (d, J = 8.0 Hz, 1H), 7.67 (s, 1H), 7.63 (dd, J = 8.8, 2.0 Hz, 1H); LC-MS: m/z [M+H]⁺ = 331.

### Example 19 7-(6-amino-2-chloropyridin-3-yl)-1,3-dihydro-2H-imidazo[4,5-b]quinolin-2-one

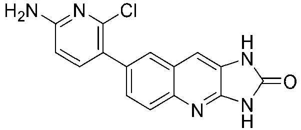

The title compound was synthesized by replacing 5-bromo-4-chloro-2-methoxypyridine (CAS: 358672-65-8) with 2-chloro-3-bromo-6-aminopyridine (CAS: 866755-20-6) using a method similar to that in Example 8. ¹H NMR (400 MHz, DMSO-d6) δ 11.27 (s, 2H), 7.86 (d, J = 2.0 Hz, 1H), 7.79 (d, J = 8.8 Hz, 1H), 7.63 (s, 1H), 7.57 - 7.50 (m, 2H), 6.53 (d, J = 8.0 Hz, 1H), 6.49 (s, 2H); LC-MS: m/z [M+H]⁺ = 312.

### Example 20 7-phenyl-1,3,5,6,7,8-hexahydro-2H-imidazo[4,5-b][1,6]naphthyridin-2-one

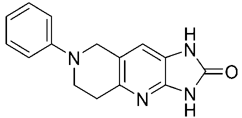

### 1) Synthesis of N²,N³,N³-tri(4-methoxybenzyl)-6-phenyl-5,6,7,8-tetrahydro-1,6-naphthyridin-2,3-diamine

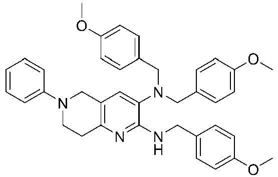

N²,N³,N³-tri(4-methoxybenzyl)-5,6,7,8-tetrahydro-1,6-naphthyridin-2,3-diamine (550 mg, 1.05 mmol, intermediate 8), bromobenzene (165.70 µL, 1.57 mmol, d = 1.491 g/mL), and cesium carbonate (683.11 mg, 2.10 mmol) were added into tertiary butanol (30 mL), and then 2-dicyclohexylphosphino-2',6'-diisopropoxy-1,1'-biphenyl (146.76 mg, 0.31 mmol, CAS: 787618-22-8) and methanesulfonato(2-dicyclohexylphosphino-2',4',6'-tri-isopropyl-1,1'-biphenyl)(2'-amino-1,1'-biphenyl-2-yl) palladium (II) (267.44 mg, 0.31 mmol, CAS: 1599466-85-9) were added. The mixture was degassed, subjected to nitrogen replacement 3 times, and stirred at 100°C for 18 hours in a nitrogen atmosphere. The reaction solution was filtered through Celite, the filter cake was washed 3 times with ethyl acetate (20 mL), and the filtrate was concentrated under reduced pressure and purified by column chromatography (petroleum ether/ethyl acetate = 10:1-2:1) to obtain the title compound as a yellow solid (350 mg, yield: 55%). LC-MS: m/z [M+H]⁺ = 601.

### 2) Synthesis of 6-phenyl-5,6,7,8-tetrahydro-1,6-naphthyridin-2,3-diamine

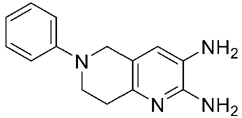

Trifluoroacetic acid (2 mL, 26 mmol, d = 1.489 g/mL) was added into a solution of N²,N³,N³-tri(4-methoxybenzyl)-6-phenyl-5,6,7,8-tetrahydro-1,6-naphthyridin-2,3-diamine (350 mg, 0.58 mmol) in dichloromethane (2 mL), and the mixture was stirred at 25°C for 18 hours. The reaction solution was directly concentrated under reduced pressure and purified by preparative liquid chromatography to obtain the title compound as a yellow solid (78 mg, yield: 56%). LC-MS: m/z [M+H]⁺ = 241.

### 3) Synthesis of 7-phenyl-1,3,5,6,7,8-hexahydro-2H-imidazo[4,5-b][1,6]naphthyridin-2-one

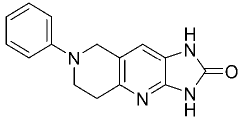

N,N'-carbonyldiimidazole (233.50 mg, 1.44 mmol) was added into a solution of 6-phenyl-5,6,7,8-tetrahydro-1,6-naphthyridin-2,3-diamine (58 mg, 0.24 mmol) and triethylamine (1.5 mL, 10.8 mmol, d = 0.728 g/mL) in N,N-dimethylformamide (3 mL), and the mixture was stirred at 60°C for 18 hours. The reaction solution was purified by preparative liquid chromatography to obtain the title compound as a yellow solid (20 mg, yield: 31%). ¹H NMR (400 MHz, DMSO-d6) δ 11.14 (s, 1H), 10.72 (s, 1H), 7.22 (s, 2H), 7.08 (s, 1H), 7.02 (s, 2H), 6.75 (s, 1H), 4.36 (s, 2H), 3.59 (s, 2H), 2.88 (s, 2H); LC-MS: m/z [M+H]⁺ = 267.

### Example 21 7-(2-chloro-4-fluorophenyl)-1,3,5,6,7,8-hexahydro-2H-imidazo[4,5-b][1,6]naphthyridin-2-one

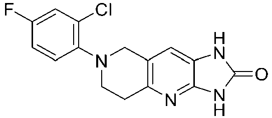

The title compound was synthesized by replacing bromobenzene with 1-bromo-2-chloro-4-fluorobenzene (CAS: 110407-59-5), replacing methanesulfonato(2-dicyclohexylphosphino-2',4',6'-triisopropyl-1,1'-biphenyl)(2'-amino-1,1'-biphenyl-2-yl) palladium (II) (CAS: 1599466-85-9) with a catalyst methanesulfonato{dicyclohexyl(3-isopropoxy-2',4',6'-triisopropyl-[1,1'-biphenyl]-2-yl)phosphine}(2'-methylamino-1,1'-biphenyl-2-yl)palladium (II) (CAS: 2132978-44-8), and replacing 2-dicyclohexylphosphino-2',6'-diisopropoxy-1,1'-biphenyl (CAS: 787618-22-8) with dicyclohexyl(3-isopropoxy-2',4',6'-triisopropyl-[1,1'-biphenyl]-2-yl)phosphine (CAS: 2118959-55-8) using a method similar to that in Example 20. ¹H NMR (400 MHz, DMSO-d6) δ 11.14 (s, 1H), 10.71 (s, 1H), 7.45 (dd, J = 8.4, 2.8 Hz, 1H), 7.28 (dd, J = 9.2, 5.6 Hz, 1H), 7.23-7.18 (m, 1H), 7.01 (s, 1H), 4.14 (s, 2H), 3.30 (t, J = 5.8 Hz, 2H), 2.92 (t, J = 5.8 Hz, 2H); LC-MS: m/z [M+H]⁺ = 319.

### Example 22 7-(2-chloro-4-cyanophenyl)-1,3,5,6,7,8-hexahydro-2H-imidazo[4,5-b][1,6]naphthyridin-2-one

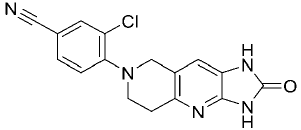

### 1) Synthesis of N²,N³,N³-tri(4-methoxybenzyl)-6-(2-chloro-4-cyanophenyl)-5,6,7,8-tetrahydro-1,6-naphthyridin-2,3-diamine

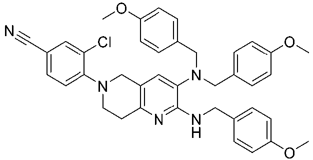

3-chloro-4-fluorobenzonitrile (160 mg, 1.02 mmol, CAS: 117482-84-5) and cesium carbonate (600 mg, 1.84 mmol) were added into a solution of N²,N³,N³-tri(4-methoxybenzyl)-5,6,7,8-tetrahydro-1,6-naphthyridin-2,3-diamine (400 mg, 0.76 mmol, intermediate 8) in N,N-dimethylformamide (10 mL). After nitrogen replacement was performed three times, the mixture was stirred overnight at 100°C under nitrogen protection. The reaction solution was cooled to room temperature, then poured into water (50 mL), and extracted 3 times with ethyl acetate (20 mL). The combined organic phases were washed 3 times with saline water (30 mL), dried over anhydrous sodium sulfate, filtered, concentrated under reduced pressure, and purified by column chromatography (petroleum ether/ethyl acetate = 7:1-4:1) to obtain the title compound as a yellow oil (100 mg, yield: 20%). LC-MS: m/z [M+H]⁺ = 660.

### 2) Synthesis of 6-(2-chloro-4-cyanophenyl)-5,6,7,8-tetrahydro-1,6-naphthyridin-2,3-diamine

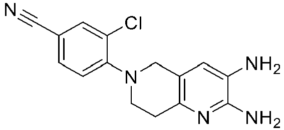

Trifluoroacetic acid (0.5 mL, 6.5 mmol, d = 1.489 g/mL) was added into a solution of N²,N³,N³-tri(4-methoxybenzyl)-6-(2-chloro-4-cyanophenyl)-5,6,7,8-tetrahydro-1,6-naphthyridin-2,3-diamine (100 mg, 0.15 mmol) in dichloromethane (1.5 mL), and the mixture was stirred at room temperature for 3 hours. The reaction solution was directly concentrated under reduced pressure to obtain the title compound (40 mg of a crude product, which was directly used in the next step without further purification). LC-MS: m/z [M+H]⁺ = 300.

### 3) Synthesis of 7-(2-chloro-4-cyanophenyl)-1,3,5,6,7,8-hexahvdro-2H-imidazo[4,5-b][1,6]naphthyridin-2-one

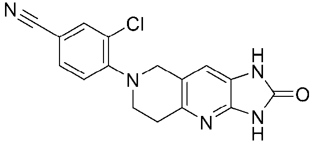

N,N'-disuccinimidyl carbonate (200 mg, 0.78 mmol, CAS: 74124-79-1) was added into a solution of 6-(2-chloro-4-cyanophenyl)-5,6,7,8-tetrahydro-1,6-naphthyridin-2,3-diamine (40 mg of a crude product) and triethylamine (0.5 mL, 3.6 mmol, d = 0.728 g/mL) in N,N-dimethylformamide (2 mL), and the mixture was stirred overnight at room temperature. The reaction solution was poured into water (10 mL) and extracted 3 times with ethyl acetate (5 mL). The combined organic phases were washed 3 times with saline water (5 mL), dried over anhydrous sodium sulfate, filtered, concentrated under reduced pressure, and purified by preparative liquid chromatography to obtain the title compound as a light yellow solid (6.6 mg, two-step yield: 13.5%). ¹H NMR (400 MHz, DMSO-d6) δ 11.18 (s, 1H), 10.74 (s, 1H), 7.99 (d, J = 2.0 Hz, 1H), 7.78 (dd, J = 8.4, 2.0 Hz, 1H), 7.31 (d, J = 8.4 Hz, 1H), 7.05 (s, 1H), 4.33 (s, 2H), 3.52 (t, J = 6.0 Hz, 2H), 2.95 (t, J = 5.8 Hz, 2H); LC-MS: m/z [M+H]⁺ = 326.

### Example 23 7-(3-chloro-5-fluoropyridin-2-yl)-1,3,6,7,8-hexahydro-2H-imidazo[4,5-b][1,6]naphthyridin-2-one

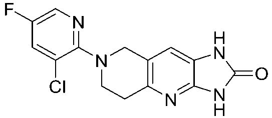

The title compound was synthesized by replacing bromobenzene with 2-bromo-3-chloro-5-fluoropyridine (CAS: 1214326-94-9) using a method similar to that in Example 20. ¹H NMR (400 MHz, DMSO-d6) δ 11.14 (s, 1H), 10.71 (s, 1H), 8.30 (d, J = 2.8 Hz, 1H), 8.05 (dd, J = 8.0, 2.8 Hz, 1H), 7.07 (s, 1H), 4.39 (s, 2H), 3.55 (t, J = 5.6 Hz, 2H), 2.96 (t, J = 5.6 Hz, 2H); LC-MS: m/z [M+H]⁺ = 320.

### Example 24 8-chloro-7-(5-fluoropyridin-2-yl)-1,3-dihydro-2H-imidazo[4,5-b]quinolin-2-one

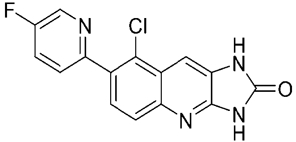

### 1) Synthesis of 8-chloro-7-(5-fluoropyridin-2-yl)-1,3-bis((2-(trimethylsilyl)ethoxy)methyl)-1,3-dihydro-2H-imidazo[4,5-b] quinolin-2-one

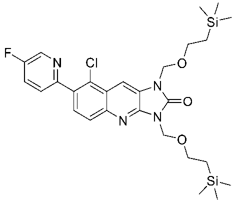

8-chloro-7-(4,4,5,5-tetramethyl-1,3,2-dioxaborolan-2-yl)-1,3-bis((2-(trimethylsilyl)ethoxy)methyl)-1,3-dihydro-2H-imidazo[4,5-b]quinolin-2-one (210 mg, 0.35 mmol, intermediate 9), 2-bromo-5-fluoropyridine (140 mg, 0.80 mmol, CAS: 41404-58-4), [1,1'-bis(di-tert-butylphosphino)ferrocene]dichloropalladium (60 mg, 0.09 mmol, CAS: 95408-45-0), and cesium carbonate (310 mg, 0.95 mmol) were dissolved in a mixed solvent of 1,4-dioxane (5 mL) and water (0.5 mL), nitrogen replacement was performed 3 times, and the reaction solution was stirred at 100°C for 3 hours in a nitrogen atmosphere. The reaction solution was cooled to room temperature, diluted with water (20 mL), and extracted twice with ethyl acetate (20 mL). The organic phases were combined, washed twice with water (20 mL), washed twice with brine (20 mL), dried over anhydrous sodium sulfate, concentrated under reduced pressure, and separated by column chromatography (petroleum ether/ethyl acetate = 6:1) to obtain the title compound as a white solid (125 mg, yield: 63%). LC-MS: m/z [M+H]⁺ = 575.

### 2) Synthesis of 8-chloro-7-(5-fluoropyridin-2-yl)-1,3-dihydro-2H-imidazo[4,5-b]quinolin-2-one

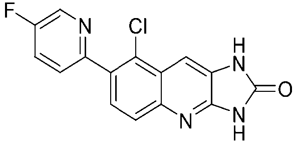

8-chloro-7-(5-fluoropyridin-2-yl)-1,3-bis((2-(trimethylsilyl)ethoxy)methyl)-1,3-dihydro-2H-imidazo[4,5-b] quinolin-2-one (100 mg, 0.17 mmol) was dissolved in trifluoroacetic acid (2 mL) to carry out a reaction under a microwave at 130°C for 1 hour. The reaction solution was directly concentrated, the residue was dissolved in dichloromethane (1 mL), methanol (2 mL), and ammonia water (1 mL), and the reaction mixture was allowed to react at 20°C for 1 hour. The reaction solution was directly concentrated and purified by preparative liquid chromatography (acetonitrile and water containing 0.1% formic acid) to obtain the title compound as a white solid (31.1 mg, yield: 57%). ¹H NMR (400 MHz, DMSO-d6) δ 11.82 (s, 1H), 11.34 (s, 1H), 8.74 (d, J = 2.8 Hz, 1H), 7.93 - 7.80 (m, 4H), 7.67 (d, J = 8.4 Hz, 1H); LC-MS: m/z [M+H]⁺ = 315.

### Example 25 7-(2-chloro-4-methylophenyl)-1,3-dihydro-2H-imidazo[4,5-b]quinolin-2-one

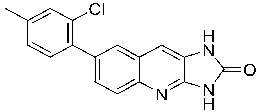

The title compound was synthesized by replacing 4-fluorophenylboronic acid (CAS: 1765-93-1) with 2-chloro-4-methylphenylboronic acid (CAS: 145349-62-8) using a method similar to that in Example 9. ¹H NMR (400 MHz, DMSO-d6) δ 11.56 (s, 1H), 11.09 (s, 1H), 7.91 (d, J = 2.0 Hz, 1H), 7.83 (d, J = 8.6 Hz, 1H), 7.66 (s, 1H), 7.56 (dd, J = 8.6, 2.0 Hz, 1H), 7.43 (s, 1H), 7.39 (d, J = 7.8 Hz, 1H), 7.27 (d, J = 7.8 Hz, 1H), 2.38 (s, 3H); LC-MS: m/z [M+H]⁺ = 310.

### Example 26 7-(2-aminopyridin-4-yl)-1,3-dihydro-2H-imidazo[4,5-b]quinolin-2-one

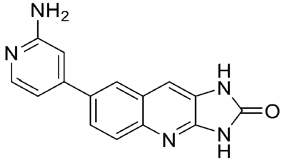

The title compound was synthesized by replacing 4-fluorophenylboronic acid (CAS: 1765-93-1) with 2-aminopyridin-4-boronic acid (CAS: 903513-62-2) and replacing the complex of [1,1'-bis(diphenylphosphino)ferrocene]dichloropalladium and dichloromethane (CAS: 95464-05-4) with a catalyst tris(dibenzylideneacetone)dipalladium (CAS: 51364-51-3) and a ligand 1,3,5,7-tetramethyl-6-phenyl-2,4,8-trioxa-6-phosphaadamante (CAS: 97739-46-3) using a method similar to that in Example 9. ¹H NMR (400 MHz, DMSO-d6) δ 11.60 (s, 1H), 11.12 (s, 1H), 8.23 - 8.16 (m, 2H), 8.00 (d, J = 5.4 Hz, 1H), 7.86 (d, J = 8.7 Hz, 1H), 7.77 (dd, J = 8.7, 2.1 Hz, 1H), 7.70 (s, 1H), 6.90 (dd, J = 5.4, 1.6 Hz, 1H), 6.81 (d, J = 1.7 Hz, 1H), 6.00 (s, 2H); LC-MS: m/z [M+H]⁺ = 278.

### Example 27 7-(2-chloro-6-(methoxy-d3)pyridin-3-yl)-1,3-dihydro-2H-imidazo[4,5-b]quinolin-2-one

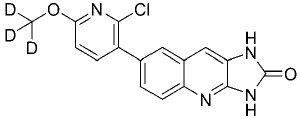

### Example 28 7-(6-chloro-2-(methoxy-d3)pyridin-3-yl)-1,3-dihydro-2H-imidazo[4,5-b]quinolin-2-one

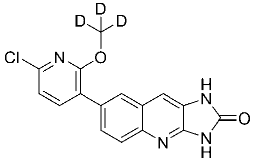

The 2 title compounds were synthesized by replacing 5-bromo-4-chloro-2-methoxypyridine (CAS: 851607-27-7) with a mixture of 3-bromo-2-chloro-6-(methoxy-d3)pyridine and 3-bromo-6-chloro-2-(methoxy-d3)pyridine (intermediate 10) using a method similar to that in Example 8, and finally purified by preparative high performance liquid chromatography (32%-62%(v/v) acetonitrile and water, 10 mM ammonium bicarbonate), respectively. Example 27: ¹H NMR (400 MHz, DMSO-d6) δ 11.38 (s, 2H), 8.05 (d, J = 2.0 Hz, 1H), 7.90 (d, J = 7.6 Hz, 1H), 7.82 (d, J = 8.4 Hz, 1H), 7.70 (dd, J = 8.4, 2.0 Hz, 1H), 7.64 (s, 1H), 7.23 (d, J = 7.6 Hz, 1H); LC-MS: m/z [M+H]⁺ =330. Example 28: ¹H NMR (400 MHz, DMSO-d6) δ 11.54 (s, 1H), 11.13 (s, 1H), 7.94 (d, J = 2.0 Hz, 1H), 7.89 (d, J = 8.0 Hz, 1H), 7.84 (d, J = 8.4 Hz, 1H), 7.65 (s, 1H), 7.58 (dd, J = 8.4, 2.0 Hz, 1H), 6.98 (d, J = 8.0 Hz, 1H); LC-MS: m/z [M+H]⁺ = 330.

### Example 29 7-(1-(trifluoromethyl)-1H-pyrazol-4-yl)-1,3-dihydro-2H-imidazo[4,5-b]quinolin-2-one

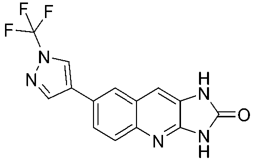

The title compound was synthesized by replacing 4-fluorobenzeneboronic acid (CAS: 1765-93-1) with 4-(4,4,5,5-tetramethyl-1,3,2-dioxaborolan-2-yl)-1-(trifluoromethyl)-1H-pyrazole (CAS: 1046831-98-4) and replacing the complex of [1,1'-bis(diphenylphosphino)ferrocene]dichloropalladium and dichloromethane (CAS: 95464-05-4) with a catalyst [1,1'-bis(di-tert-butylphosphino)ferrocene]dichloropalladium (CAS: 95408-45-0) using a method similar to that in Example 9. ¹H NMR (400 MHz, DMSO-d6) δ 11.56 (s, 1H), 11.05 (s, 1H), 9.04 (s, 1H), 8.54 (s, 1H), 8.26 (s, 1H), 7.89 (d, *J=* 8.0 Hz, 1H), 7.82 (d, *J=* 8.4 Hz, 1H), 7.53 (s, 1H); LC-MS: m/z [M+H]⁺ = 320.

### Example 30 7-(1-(difluoromethyl)-5-(trifluoromethyl)-1H-pyrazol-4-yl)-1,3-dihydro-2H-imidazo[4,5-b]quinolin-2-one

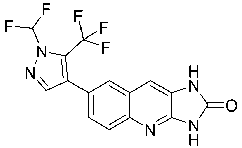

### Example 31 7-(1-(difluoromethyl)-3-(trifluoromethyl)-1H-pyrazol-4-yl)-1,3-dihydro-2H-imidazo[4,5-b]quinolin-2-one

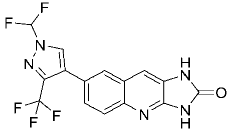

The 2 title compounds were synthesized by replacing 5-bromo-4-chloro-2-methoxypyridine (CAS: 851607-27-7) with a mixture of 1-(difluoromethyl)-4-iodo-5-(trifluoromethyl)-1H-pyrazole and 1-(difluoromethyl)-4-iodo-3-(trifluoromethyl)-1H-pyrazole (intermediate 11) using a method similar to that in Example 8, and finally purified by a chiral column, respectively. Example 30: ¹H NMR (400 MHz, DMSO-d6) δ 11.63 (s, 1H), 11.14 (s, 1H), 8.23 (s, 1H), 8.14 (t, J = 57.6 Hz, 1H), 7.98 (d, J = 2.0 Hz, 1H), 7.86 (d, J = 8.4 Hz, 1H), 7.66 (s, 1H), 7.53 (dd, J = 8.4, 2.0 Hz, 1H); LC-MS: m/z [M+H]⁺ =370. Example 31: ¹H NMR (400 MHz, DMSO-d6) δ 11.62 (s, 1H), 11.13 (s, 1H), 8.83 (s, 1H), 8.21 - 7.78 (m, 3H), 7.64 (s, 1H), 7.57 (dd, J = 8.4, 2.0 Hz, 1H); LC-MS: m/z [M+H]⁺ = 370.

### Example 32 7-(5-bromo-1-(difluoromethyl)-1H-pyrazol-4-yl)-1,3-dihydro-2H-imidazo[4,5-b]quinolin-2-one

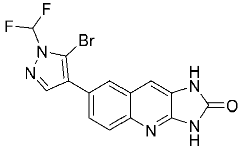

### Example 33 7-(3-bromo-1-(difluoromethyl)-1H-pyrazol-4-yl)-1,3-dihydro-2H-imidazo[4,5-b]quinolin-2-one

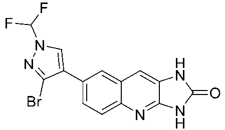

The 2 title compounds were synthesized by replacing 5-bromo-4-chloro-2-methoxypyridine (CAS: 851607-27-7) with a mixture of 5-bromo-1-(difluoromethyl)-4-iodo-1H-pyrazole and 3-bromo-1-(difluoromethyl)-4-iodo-1H-pyrazole (intermediate 12) using a method similar to that in Example 8, and finally purified by a chiral column, respectively. Example 32: ¹H NMR (400 MHz, DMSO-d6) δ 11.60 (s, 1H), 11.11 (s, 1H), 8.28 (s, 1H), 8.17 - 7.83 (m, 3H), 7.77 (dd, J *=* 8.8, 2.1 Hz, 1H), 7.64 (s, 1H); LC-MS: m/z [M+H]⁺ =380/382. Example 33: ¹H NMR (400 MHz, DMSO-d6) δ 11.59 (s, 1H), 11.11 (s, 1H), 8.66 (s, 1H), 8.12 (d, J = 2.0 Hz, 1H), 8.05 - 7.68 (m, 3H), 7.64 (s, 1H); LC-MS: m/z [M+H]⁺ = 380/382.

### Example 34 7-(4-chloro-3-(difluoromethyl)isoxazol-5-yl)-1,3-dihydro-2H-imidazo[4,5-b]quinolin-2-one

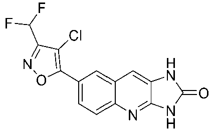

The title compound was synthesized by replacing 5-bromo-4-chloro-2-methoxypyridine (CAS: 851607-27-7) with 5-bromo-4-chloro-3-(difluoromethyl)isoxazole (intermediate 13) using a method similar to that in Example 8. ¹H NMR (400 MHz, DMSO-d6) δ 11.79 (s, 1H), 11.28 (s, 1H), 8.61 (d, J = 2.1 Hz, 1H), 8.07 (dd, J = 8.8, 2.1 Hz, 1H), 7.98 (d, J = 8.8 Hz, 1H), 7.84 (s, 1H), 7.45 (t, J = 51.7 Hz, 1H); LC-MS: m/z [M+H]⁺ = 337.

### Example 35 7-(2-chloro-6-(difluoromethyl)pyridin-3-yl)-1,3-dihydro-2H-imidazo[4,5-b]quinolin-2-one

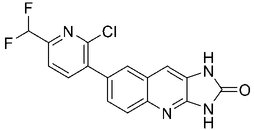

The title compound was synthesized by replacing 5-bromo-4-chloro-2-methoxypyridine (CAS: 851607-27-7) with 3-bromo-2-chloro-6-(difluoromethyl)pyridine (CAS: 1805221-46-8) using a method similar to that in Example 8. ¹H NMR (400 MHz, DMSO-d6) δ 11.07 (s, 2H), 8.18 (d, J = 7.6 Hz, 1H), 8.05 (s, 1H), 7.89 (d, J = 8.8 Hz, 1H), 7.86 (d, J = 8.0 Hz, 1H), 7.69 (s, 1H), 7.66 (dd, J = 8.8, 2.4 Hz, 1H), 7.07 (s, 1H); LC-MS: m/z [M+H]⁺ = 347.

### Example 36 7-(2-chloro-4-(difluoromethy)phenyl)-1,3-dihydro-2H-imidazo[4,5-b]quinolin-2-one

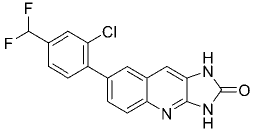

The title compound was synthesized by replacing 5-bromo-4-chloro-2-methoxypyridine (CAS: 851607-27-7) with 1-bromo-2-chloro-4-(difluoromethyl)benzene (intermediate 19) using a method similar to that in Example 8. ¹H NMR (400 MHz, DMSO-d6) δ 11.31 (s, 2H), 7.98 (d, J = 2.4 Hz, 1H), 7.86 (d, J = 8.8 Hz, 1H), 7.82 (s, 1H), 7.70 - 7.65 (m, 3H), 7.60 (dd, J = 8.8, 2.4 Hz, 1H), 7.13 (t, J = 55.6 Hz, 1H); LC-MS: m/z [M+H]⁺ = 346.

### Example 37 7-(5-chloro-1-(difluoromethyl)-1H-imidazol-4-yl)-1,3-dihydro-2H-imidazo [4,5-b]quinolin-2-one

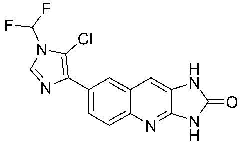

### Example 38 7-(4-chloro-1-(difluoromethyl)-1H-imidazol-5-yl)-1,3-dihydro-2H-imidazo[4,5-b]quinolin-2-one

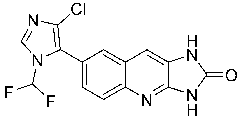

The 2 title compounds were synthesized by replacing 5-bromo-4-chloro-2-methoxypyridine (CAS: 851607-27-7) with a mixture of 5-chloro-1-(difluoromethyl)-4-iodo-1H-imidazole and 4-chloro-1-(difluoromethyl)-5-iodo-1H-imidazole (intermediate 14) using a method similar to that in Example 8, and finally separated by a chiral column, respectively. Example 37: ¹H NMR (400 MHz, DMSO-d6) δ 11.55 (s, 1H), 11.13 (s, 1H), 8.42 - 8.37 (m, 2H), 8.10 - 7.75 (m, 3H), 7.71 (s, 1H); LC-MS: m/z [M+H]⁺ =336. Example 38: ¹H NMR (400 MHz, DMSO-d6) δ 11.65 (s, 1H), 11.18 (s, 1H), 8.35 (s, 1H), 8.01 (d, J = 2.0 Hz, 1H), 7.90 (d, J = 8.4 Hz, 1H), 7.85 - 7.52 (m, 3H); LC-MS: m/z [M+H]⁺ = 336.

### Example 39 7-(6-chloro-2-(difluoromethoxy)pyridin-3-yl)-1,3-dihydro-2H-imidazo[4,5-b]quinolin-2-one

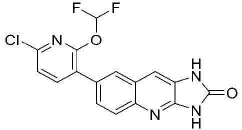

The title compound was synthesized by replacing 5-bromo-4-chloro-2-methoxypyridine (CAS: 851607-27-7) with 3-bromo-6-chloro-2-(difluoromethoxy)pyridine (intermediate 15) using a method similar to that in Example 8. ¹H NMR (400 MHz, DMSO-d6) δ 11.61 (s, 1H), 11.12 (s, 1H), 8.13 (d, J = 8.0 Hz, 1H), 8.06 (d, J = 2.4 Hz, 1H), 7.93 - 7.50 (m, 5H); LC-MS: m/z [M+H]⁺ = 363.

### Example 40 7-(2-chloro-6-(methylamino)pyridin-3-yl)-1,3-dihydro-2H-imidazo[4,5-b]quinolin-2-one

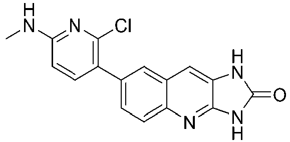

The title compound was synthesized by replacing 5-bromo-4-chloro-2-methoxypyridine (CAS: 851607-27-7) with 5-bromo-6-chloro-N-methylpyridin-2-amine (intermediate 16a) using a method similar to that in Example 8. ¹H NMR (400 MHz, DMSO-d6) δ 11.49 (s, 1H), 11.07 (s, 1H), 7.86 (d, J = 2.0 Hz, 1H), 7.79 (d, J = 8.8 Hz, 1H), 7.63 (s, 1H), 7.58 - 7.51 (m, 2H), 7.01 (q, J = 5.2 Hz, 1H), 6.54 (d, J = 8.4 Hz, 1H), 2.80 (d, J = 4.8 Hz, 3H); LC-MS: m/z [M+H]⁺ = 326.

### Example 41 7-(6-chloro-2-(methylamino)pyridin-3-yl)-1,3-dihydro-2H-imidazo[4,5-b]quinolin-2-one

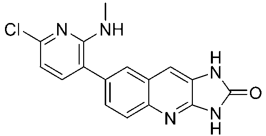

The title compound was synthesized by replacing 5-bromo-4-chloro-2-methoxypyridine (CAS: 851607-27-7) with 3-bromo-6-chloro-N-methylpyridin-2-amine (intermediate 16b) using a method similar to that in Example 8, and finally obtained by purification with column chromatography, respectively. ¹H NMR (400 MHz, DMSO-d6) δ 11.04 (s, 2H), 7.90 (d, J = 2.0 Hz, 1H), 7.85 (d, J = 8.4 Hz, 1H), 7.64 (s, 1H), 7.49 (dd, J = 8.4, 2.0 Hz, 1H), 7.35 (d, J = 7.6 Hz, 1H), 6.66 (d, J = 7.6 Hz, 1H), 6.23 (d, J = 4.8 Hz, 1H), 2.76 (d, J = 4.8 Hz, 3H); LC-MS: m/z [M+H]⁺ = 326.

### Example 42 7-(5-(difluoromethoxy)-1-(difluoromethyl)-1H-pyrazol-4-yl)-1,3-dihydro-2H-imidazo[4,5-b]quinolin-2-one

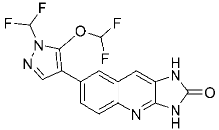

The title compound was synthesized by replacing 5-bromo-4-chloro-2-methoxypyridine (CAS: 851607-27-7) with 5-(difluoromethoxy)-1-(difluoromethyl)-4-iodo-1H-pyrazole (intermediate 17a) using a method similar to that in Example 8. ¹H NMR (400 MHz, DMSO-d6) δ 11.57 (s, 1H), 11.09 (s, 1H), 8.31 (s, 1H), 8.11 (d, J = 2.0 Hz, 1H), 8.03 - 7.68 (m, 3H), 7.60 (s, 1H), 7.24 (t, J = 70.4 Hz, 1H); LC-MS: m/z [M+H]⁺ = 368.

### Example 43 7-(3-(difluoromethoxy)-1-(difluoromethyl)-1H-pyrazol-4-yl)-1,3-dihydro-2H-imidazo[4,5-b]quinolin-2-one

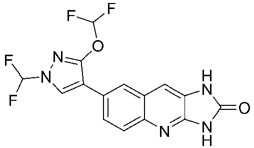

The title compound was synthesized by replacing 5-bromo-4-chloro-2-methoxypyridine (CAS: 851607-27-7) with a mixture of 3-(difluoromethoxy)-1-(difluoromethyl)-4-iodo-1H-pyrazole (intermediate 17b) using a method similar to that in Example 8, and finally obtained by purification with column chromatography, respectively. ¹H NMR (400 MHz, DMSO-d6) δ 11.57 (s, 1H), 11.09 (s, 1H), 8.74 (s, 1H), 8.11 (d, J = 1.8 Hz, 1H), 7.96 - 7.34 (m, 5H); LC-MS: m/z [M+H]⁺ = 368.

### Example 44 7-(1-(difluoromethyl)-5-methyl-1H-pyrazol-4-yl)-1,3-dihydro-2H-imidazo[4,5-b]quinolin-2-one

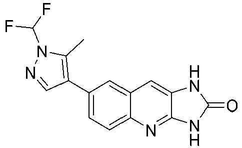

### 1) Synthesis of 7-(1-(difluoromethyl)-5-methyl-1H-pyrazol-4-yl)-1,3-bis((2-(trimethylsilyl)ethoxy)methyl)-1,3-dihydro-2H-imidazo [4,5-b]quinolin-2-one

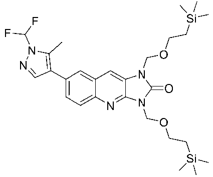

7-(5-chloro-1-(difluoromethyl)-1H-pyrazol-4-yl)-1,3-bis((2-(trimethylsilyl)ethoxy)methyl)-1,3-dihydro-2H-imidazo[4,5-b]quinolin-2-one (250 mg, 0.42 mmol, synthesized from 7-(4,4,5,5-tetramethyl-1,3,2-dioxazoboran-2-yl)-1,3-bis((2-(trimethylsilyl)ethoxy)methyl)-1,3-dihydro-2H-imidazo[4,5-b]quinolin-2-one (intermediate 5) and 5-chloro-1-(difluoromethyl)-4-iodo-1H-pyrazole (intermediate 6) using a method similar to that in the first step of Example 8), trimethylcyclotriboroxane (0.14 mL, 0.50 mmol, 3.5 M tetrahydrofuran solution, CAS: 823-96-1), [1,1'-bis(di-tert-butylphosphino)ferrocene]dichloropalladium (27.07 mg, 0.04 mmol,CAS: 95408-45-0), and cesium carbonate (409.86 mg, 1.26 mmol) were dissolved in a mixed solvent of 1,4-dioxane (6 mL) and water (1.5 mL), nitrogen replacement was performed 3 times, and the reaction solution was stirred at 100°C for 16 hours in a nitrogen atmosphere. The reaction solution was cooled to room temperature, diluted with water (15 mL), and extracted 3 times with ethyl acetate (3 mL). The organic phases were combined, dried over anhydrous sodium sulfate, concentrated under reduced pressure, and separated by column chromatography (petroleum ether/ethyl acetate = 5:1) to obtain the title compound as a yellow solid (167 mg, yield: 69%). LC-MS: m/z [M+H]⁺ = 576.

### 2) Synthesis of 7-(1-(difluoromethyl)-5-methyl-1H-pyrazol-4-yl)-1,3-dihydro-2H-imidazo[4,5-b]quinolin-2-one

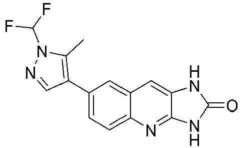

7-(1-(difluoromethyl)-5-methyl-1H-pyrazol-4-yl)-1,3-bis((2-(trimethylsilyl)ethoxy)methyl)-1,3-dihydro-2H-imidazo[4,5-b]quinolin-2-one (167 mg, 0.29 mmol) was dissolved in trifluoroacetic acid (2 mL), then trifluoromethanesulfonic acid (30 µL, 0.34 mmol, d = 1.696 g/mL) was added, and the mixture was stirred at 25°C for 1 hour. The reaction solution was directly concentrated under reduced pressure, then a 7 M solution of ammonia in methanol (5 mL) and dichloromethane (5 mL) were added, and the mixture was stirred at room temperature for 0.5 hour. The mixture was filtered, the filter cake was washed with dichloromethane (5 mL) and dissolved in N,N-dimethylformamide (5 mL), then the solution was added dropwise into water (25 mL), and the precipitated solid was collected by filtration and then dried to obtain the title compound as a white solid (23 mg, yield: 25%). ¹H NMR (400 MHz, DMSO-d6) δ 11.55 (s, 1H), 11.09 (s, 1H), 8.06 - 7.75 (m, 4H), 7.66 - 7.60 (m, 2H), 2.59 (s, 3H); LC-MS: m/z [M+H]⁺ = 316.

### Example 45 7-(1-(difluoromethyl)-5-(methyl-d3)-1H-pyrazol-4-yl)-1,3-dihydro-2H-imidazo[4,5-b]quinolin-2-one

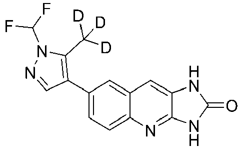

The title compound was synthesized by replacing trimethylcyclotriboroxane (CAS: 823-96-1) with (methyl-d3)boronic acid (intermediate 18) using a method similar to that in Example 44. ¹H NMR (400 MHz, DMSO-d6) δ 11.25 (s, 2H), 8.07 - 7.72 (m, 4H), 7.65 - 7.58 (m, 2H); LC-MS: m/z [M+H]⁺ = 319.

### Example 46 7-(2-chloro-4-(difluoromethyl)phenyl)-1,3,5,6,7,8-hexahydro-2H-imidazo[4,5-b][1,6]naphthyridin-2-one

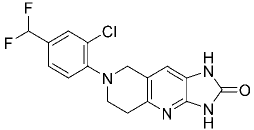

The title compound was synthesized by replacing bromobenzene with 1-bromo-2-chloro-4-(difluoromethyl)benzene (intermediate 19) using a method similar to that in Example 20. ¹H NMR (400 MHz, DMSO-d6) δ 11.15 (s, 1H), 10.72 (s, 1H), 7.65 (s, 1H), 7.53 (d, J = 7.3 Hz, 1H), 7.33 (d, J = 8.4 Hz, 1H), 7.18 - 6.78 (m, 2H), 4.25 (s, 2H), 3.43 (t, J = 5.7 Hz, 2H), 2.94 (t, J = 6.0 Hz, 2H); LC-MS: m/z [M+H]⁺ = 351.

### Example 47 7-(2,4-dichlorophenyl)-1,3,5,6,7,8-hexahydro-2H-imidazo[4,5-b][1,6]naphthyridin-2-one

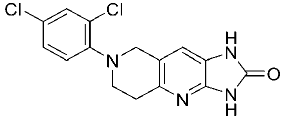

The title compound was synthesized by replacing bromobenzene with 2,4-dichlorobromobenzene (CAS: 1193-72-2) using a method similar to that in Example 20. ¹H NMR (400 MHz, DMSO-d6) δ 11.15 (s, 1H), 10.72 (s, 1H), 7.58 (d, J = 2.5 Hz, 1H), 7.39 (dd, J = 8.7, 2.5 Hz, 1H), 7.23 (d, J = 8.7 Hz, 1H), 7.02 (s, 1H), 4.18 (s, 2H), 3.36 (d, J = 5.7 Hz, 2H), 2.92 (t, J = 5.7 Hz, 2H); LC-MS: m/z [M+H]⁺ = 335.

### Example 48 7-(2-chloro-6-(trifluoromethyl)pyridin-3-yl)-1,3,5,6,7,8-hexahydro-2H-imidazo[4,5-b][1,6]naphthyridin-2-one

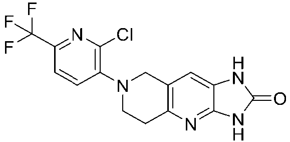

The title compound was synthesized by replacing bromobenzene with 3-bromo-2-chloro-6-(trifluoromethyl)pyridine (CAS: 1159512-34-1) using a method similar to that in Example 20. ¹H NMR (400 MHz, DMSO-d6) δ 11.18 (s, 1H), 10.74 (s, 1H), 7.89 (d, J = 8.2 Hz, 1H), 7.75 (d, J = 7.9 Hz, 1H), 7.04 (s, 1H), 4.35 (s, 2H), 3.56 (t, J = 6.1 Hz, 2H), 2.96 (t, J = 5.5 Hz, 2H); LC-MS: m/z [M+H]⁺ = 370.

### Example 49 7-(3-fluorophenyl)-1,3,5,6,7,8-hexahydro-2H-imidazo[4,5-b][1,6]naphthyridin-2-one

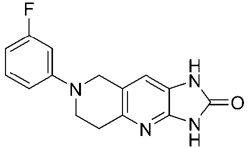

The title compound was synthesized by replacing bromobenzene with m-bromofluorobenzene (CAS: 1073-06-9) using a method similar to that in Example 20. ¹H NMR (400 MHz, DMSO-d6) δ 11.15 (s, 1H), 10.73 (s, 1H), 7.22 (dd, J = 15.9, 8.2 Hz, 1H), 7.09 (s, 1H), 6.81 (t, J = 9.7 Hz, 2H), 6.51 (t, J = 7.5 Hz, 1H), 4.40 (s, 2H), 3.62 (t, J = 5.8 Hz, 2H), 2.88 (t, J = 5.6 Hz, 2H); LC-MS: m/z [M+H]⁺ = 285.

### Example 50 7-(5-chloro-1-(difluoromethyl)-1H-pyrazol-4-yl)-1,3,5,6,7,8-hexahydro-2H-imidazo[4,5-b][1,6]naphthyridin-2-one

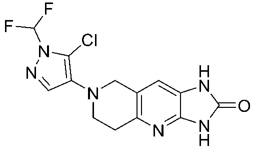

### Example 51 7-(3-chloro-1-(difluoromethyl)-1H-pyrazol-4-yl)-1,3,5,6,7,8-hexahydro-2H-imidazo[4,5-b][1,6]naphthyridin-2-one

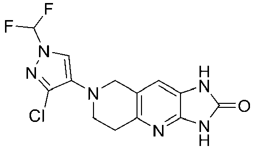

The 2 title compounds were synthesized by replacing bromobenzene with a mixture of 3-chloro-1-(difluoromethyl)-4-iodo-1H-pyrazole and 5-chloro-1-(difluoromethyl)-4-iodo-1H-pyrazole (intermediate 6) using a method similar to that in Example 20, and finally obtained by purification with preparative high performance liquid chromatography (acetonitrile and water, 0.1% formic acid), respectively. Example 50: ¹H NMR (400 MHz, DMSO-d6) δ 11.12 (s, 1H), 10.71 (s, 1H), 7.84 (s, 1H), 7.82 (t, J = 57.2 Hz, 1H), 6.99 (s, 1H), 4.20 (s, 2H), 3.39 (t, J = 6.0 Hz, 2H), 2.87 (t, J = 6.0 Hz, 2H); LC-MS: m/z [M+H]⁺ =341. Example 51: ¹H NMR (400 MHz, DMSO-d6) δ 10.92 (s, 1H), 10.50 (s, 1H), 7.96 (s, 1H), 7.60 (t, J = 59.2 Hz, 1H), 6.96 (s, 1H), 4.14 (s, 2H), 3.35 (t, J = 6.0 Hz, 2H), 2.90 (t, J = 6.0 Hz, 2H); LC-MS: m/z [M+H]⁺ = 341.

### Example 52 7-(2-fluoro-4-cyanophenyl)-1,3,5,6,7,8-hexahydro-2H-imidazo[4,5-b][1,6]naphthyridin-2-one

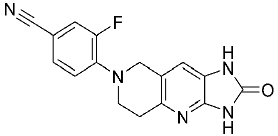

The title compound was synthesized by replacing 3-chloro-4-fluorobenzonitrile (CAS: 117482-84-5) with 3,4-difluorobenzonitrile (CAS: 64248-62-0) using a method similar to that in Example 22. ¹H NMR (400 MHz, DMSO-d6) δ 11.17 (s, 1H), 10.74 (s, 1H), 7.73 (dd, J = 13.6, 2.0 Hz, 1H), 7.57 (dd, J = 8.4, 2.0 Hz, 1H), 7.17 (t, J = 8.4 Hz, 1H), 7.07 (s, 1H), 4.42 (s, 2H), 3.62 (t, J = 6.0 Hz, 2H), 2.90 (t, J = 6.0 Hz, 2H); LC-MS: m/z [M+H]⁺ = 310.

### Example 53 7-(3-fluoro-4-cyanophenyl)-1,3,5,6,7,8-hexahydro-2H-imidazo[4,5-b][1,6]naphthyridin-2-one

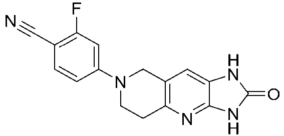

The title compound was synthesized by replacing 3-chloro-4-fluorobenzonitrile (CAS: 117482-84-5) with 2,4-difluorobenzonitrile (CAS: 3939-09-1) using a method similar to that in Example 22. ¹H NMR (400 MHz, DMSO-d6) δ 11.19 (s, 1H), 10.75 (s, 1H), 7.62 (t, J = 8.8 Hz, 1H), 7.09 (s, 1H), 6.98 (dd, J = 14.0, 2.4 Hz, 1H), 6.90 (dd, J = 9.2, 2.4 Hz, 1H), 4.57 (s, 2H), 3.75 (t, J = 6.0 Hz, 2H), 2.91 (t, J = 6.0 Hz, 2H); LC-MS: m/z [M+H]⁺ = 310.

### Example 54 7-(2-chloro-4-(trifluoromethoxy)phenyl)-1,3,5,6,7,8-hexahydro-2H-imidazo[4,5-b][1,6]naphthyridin-2-one

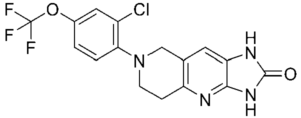

### Example 55 7-(3-(trifluoromethoxy)phenyl)-1,3,5,6,7,8-hexahydro-2H-imidazo[4,5-b][1,6]naphthyridin-2-one

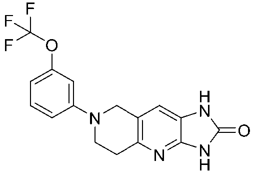

The 2 title compounds were synthesized by replacing bromobenzene with 1-bromo-2-chloro-4-(trifluoromethoxy)benzene (CAS: 892845-59-9) using a method similar to that in Example 20, wherein the compound in Example 55 was synthesized from a by-product generated during preparation of the compound in Example 54. Example 54: ¹H NMR (400 MHz, DMSO-d6) δ 11.16 (s, 1H), 10.72 (s, 1H), 7.57 (d, J = 2.2 Hz, 1H), 7.44 - 7.26 (m, 2H), 7.02 (s, 1H), 4.20 (s, 2H), 3.37 (t, J = 5.6 Hz, 2H), 2.93 (t, J = 5.4 Hz, 2H); LC-MS: m/z [M+H]⁺ =385. Example 55: ¹H NMR (400 MHz, DMSO-d6) δ 11.15 (s, 1H), 10.72 (s, 1H), 7.31 (t, J = 8.3 Hz, 1H), 7.11 (d, J = 11.4 Hz, 1H), 7.02 (d, J = 8.6 Hz, 1H), 6.91 (s, 1H), 6.67 (d, J = 9.5 Hz, 1H), 4.42 (s, 2H), 3.65 (t, J = 5.9 Hz, 2H), 2.89 (t, J = 5.3 Hz, 2H); LC-MS: m/z [M+H]⁺ = 351.

### Example 56 7-(3-chloro-5-cyanopyridin-2-yl)-1,3,5,6,7,8-hexahydro-2H-imidazo[4,5-b][1,6]naphthyridin-2-one

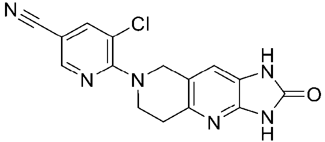

The title compound was synthesized by replacing 3-chloro-4-fluorobenzonitrile (CAS: 117482-84-5) with 5,6-dichloronicotinonitrile (CAS: 65189-15-3) using a method similar to that in Example 22. ¹H NMR (400 MHz, DMSO-d6) δ 11.17 (s, 1H), 10.74 (s, 1H), 8.62 (d, J = 2.0 Hz, 1H), 8.32 (d, J = 2.0 Hz, 1H), 7.10 (s, 1H), 4.67 (s, 2H), 3.86 (t, J = 5.6 Hz, 2H), 2.98 (t, J = 5.6 Hz, 2H); LC-MS: m/z [M+H]⁺ = 327.

### Example 57 7-(4-fluorophenyl)-1,3,5,6,7,8-hexahydro-2H-imidazo[4.5-b]quinolin-2-one

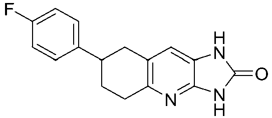

### 1) Synthesis of 8-(4-fluorophenyl)-1,4-dioxaspiro[4.5]dec-7-ene

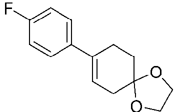

At room temperature, [1,1'-bis(diphenylphosphino)ferrocene]dichloropalladium (988.80 mg, 1.35 mmol, CAS: 72287-26-4) was added into a solution of p-fluoroiodobenzene (1.55 mL, 13.51 mmol, CAS: 352-34-1, d = 1.925 g/mL), 1,4-dioxaspiro[4,5]dec-7-en-8-boronic acid pinacol ester (3596.49 mg, 13.51 mmol, CAS: 680596-79-6), and cesium carbonate (8805.95 mg, 27.03 mmol) in dioxane (40 mL) and water (8 mL), and the mixture was stirred overnight at 80°C under nitrogen protection. The reaction solution was filtered through Celite, and the filtrate was concentrated under reduced pressure, and then separated and purified by column chromatography (petroleum ether/ethyl acetate = 50:1-10:1) to obtain the title compound as a light yellow solid (1900 mg, yield: 60%).

### 2) Synthesis of 8-(4-fluorophenyl)-1,4-dioxaspiro[4.5]decane

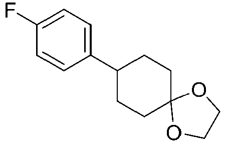

At room temperature, a 10% palladium on carbon catalyst (0.98 g, CAS:7440-05-3) was added in batches into a solution of 8-(4-fluorophenyl)-1,4-dioxaspiro[4.5]dec-7-ene (4500 mg, 19.21 mmol) in tetrahydrofuran (100 mL), and the mixture was stirred overnight at 35°C in a hydrogen balloon atmosphere. The reaction solution was filtered through Celite, and the filtrate was concentrated under reduced pressure to obtain the title compound as a colorless oil (3800 mg of a crude product), which was directly used in the next step.

### 3) Synthesis of 4-(4-fluorophenyl)cyclohexa-1-one

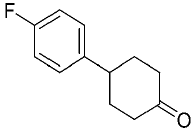

Trifluoroacetic acid (5 mL, 67.09 mmol, d = 1.489 g/mL) was added into a solution of 8-(4-fluorophenyl)-1,4-dioxaspiro[4.5]decane (3.8 g, 16.08 mmol) in dichloromethane (30 mL), and the mixture was stirred at 25°C for 3 hours. The reaction solution was directly concentrated under reduced pressure, and then separated and purified by column chromatography (petroleum ether/ethyl acetate = 5:1) to obtain the title compound as a light yellow oil (3 g, 15.61 mmol, yield: 97%).

### 4) Synthesis of 2-((dimethylamino)methyl)-4-(4-fluorophenyl)cyclohexa-1-one

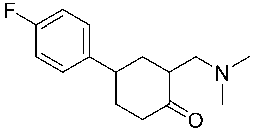

N,N-dimethylmethyleneiminium chloride (146.00 mg, 1.56 mmol, CAS: 30354-18-8) was added into a solution of 4-(4-fluorophenyl)cyclohexa-1-one (200 mg, 1.04 mmol) in acetonitrile (15 mL), and the mixture was stirred at 70°C for 3 hours. The reaction solution was directly concentrated under reduced pressure to obtain the title compound as a yellow oil (260 mg of a crude product), which was directly used in the next step. LC-MS: m/z [M+H]⁺ = 250.

### 5) Synthesis of 6-(4-fluorophenyl)-3-nitro-5,6,7,8-tetrahydroquinolin-2-amine

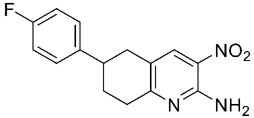

At room temperature, 2-nitroethene-1,1-diamine (107.49 mg, 1.04 mmol, CAS: 71090-35-2) was added into a solution of 2-((dimethylamino)methyl)-4-(4-fluorophenyl)cyclohexa-1-one (260 mg, 1.04 mmol) in N,N-dimethylformamide (4 mL), and the mixture was stirred at 130°C for 4 hours. The reaction solution was cooled to room temperature, diluted with water (20 mL), and extracted 3 times with ethyl acetate (5 mL). The organic phases were combined, dried over anhydrous sodium sulfate, filtered, concentrated under reduced pressure, and separated and purified by column chromatography (petroleum ether/ethyl acetate = 1:100) to obtain the title compound as a yellow solid (100 mg, yield: 33%). LC-MS: m/z [M+H]⁺ = 288.

### 6) Synthesis of 6-(4-fluorophenyl)-5,6,7,8-tetrahydroquinolin-2,3-diamine

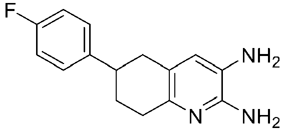

At room temperature, a 10% palladium on carbon catalyst (9 mg, CAS: 7440-05-3) was added in batches into a solution of 6-(4-fluorophenyl)-3-nitro-5,6,7,8-tetrahydroquinolin-2-amine (80 mg, 0.28 mmol) in methanol (4 mL), and the mixture was stirred overnight at 25°C in a hydrogen balloon atmosphere. The reaction solution was filtered through Celite, and the filtrate was concentrated under reduced pressure to obtain the title compound as a yellow oil (65 mg of a crude product), which was directly used in the next step.

### 7) Synthesis of 7-(4-fluorophenyl)-1,3,5,6,7,8-hexahydro-2H-imidazo[4,5-b]quinolin-2-one

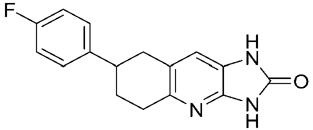

N,N'-carbonyldiimidazole (135.46 mg, 0.84 mmol) was added into a solution of 6-(4-fluorophenyl)-5,6,7,8-tetrahydroquinolin-2,3-diamine (71.65 mg, 0.28 mmol) and N,N-diisopropylethylamine (138.43 µL, 0.84 mmol, d = 0.742 g/mL) in N,N-dimethylformamide (2 mL), and the mixture was stirred at 60°C for 16 hours. The reaction solution was separated and purified by preparative high efficiency liquid chromatography (acetonitrile and water containing 0.1% formic acid) to obtain the title compound as a light yellow solid (23.3 mg, yield: 29.5%). ¹H NMR (400 MHz, DMSO-d6) δ 11.05 (s, 1H), 10.63 (s, 1H), 7.40 - 7.32 (m, 2H), 7.18 - 7.09 (m, 2H), 6.93 (s, 1H), 3.01 - 2.79 (m, 5H), 2.09 - 1.89 (m, 2H); LC-MS: m/z [M+H]⁺ = 284.

### Example 58 7-(6-(trifluoromethyl)pyridin-2-yl)-1,3,5,6,7,8-hexahydro-2H-imidazo[4,5-b][1,6]naphthyridin-2-one

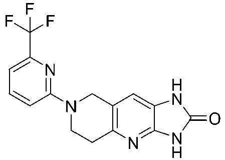

The title compound was synthesized by replacing 3-chloro-4-fluorobenzonitrile (CAS: 117482-84-5) with 2-fluoro-6-(trifluoromethyl)pyridine (CAS: 94239-04-0) using a method similar to that in Example 22. ¹H NMR (400 MHz, DMSO-d6) δ 11.17 (s, 1H), 10.74 (s, 1H), 7.78 (t, J = 8.0 Hz, 1H), 7.19 (d, J = 8.8 Hz, 1H), 7.12 (s, 1H), 7.05 (d, J = 7.2 Hz, 1H), 4.71 (s, 2H), 3.93 (t, J = 6.0 Hz, 2H), 2.90 (t, J = 6.0 Hz, 2H); LC-MS: m/z [M+H]⁺ = 336.

### Example 59 7-(3-chlorophenyl)-1,3,5,6,7,8-hexahydro-2H-imidazo[4,5-b][1,6]naphthyridin-2-one

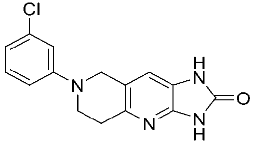

The title compound was synthesized by replacing bromobenzene with m-chlorobromobenzene (CAS: 108-37-2) using a method similar to that in Example 20. ¹H NMR (400 MHz, DMSO-d6) δ 11.14 (s, 1H), 10.71 (s, 1H), 7.22 (t, J = 8.4 Hz, 1H), 7.10 (s, 1H), 7.04 - 6.94 (m, 2H), 6.78 - 6.72 (m, 1H), 4.40 (s, 2H), 3.63 (t, J = 6.0 Hz, 2H), 2.88 (t, J = 6.0 Hz, 2H); LC-MS: m/z [M+H]⁺ = 301.

### Example 60 7-(3-fluoro-6-(trifluoromethyl)pyridin-2-yl)-1,3,5,6,7,8-hexahydro-2H-imidazo[4,5-b][1,6]naphthyridin-2-one

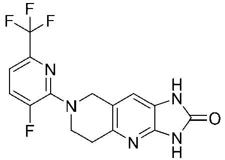

The title compound was synthesized by replacing 3-chloro-4-fluorobenzonitrile (CAS: 117482-84-5) with 2-chloro-3-fluoro-6-(trifluoromethyl)pyridine (CAS: 1159512-39- 6) using a method similar to that in Example 22. ¹H NMR (400 MHz, DMSO-d6) δ 11.16 (s, 1H), 10.75 (s, 1H), 7.75 (dd, J = 13.2, 8.0 Hz, 1H), 7.29 (dd, J = 8.0, 2.4 Hz, 1H), 7.12 (s, 1H), 4.68 (s, 2H), 3.85 (t, J = 6.0 Hz, 2H), 2.95 (t, J = 6.0 Hz, 2H); LC-MS: m/z [M+H]⁺ = 354.

### Example 61 7-(5-fluoro-6-(trifluoromethyl)pyridin-2-yl)-1,3,5,6,7,8-hexahydro-2H-imidazo[4,5-b][1,6]naphthyridin-2-one

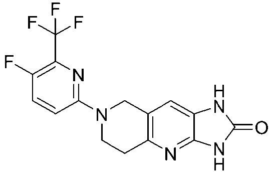

### Example 62 7-(6-chloro-2-(trifluoromethyl)pyridin-3-yl)-1,3,5,6,7,8-hexahydro-2H-imidazo[4,5-b][1,6]naphthyridin-2-one

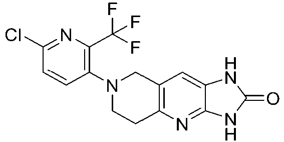

The 2 title compounds were synthesized by replacing 3-chloro-4-fluorobenzonitrile (CAS: 117482-84-5) with 6-chloro-3-fluoro-2-(trifluoromethyl)pyridine (CAS: 1227511-58-1) using a method similar to that in Example 22, wherein the compound in Example 62 was synthesized from a by-product generated during preparation of the compound in Example 61. Example 61: ¹H NMR (400 MHz, DMSO-d6) δ 11.17 (s, 1H), 10.73 (s, 1H), 7.83 (t, J = 9.6 Hz, 1H), 7.28 (dd, J = 9.2, 2.4 Hz, 1H), 7.10 (s, 1H), 4.67 (s, 2H), 3.89 (t, J = 6.0 Hz, 2H), 2.89 (t, J = 6.0 Hz, 2H); LC-MS: m/z [M+H]⁺ =354. Example 62: ¹H NMR (400 MHz, DMSO-d6) δ 11.17 (s, 1H), 10.72 (s, 1H), 8.13 (d, J = 8.8 Hz, 1H), 7.85 (d, *J =* 8.8 Hz, 1H), 6.99 (s, 1H), 4.16 (s, 2H), 3.29 (t, J = 6.0 Hz, 2H), 2.90 (t, J = 6.0 Hz, 2H); LC-MS: m/z [M+H]⁺ = 370.

### Example 63 7-(3-chloro-1-(difluoromethyl)-1H-pyrazol-4-yl)-1,3,5,6,7,8-hexahydro-2H-imidazo[4,5-b]quinolin 2-one

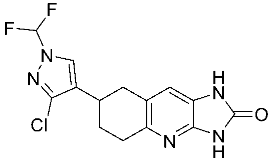

### Example 64 7-(5-chloro-1-(difluoromethyl)-1H-pyrazol-4-yl)-1,3,5,6,7,8-hexahydro-2H-imidazo[4,5-b]quinolin-2-one

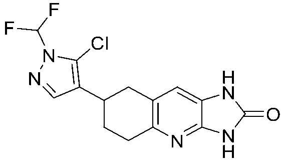

The 2 title compounds were synthesized by replacing p-fluoroiodobenzene (CAS: 352-34-1) with a mixture of 3-chloro-1-(difluoromethyl)-4-iodo-1H-pyrazole and 5-chloro-1-(difluoromethyl)-4-iodo-1H-pyrazole (intermediate 6) using a method similar to that in Example 57, and finally obtained by purification with column chromatography, respectively. Example 63: ¹H NMR (400 MHz, DMSO-d6) δ 11.06 (s, 1H), 10.63 (s, 1H), 8.18 (s, 1H), 7.72 (t, J = 58.8 Hz, 1H), 6.94 (s, 1H), 3.01 (dd, J = 14.8, 3.8 Hz, 1H), 2.94 - 2.76 (m, 4H), 2.10 (d, J= 13.0 Hz, 1H), 1.88 (qd, J = 11.0, 5.6 Hz, 1H); LC-MS: m/z [M+H]⁺=340. Example 64: ¹H NMR (400 MHz, DMSO-d6) δ 11.06 (s, 1H), 10.62 (s, 1H), 8.06 - 7.72 (m, 2H), 6.94 (s, 1H), 2.99 - 2.76 (m, 5H), 2.10 - 1.88 (m, 2H); LC-MS: m/z [M+H]⁺ = 340.

### Example 64-A (*S)-7-(5-chloro-1-(difluoromethyl)-1H-pyrazol-4-yl)-1,3,5,6,7,8-hexahydro-2H-imidazo[4,5-b]quinolin-2-one

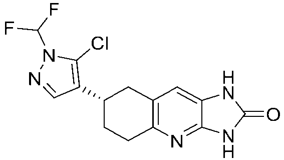

### Example 64-B (*R)-7-(5-chloro-1-(difluoromethyl)-1H-pyrazol-4-yl)-1,3,5,6,7,8-hexahydro-2H-imidazo[4,5-b]quinolin-2-one

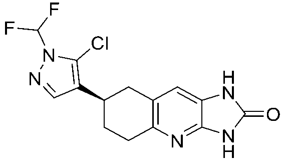

223 mg of the racemic compound 7-(5-chloro-1-(difluoromethyl)-1H-pyrazol-4-yl)-1,3,5,6,7,8-hexahydro-2H-imidazo[4,5-b]quinolin-2-one (Example 64) was subjected to chiral resolution to obtain (*S)-7-(5-chloro-1-(difluoromethyl)-1H-pyrazol-4-yl)-1,3,5,6,7,8-hexahydro-2H-imidazo[4,5-b]quinolin-2-one (first elution peak, RT = 2.496 minutes, temporarily designated as "S" ABS) (83.1 mg, %ee 100, solid white) in Example 64-A and (*R)-7-(5-chloro-1-(difluoromethyl)-1H-pyrazol-4-yl)-1,3,5,6,7,8-hexahydro-2H-imidazo[4,5-b]quinolin-2-one (second elution peak, RT = 2.887 minutes, temporarily designated as 'R' ABS) (66.2 mg, %ee 100, white solid) in Example 64-B, respectively.

### Chiral analysis method (analytical separation method):

Instrument: Waters UPC2 analytical SFC (SFC-H).
Chromatographic column: ChiralPak IG, 100 × 4.6 mm I.D., 3 µm.
Mobile phase: A for CO₂ and B for ethanol (0.05% DEA).
Isocratic elution: B 50%.
Flow rate: 2.0 mL/min.
Chromatographic column temperature: 35°C.
Wavelength: 220 nm.

### Chiral preparation method (preparative separation method):

Instrument: WATERS 150 preparative SFC (SFC-26).
Chromatographic column: ChiralPak IG, 250 × 30 mm I.D., 10 µm.
Mobile phase: A for CO₂ and B for ethanol (0.1% NH₃.H₂O).
Isocratic elution: B 40%.
Flow rate: 120 mL/min.
Back pressure: 100 bar.
Chromatographic column temperature: 38°C.
Wavelength: 220 nm.
Cycle time: ~ 3 min.
Example 64-A: ¹H NMR (400 MHz, DMSO-d6) δ 11.07 (s, 1H), 10.63 (s, 1H), 8.06 - 7.72 (m, 2H), 6.94 (s, 1H), 2.99 - 2.77 (m, 5H), 2.10 - 1.88 (m, 2H); LC-MS: m/z [M+H]⁺ = 340.
Example 64-B: ¹H NMR (400 MHz, DMSO-d6) δ 11.07 (s, 1H), 10.63 (s, 1H), 8.06 - 7.74 (m, 2H), 6.94 (s, 1H), 3.00 - 2.76 (m, 5H), 2.10 - 1.88 (m, 2H); LC-MS: m/z [M+H]⁺ = 340.

### Example 65 8-chloro-7-phenyl-1,3-dihydro-2H-imidazo[4,5-b]quinolin-2-one

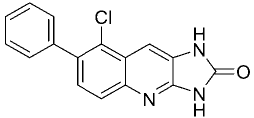

### 1) Synthesis of 8-chloro-7-phenyl-1,3-bis((2-(trimethylsilyl)ethoxy)methyl)-1,3-dihydro-2H-imidazo[4,5-b]quinolin-2-one

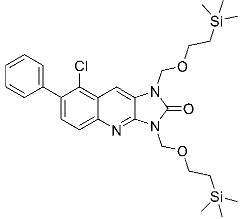

In a nitrogen atmosphere, [1,1'-bis(diphenylphosphino)ferrocene]dichloropalladium (50 mg, 0.06 mmol, CAS: 72287-26-4) was added into a mixed solution of 7-bromo-8-chloro-1,3-bis((2-(trimethylsilyl)ethoxy)methyl)-1,3-dihydro-2H-imidazo[4,5-b]quinolin-2-one (140 mg, 0.25 mmol, intermediate 1a), cesium carbonate (300 mg, 0.92 mmol), phenylboronic acid (40 mg, 0.33 mmol), water (0.5 mL), and 1,4-dioxane (2.5 mL), and the reaction mixture was allowed to react at 100°C for 3 hours. The reaction solution was cooled to room temperature, diluted with water (20 mL), and extracted twice with ethyl acetate (20 mL). The organic phases were combined, washed twice with water (20 mL), washed twice with brine (20 mL), dried over anhydrous sodium sulfate, concentrated under reduced pressure, and separated by column chromatography (petroleum ether/ethyl acetate = 10:1) to obtain the title compound as a yellow oil (110 mg, yield: 79%). LC-MS: m/z [M+H]⁺ = 556.

### 2) Synthesis of 8-chloro-7-phenyl-1,3-dihydro-2H-imidazo[4,5-b]quinolin-2-one

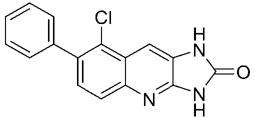

8-chloro-7-phenyl-1,3-bis((2-(trimethylsilyl)ethoxy)methyl)-1,3-dihydro-2H-imidazo[4,5-b]quinolin-2-one (100 mg, 0.20 mmol) was dissolved in trifluoroacetic acid (2 mL) to carry out a reaction under a microwave at 130°C for 1 hour. The reaction solution was directly concentrated, the residue was dissolved in dichloromethane (1 mL), a 7 M solution of ammonia in methanol (1 mL), and ammonia water (1 mL), and the reaction mixture was allowed to react at 20°C for 1 hour. The reaction solution was directly concentrated and purified by preparative liquid chromatography (acetonitrile and water containing 0.1% ammonium bicarbonate) to obtain the title compound as a white solid (7.5 mg, yield: 13%). ¹H NMR (400 MHz, DMSO-d6) δ 11.49 (s, 2H), 7.85 (d, J = 8.7 Hz, 1H), 7.82 (s, 1H), 7.57 - 7.47 (m, 5H), 7.47 - 7.39 (m, 1H); LC-MS: m/z [M+H]⁺ = 296.

### Example 66 8-chloro-7-(4-fluorophenyl)-1,3-dihydro-2H-imidazo[4,5-b]quinolin-2-one

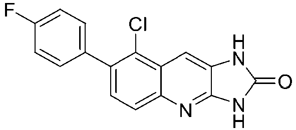

The title compound was synthesized by replacing phenylboronic acid with 4-fluorophenylboronic acid using a method similar to that in Example 65. ¹H NMR (400 MHz, DMSO-d6) δ 11.78 (s, 1H), 11.27 (s, 1H), 7.85 (d, J = 8.6 Hz, 1H), 7.82 (s, 1H), 7.59 - 7.49 (m, 3H), 7.33 (t, J = 8.9 Hz, 2H); LC-MS: m/z [M+H]⁺ = 314.

### Example 67 8-chloro-7-(4-(difluoromethy)phenyl)-1,3-dihydro-2H-imidazo[4,5-b]quinolin-2-one

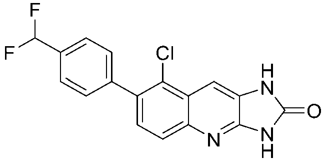

The title compound was synthesized by replacing phenylboronic acid with 4-(difluoromethyl)phenylboronic acid (CAS: 946525-43-5) using a method similar to that in Example 65. ¹H NMR (400 MHz, DMSO-d6) δ 11.84 (s, 1H), 11.33 (s, 1H), 7.88 (d, J = 8.6 Hz, 1H), 7.83 (s, 1H), 7.71 (d, J = 8.1 Hz, 2H), 7.66 (d, J = 8.2 Hz, 2H), 7.55 (d, J = 8.6 Hz, 1H), 7.13 (t, J = 55.9 Hz, 1H); LC-MS: m/z [M+H]⁺ = 346.

### Example 68 8-fluoro-7-(p-tolyl)-1,3-dihydro-2H-imidazo[4,5-b]quinolin-2-one

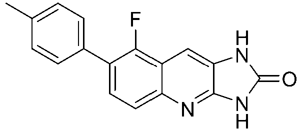

The title compound was synthesized by replacing 7-bromo-8-chloro-1,3-bis((2-(trimethylsilyl)ethoxy)methyl)-1,3-dihydro-2H-imidazo[4,5-b]quinolin-2-one (intermediate 1a) with 7-bromo-8-fluoro-1,3-bis((2-(trimethylsilyl)ethoxy)methyl)-1,3-dihydro-2H-imidazo[4,5-b]quinolin-2-one (intermediate 20) using a method similar to that in Example 65. ¹H NMR (400 MHz, DMSO-d6) δ 11.72 (s, 1H), 11.22 (s, 1H), 7.71 (d, J = 8.8 Hz, 1H), 7.66 - 7.59 (m, 2H), 7.58 - 7.51 (m, 2H), 7.33 (d, J = 7.9 Hz, 2H), 2.38 (s, 3H); LC-MS: m/z [M+H]⁺ = 294.

### Example 69 6-chloro-7-(p-tolyl)-1,3-dihydro-2H-imidazo[4,5-b]quinolin-2-one

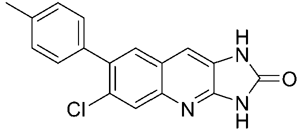

The title compound was synthesized by replacing 7-bromo-8-chloro-1,3-bis((2-(trimethylsilyl)ethoxy)methyl)-1,3-dihydro-2H-imidazo[4,5-b]quinolin-2-one (intermediate 1a) with 7-bromo-6-chloro-1,3-bis((2-(trimethylsilyl)ethoxy)methyl)-1,3-dihydro-2H-imidazo[4,5-b]quinolin-2-one (intermediate 21) using a method similar to that in Example 65. ¹H NMR (400 MHz, DMSO-d6) δ 11.58 (s, 1H), 11.21 (s, 1H), 7.92 (d, J = 4.5 Hz, 2H), 7.66 (s, 1H), 7.39 (d, J = 7.8 Hz, 2H), 7.30 (d, J = 7.9 Hz, 2H), 2.38 (s, 3H); LC-MS: m/z [M+H]⁺ = 310.

### Example 70 8-chloro-7-(5-methylpyridin-2-yl)-1,3-dihydro-2H-imidazo[4,5-b]quinolin-2-one

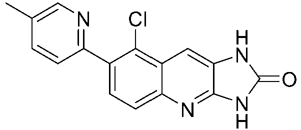

The title compound was synthesized by replacing phenylboronic acid with 5-methylpyridin-2-boronic acid (CAS: 372963-49-0) using a method similar to that in Example 65. ¹H NMR (400 MHz, DMSO-d6) δ 11.24 (s, 2H), 8.56 (s, 1H), 7.87 (d, J = 8.8 Hz, 1H), 7.84 (s, 1H), 7.78 - 7.72 (m, 1H), 7.70 - 7.62 (m, 2H), 2.39 (s, 3H); LC-MS: m/z [M+H]⁺ = 311.

### Example 71 8-chloro-7-(5-fluoropyrimidin-2-yl)-1,3-dihydro-2H-imidazo[4,5-b]quinolin-2-one

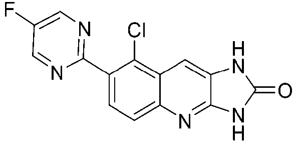

The title compound was synthesized by replacing 2-bromo-5-fluoropyridine (CAS: 41404-58-4) with 2-chloro-5-fluoropyrimidine (CAS: 62802-42-0) using a method similar to that in Example 24. ¹H NMR (400 MHz, DMSO-d6) δ 11.47 (s, 2H), 9.10 (s, 2H), 7.99 - 7.84 (m, 2H), 7.79 (d, J = 8.7 Hz, 1H); LC-MS: m/z [M+H]⁺ = 316.

### Example 72 8-chloro-7-(6-methoxypyridin-3-yl)-1,3-dihydro-2H-imidazo[4,5-b]quinolin-2-one

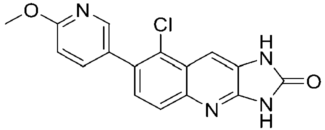

The title compound was synthesized by replacing 4-methyphenylboronic acid with 2-methoxy-5-pyridineboronic acid (CAS: 163105-89-3) using a method similar to that in Example 1. ¹H NMR (400 MHz, DMSO-d6) δ 11.55 (br.s, 2H), 8.30 (d, J = 2.3 Hz, 1H), 7.93 - 7.84 (m, 2H), 7.81 (s, 1H), 7.56 (d, J = 8.6 Hz, 1H), 6.96 (d, J = 8.6 Hz, 1H), 3.92 (s, 3H); LC-MS: m/z [M+H]⁺ = 327.

### Example 73 8-chloro-7-(6-chloropyridazin-3-yl)-1,3-dihydro-2H-imidazo[4,5-b]quinolin-2-one

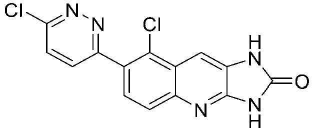

The title compound was synthesized by replacing 2-bromo-5-fluoropyridine (CAS: 41404-58-4) with 3-chloro-6-iodopyridazine (CAS: 135034-10- 5) using a method similar to that in Example 24. ¹H NMR (400 MHz, DMSO-d6) δ 11.50 (br.s, 2H), 8.19 (d, J = 8.9 Hz, 1H), 8.10 (d, J = 8.9 Hz, 1H), 7.96 (d, J = 8.5 Hz, 1H), 7.86 (s, 1H), 7.76 (d, J = 8.7 Hz, 1H); LC-MS: m/z [M+H]⁺ = 332.

### Example 74 8-chloro-7-(5-(difluoromethyl)pyridin-2-yl)-1,3-dihydro-2H-imidazo[4,5-b]quinolin-2-one

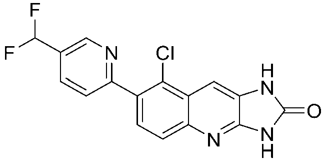

The title compound was synthesized by replacing 2-bromo-5-fluoropyridine (CAS: 41404-58-4) with 2-bromo-5-(difluoromethyl)pyridine (CAS: 1221272-81-6) using a method similar to that in Example 24. ¹H NMR (400 MHz, DMSO-d6) δ 11.84 (s, 1H), 11.30 (s, 1H), 8.94 (s, 1H), 8.16 (d, J = 7.9 Hz, 1H), 7.91 (t, J = 8.4 Hz, 2H), 7.85 (s, 1H), 7.71 (d, J = 8.6 Hz, 1H), 7.26 (t, J = 55.3 Hz, 1H); LC-MS: m/z [M+H]⁺ = 347.

### Example 75 8-chloro-7-(4-cyanophenyl)-1,3-dihydro-2H-imidazo[4,5-b]quinolin-2-one

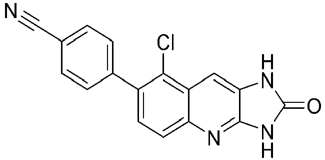

The title compound was synthesized by replacing 2-bromo-5-fluoropyridine (CAS: 41404-58-4) with 4-iodocyanobenzene (CAS: 3058-39-7) using a method similar to that in Example 24. ¹H NMR (400 MHz, DMSO-d6) δ 11.73 (s, 1H), 11.37 (s, 1H), 7.98 (d, J = 8.3 Hz, 2H), 7.89 (d, J = 8.8 Hz, 1H), 7.82 (s, 1H), 7.73 (d, J = 8.3 Hz, 2H), 7.55 (d, J = 8.6 Hz, 1H); LC-MS: m/z [M+H]⁺ = 321.

### Example 76 8-chloro-7-(2-azaspiro[3.3]heptan-2-yl)-1,3-dihydro-2H-imidazo[4,5-b]quinolin-2-one

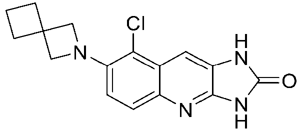

### 1) Synthesis of 8-chloro-7-(2-azaspiro[3.3]heptan-2-yl)-1,3-bis((2-(trimethylsilyl)ethoxy)methyl)-1,3-dihydro-2H-imidazo[4,5-b]quinolin-2-one

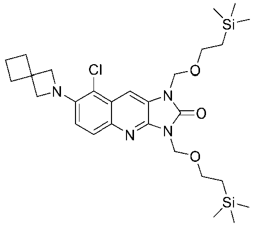

In a nitrogen atmosphere, 2-dicyclohexylphosphino-2',6'-diisopropoxy-1,1'-biphenyl (50 mg, 0.11 mmol, CAS: 787618-22-8) and methanesulfonato(2-dicyclohexylphosphino-2',4',6'-tri-isopropyl-1,1'-biphenyl)(2'-amino-1,1'-biphenyl-2-yl) palladium (II) (50 mg, 0.06 mmol, CAS: 1599466-85-9) were added into a solution of 7-bromo-8-chloro-1,3-bis((2-(trimethylsilyl)ethoxy)methyl)-1,3-dihydro-2H-imidazo[4,5-b]quinolin-2-one (200 mg, 0.36 mmol, intermediate 1a), cesium carbonate (300 mg, 0.92 mmol), and 2-azaspiro[3.3]heptane hemioxalate (56 mg, 0.20 mmol, CAS: 1365639-13-9) in tertiary butanol (5 mL), and the mixture was allowed to react at 90°C for 3 hours. The reaction solution was cooled to room temperature, diluted with water (20 mL), and extracted twice with ethyl acetate (20 mL). The organic phases were combined, washed twice with water (20 mL), washed twice with brine (20 mL), dried over anhydrous sodium sulfate, concentrated under reduced pressure, and separated by column chromatography (petroleum ether/ethyl acetate = 5:1) to obtain the title compound as a white solid (110 mg, yield: 43%). LC-MS: m/z [M+H]⁺ = 575.

### 2) 8-chloro-7-(2-azaspiro[3.3]heptan-2-yl)-1,3-dihydro-2H-imidazo[4,5-b]quinolin-2-one

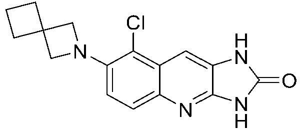

8-chloro-7-(2-azaspiro[3.3]heptan-2-yl)-1,3-bis((2-(trimethylsilyl)ethoxy)methyl)-1,3-dihydro-2H-imidazo[4,5-b]quinolin-2-one (100 mg, 0.17 mmol) was dissolved in trifluoroacetic acid (2 mL), trifluoromethanesulfonic acid (0.4 mL) was added, and the mixture was allowed to react at 20°C for 1 hour. The reaction solution was directly concentrated, the residue was dissolved in dichloromethane (2 mL), a methanol solution (2 mL), and ammonia water (1 mL), and the reaction mixture was allowed to react at 20°C for 1 hour. The reaction solution was directly concentrated and purified by preparative liquid chromatography (acetonitrile and water containing 0.1% ammonium bicarbonate) to obtain the title compound as a white solid (14.2 mg, yield: 26%). ¹H NMR (400 MHz, DMSO-d6) δ 11.46 (s, 1H), 11.05 (s, 1H), 7.64 (d, J = 8.9 Hz, 1H), 7.57 (s, 1H), 6.93 (d, J = 9.1 Hz, 1H), 4.07 (s, 4H), 2.19 (t, J = 7.6 Hz, 4H), 1.89 - 1.78 (m, 2H); LC-MS: m/z [M+H]⁺ = 315.

### Example 77 8-(difluoromethoxy)-7-(4-fluorophenyl)-1,3-dihydro-2H-imidazo[4,5-b]quinolin-2-one

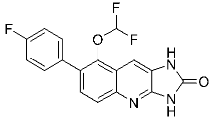

### 1) Synthesis of (6-amino-3-bromo-2-methoxyphenyl)methanol

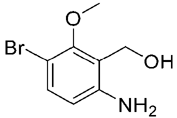

Lithium borohydride (0.59 g, 26.91 mmol) was added into a solution of methyl 6-amino-3-bromo-2-methoxybenzoate (3.5 g, 13.46 mmol, intermediate 23) in tetrahydrofuran (50 mL), and the reaction mixture was heated to 50°C and stirred for 3 hours. The reaction solution was cooled to room temperature, treated with a saturated aqueous solution of ammonium chloride (200 mL) for quenching the reaction, and then extracted 3 times with ethyl acetate (100 mL). The organic phases were combined, washed with brine (50 mL), dried over anhydrous sodium sulfate, filtered, concentrated under reduced pressure, and purified by column chromatography (petroleum ether/ethyl acetate = 10:1-1:1) to obtain the title compound as a yellow solid (3.1 g, yield: 99%). LC-MS: m/z [M+H]⁺ = 232/234.

### 2) Synthesis of 3-amino-6-bromo-2-(hydroxymethyl)phenol

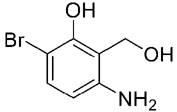

Anhydrous aluminum chloride (12.82 g, 96.17 mmol) was added into a solution of (6-amino-3-bromo-2-methoxyphenyl)methanol (2.79 g, 12.02 mmol) in dichloromethane (100 mL), and the mixture was subjected to nitrogen replacement 3 times and stirred at 25°C for 18 hours in a nitrogen atmosphere. The reaction solution was poured into a saturated aqueous solution of potassium tartrate (500 mL), and the mixture was stirred at room temperature for 2 hours and then extracted 3 times with ethyl acetate (300 mL). The organic phases were combined, washed with brine (50 mL), dried over anhydrous sodium sulfate, filtered, concentrated under reduced pressure, and purified by column chromatography (dichloromethane/methanol = 100:1-20:1) to obtain the title compound as a brownish-red solid (1585 mg, yield: 60.5%). LC-MS: m/z [M+H]⁺ = 218/220.

### 3) Synthesis of (6-amino-3-bromo-2-(difluoromethoxy)phenyl)methanol

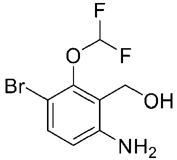

Bromodifluoromethane diethyl phosphate (3918.51 mg, 14.68 mmol, CAS: 65094-22-6) was added into a mixed solution of 3-amino-6-bromo-2-(hydroxymethyl)phenol (1600 mg, 7.34 mmol) and potassium hydroxide (8234.44 mg, 146.76 mmol) in acetonitrile (40 mL) and water (40 mL), and the mixture was stirred at 60°C for 5 hours. The reaction solution was poured into brine (100 mL) and extracted 3 times with ethyl acetate (150 mL). The organic phases were combined, dried over anhydrous sodium sulfate, filtered, concentrated under reduced pressure, and purified by column chromatography (petroleum ether/ethyl acetate = 10:1-2:1) to obtain the title compound as a yellow solid (520 mg, yield: 26%). LC-MS: m/z [M+H]⁺ = 268/270.

### 4) Synthesis of 6-amino-3-bromo-2-(difluoromethoxy)benzaldehyde

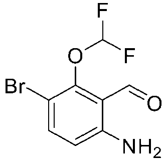

Active manganese dioxide (1362.19 mg, 15.67 mmol) was added into a solution of (6-amino-3-bromo-2-(difluoromethoxy)phenyl)methanol (840 mg, 3.13 mmol) in dichloromethane (30 mL), and the reaction mixture was stirred at 25°C for 18 hours. The reaction solution was filtered through Celite, the filter cake was washed 3 times with dichloromethane (30 mL), and the filtrate was concentrated under reduced pressure to obtain the title compound as a yellow solid (750 mg, 89.96%). LC-MS: m/z [M+H]⁺ = 266/268.

### 5) Synthesis of tert-butyl (2-amino-6-bromo-5-(difluoromethoxy)quinolin-3-yl)carbamate

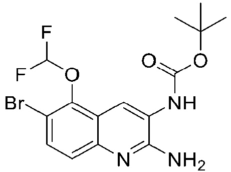

At 0°C, a 1 M solution of potassium tert-butoxide in tetrahydrofuran (5.56 mL, 5.56 mmol) was added into a solution of 6-amino-3-bromo-2-(difluoromethoxy)benzaldehyde (740 mg, 2.78 mmol) and N-bis(tert-butoxycarbonyl)-2-aminoacetonitrile (1425.82 mg, 5.56 mmol, intermediate 22) in tetrahydrofuran (30 mL), and the reaction mixture was stirred at 25°C for 18 hours. The reaction solution was poured into water (100 mL) and extracted 3 times with ethyl acetate (80 mL). The organic phases were combined, dried over anhydrous sodium sulfate, filtered, concentrated under reduced pressure, and purified by column chromatography (petroleum ether/ethyl acetate = 10:1-1:1) to obtain the title compound as a yellow solid (145 mg, yield: 13%). LC-MS: m/z [M+H]⁺ = 404/406.

### 6) Synthesis of tert-butyl (2-amino-5-(difluoromethoxy)-6-(4-fluorophenyl)quinolin-3-yl)carbamate

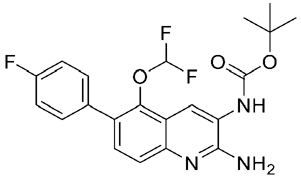

Tert-butyl (2-amino-6-bromo-5-(difluoromethoxy)quinolin-3-yl)carbamate (125 mg, 0.31 mmol), 4-fluorophenylboronic acid (43.27 mg, 0.31 mmol), cesium carbonate (201.52 mg, 0.62 mmol), and [1,1'-bis(di-tert-butylphosphino)ferrocene]dichloropalladium (40.43 mg, 0.06 mmol, CAS: 95408-45-0) were added into a mixed solvent of 1,4-dioxane (5 mL) and water (0.5 mL), nitrogen replacement was performed 3 times, and the mixture was stirred at 90°C for 18 hours in a nitrogen atmosphere. The reaction solution was filtered through Celite, and the filtrate was concentrated under reduced pressure and purified by column chromatography (petroleum ether/ethyl acetate = 10:1-1:1) to obtain the title compound as a yellow solid (55 mg, yield: 42%). LC-MS: m/z [M+H]⁺ = 420.

### 7) Synthesis of 8-(difluoromethoxy)-7-(4-fluorophenyl)-1,3-dihydro-2H-imidazo[4,5-b]quinolin-2-one

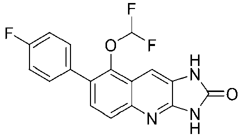

Tert-butyl (2-amino-5-(difluoromethoxy)-6-(4-fluorophenyl)quinolin-3-yl)carbamate (45 mg, 0.11 mmol) was added into a 1 M solution of potassium tert-butoxide in tetrahydrofuran (321.89 µL, 0.32 mmol), and the reaction mixture was stirred at 25°C for 18 hours. The reaction solution was purified by preparative liquid chromatography to obtain the title compound as a white solid (12.8 mg, yield: 34.6%). ¹H NMR (400 MHz, DMSO-d6) δ 11.75 (s, 1H), 11.20 (s, 1H), 7.84 (d, J = 8.7 Hz, 1H), 7.73 - 7.53 (m, 4H), 7.33 (t, J = 8.9 Hz, 2H), 6.72 (t, J = 73.5 Hz, 1H); LC-MS: m/z [M+H]⁺ = 346.

### Example 78 8-(methoxy)-7-(4-fluorophenyl)-1,3-dihydro-2H-imidazo[4,5-b]quinolin-2-one

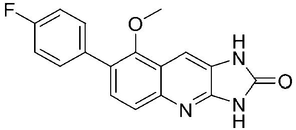

The title compound was synthesized by adopting methyl 6-amino-3-bromo-2-methoxybenzoate (intermediate 23) as a starting material using a method similar to that in Example 77. ¹H NMR (400 MHz, DMSO-d6) δ 11.60 (s, 1H), 11.08 (s, 1H), 7.79 - 7.60 (m, 4H), 7.51 (d, J = 8.4 Hz, 1H), 7.35 - 7.30 (m, 2H), 3.50 (s, 3H); LC-MS: m/z [M+H]⁺ = 310.

### Example 79 7-(p-tolyl)-8-(trifluoromethyl)-1,3-dihydro-2H-imidazo[4,5-b]quinolin-2-one

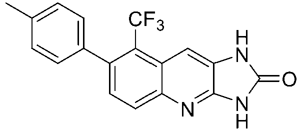

The title compound was synthesized by replacing 7-bromo-8-chloro-1,3-dihydro-2H-imidazo[4,5-b]quinolin-2-one (intermediate 1) with 7-bromo-8-(trifluoromethyl)-1,3-dihydro-2H-imidazo[4,5-b]quinolin-2-one (intermediate 25) using a method similar to that in Example 1. ¹H NMR (400 MHz, DMSO-d6) δ 11.82 (s, 1H), 11.20 (s, 1H), 8.05 (d, J = 8.4 Hz, 1H), 7.70 (d, J = 2.0 Hz, 1H), 7.39 (d, J = 8.8 Hz, 1H), 7.29 - 7.21 (m, 4H), 2.38 (s, 3H); LC-MS: m/z [M+H]⁺ = 344.

### Example 80 7-phenyl-8-(trifluoromethyl)-1,3-dihydro-2H-imidazo[4,5-b]quinolin-2-one

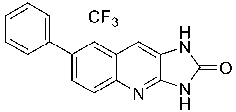

The title compound was synthesized by replacing 7-bromo-8-chloro-1,3-dihydro-2H-imidazo[4,5-b]quinolin-2-one (intermediate 1) with 7-bromo-8-(trifluoromethyl)-1,3-dihydro-2H-imidazo[4,5-b]quinolin-2-one (intermediate 25) and replacing 4-methylphenylboronic acid with phenylboronic acid using a method similar to that in Example 1. ¹H NMR (400 MHz, DMSO-d6) δ 11.83 (s, 1H), 11.22 (s, 1H), 8.07 (d, J = 8.4 Hz, 1H), 7.71 (d, J = 2.0 Hz, 1H), 7.50 - 7.33 (m, 6H); LC-MS: m/z [M+H]⁺ = 330.

### Example 81 8-chloro-7-(6-azaspiro[2.5]octan-6-yl)-1,3-dihydro-2H-imidazo[4,5-b]quinolin-2-one

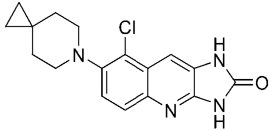

### Example 82 8-chloro-7-(4-ethyl-3,6-dihydropyridin-1(2H)-yl)-1,3-dihydro-2H-imidazo[4,5-b]quinolin-2-one

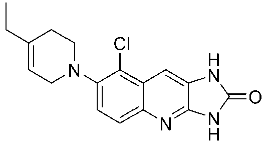

The 2 title compounds were synthesized by replacing 2-azaspiro[3.3]heptane hemioxalate (CAS: 1365639-13-9) with 6-azaspiro[2.5]octane hydrochloride (CAS: 1037834-62-0) using a method similar to that in Example 76, wherein the compound in Example 82 was a by-product generated during preparation of the compound in Example 81. Example 81: ¹H NMR (400 MHz, DMSO-d6) δ 11.60 (s, 1H), 11.15 (s, 1H), 7.76 (d, J = 9.0 Hz, 1H), 7.71 (s, 1H), 7.49 (d, J = 9.0 Hz, 1H), 3.06 (t, J = 5.3 Hz, 4H), 1.54 (s, 4H), 0.36 (s, 4H); LC-MS: m/z [M+H]⁺ =329. Example 82: ¹H NMR (400 MHz, DMSO-d6) δ 11.60 (s, 1H), 11.15 (s, 1H), 7.76 (d, J = 9.0 Hz, 1H), 7.70 (s, 1H), 7.49 (d, J = 9.1 Hz, 1H), 5.52 (dt, J = 3.6, 2.0 Hz, 1H), 3.66 - 3.54 (m, 2H), 3.20 (t, J = 5.5 Hz, 2H), 2.19 (d, J = 7.0 Hz, 2H), 2.04 (q, J = 8.1, 7.5 Hz, 2H), 1.03 (t, J = 7.4 Hz, 3H); LC-MS: m/z [M+H]⁺ = 329.

### Example 83 8-chloro-7-(2-chloro-4-fluorophenyl)-1,3-dihydro-2H-imidazo[4,5-b]quinolin-2-one

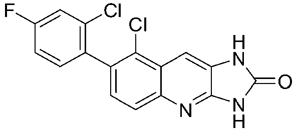

The title compound was synthesized by replacing 4-methyphenylboronic acid with 2-chloro-4-fluorophenylboronic acid (CAS: 313545-72-1) using a method similar to that in Example 1. ¹H NMR (400 MHz, DMSO-d6) δ 11.70 (s, 1H), 11.34 (s, 1H), 7.86 (d, J = 8.7 Hz, 1H), 7.78 (s, 1H), 7.64 (d, J = 8.8 Hz, 1H), 7.53 - 7.47 (m, 1H), 7.44 (d, J = 8.4 Hz, 1H), 7.36 (t, J = 7.8 Hz, 1H); LC-MS: m/z [M+H]⁺ = 348.

### Example 84 7-(4-fluorophenyl)-8-(trifluoromethyl)-1,3-dihydro-2H-imidazo[4,5-b]quinolin-2-one

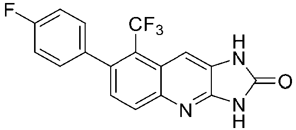

The title compound was synthesized by replacing 7-bromo-8-chloro-1,3-dihydro-2H-imidazo[4,5-b]quinolin-2-one (intermediate 1) with 7-bromo-8-(trifluoromethyl)-1,3-dihydro-2H-imidazo[4,5-b]quinolin-2-one (intermediate 25) and replacing 4-methylphenylboronic acid with p-fluorophenylboronic acid using a method similar to that in Example 1. ¹H NMR (400 MHz, DMSO-d6) δ 11.83 (s, 1H), 11.22 (s, 1H), 8.07 (d, J = 8.4 Hz, 1H), 7.71 (d, J = 2.0 Hz, 1H), 7.45 - 7.25 (m, 5H); LC-MS: m/z [M+H]⁺ = 348.

### Example 85 7-(2-chloro-4-fluorophenyl)-8-methoxy-1,3-dihydro-2H-imidazo[4,5-b]quinolin-2-one

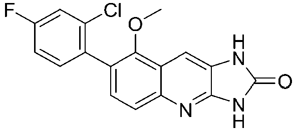

The title compound was synthesized by adopting methyl 6-amino-3-bromo-2-methoxybenzoate (intermediate 23) as a starting material and replacing 4-fluorophenylboronic acid with 2-chloro-4-fluorophenylboronic acid (CAS: 313545-72-1) using a method similar to that in Example 77. ¹H NMR (400 MHz, DMSO-d6) δ 11.65 (s, 1H), 11.13 (s, 1H), 7.69 - 7.59 (m, 3H), 7.53 (dd, J = 8.6, 6.3 Hz, 1H), 7.40 - 7.26 (m, 2H), 3.49 (s, 3H); LC-MS: m/z [M+H]⁺ = 344.

### Example 86 8-chloro-7-(5-chloro-1-(difluoromethyl)-1H-pyrazol-4-yl)-1,3-dihydro-2H-imidazo[4,5-b]quinolin-2-one

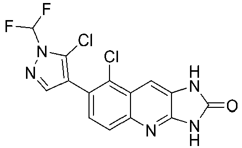

### Example 87 8-chloro-7-(3-chloro-1-(difluoromethyl)-1H-pyrazol-4-yl)-1,3-dihydro-2H-imidazo[4,5-b]quinolin-2-one

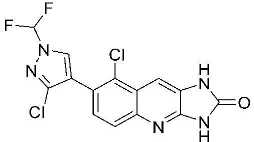

The title compounds were synthesized by replacing 2-bromo-5-fluoropyridine (CAS: 41404-58-4) with a mixture of 3-chloro-1-(difluoromethyl)-4-iodo-1H-pyrazole and 5-chloro-1-(difluoromethyl)-4-iodo-1H-pyrazole (intermediate 6) using a method similar to that in Example 24, and finally purified by a chiral column, respectively. Example 86: ¹H NMR (400 MHz, DMSO-d6) δ 11.82 (s, 1H), 11.31 (s, 1H), 8.19 (s, 1H), 8.04 (t, J = 56.8 Hz, 1H), 7.88 (d, J = 8.8 Hz, 1H), 7.80 (s, 1H), 7.58 (d, J = 8.8 Hz, 1H); LC-MS: m/z [M+H]⁺ =370. Example 87: ¹H NMR (400 MHz, DMSO-d6) δ 11.81 (s, 1H), 11.31 (s, 1H), 8.65 (s, 1H), 8.07 - 7.68 (m, 3H), 7.57 (d, J = 8.8 Hz, 1H); LC-MS: m/z [M+H]⁺ = 370.

### Example 88 6-chloro-7-(5-chloro-1-(difluoromethyl)-1H-pyrazol-4-yl)-1,3-dihydro-2H-imidazo[4,5-b]quinolin-2-one

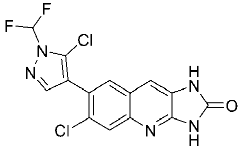

### Example 89 6-chloro-7-(3-chloro-1-(difluoromethyl)-1H-pyrazol-4-yl)-1,3-dihydro-2H-imidazo[4,5-b]quinolin-2-one

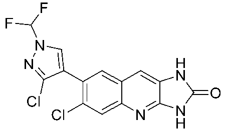

The title compounds were synthesized by replacing 8-chloro-7-(4,4,5,5-tetramethyl-1,3,2-dioxaboran-2-yl)-1,3-bis((2-(trimethylsilyl)ethoxy)methyl)-1,3-dihydro-2H-imidazo[4,5-b]quinolin-2-one (intermediate 9) with 6-chloro-7-(4,4,5,5-tetramethyl-1,3,2-dioxaboran-2-yl)-1,3-bis((2-(trimethylsilyl)ethoxy)methyl)-1,3-dihydro-2H-imidazo[4,5-b]quinolin-2-one (intermediate 26), and replacing 2-bromo-5-fluoropyridine (CAS: 41404-58-4) with a mixture of 3-chloro-1-(difluoromethyl)-4-iodo-1H-pyrazole and 5-chloro-1-(difluoromethyl)-4-iodo-1H-pyrazole (intermediate 6) using a method similar to that in Example 24, and finally obtained by purification with a chiral column, respectively. Example 88: ¹H NMR (400 MHz, DMSO-d6) δ 11.75 (s, 1H), 11.21 (s, 1H), 8.20 - 7.87 (m, 4H), 7.68 (s, 1H); LC-MS: m/z [M+H]⁺ =370. Example 89: ¹H NMR (400 MHz, DMSO-d6) δ 11.75 (s, 1H), 11.21 (s, 1H), 8.64 (s, 1H), 8.05 (s, 1H), 8.03 - 7.71 (m, 2H), 7.68 (s, 1H); LC-MS: m/z [M+H]⁺ = 370.

### Example 90 8-chloro-7-(3-(trifluoromethyl)-1H-pyrazol-1-yl)-1,3-dihydro-2H-imidazo[4,5-b]quinolin-2-one

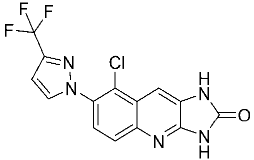

### 1) Synthesis of 8-chloro-7-(3-(trifluoromethyl)-1H-pyrazol-1-yl)-1,3-bis((2-(trimethylsilyl)ethoxy)methyl)-1,3-dihydro-2H-imidazo [4,5-b]quinolin-2-one

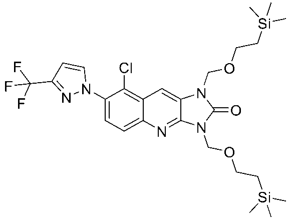

Cuprous oxide (25.21 mg, 0.17 mmol) was added into a solution of 8-chloro-7-iodo-1,3-bis((2-(trimethylmethylsilyl)ethoxy)methyl)-1,3-dihydro-2H-imidazo[4,5-b]quinolin-2-one (351 mg, 0.58 mmol, intermediate 28), 3-(trifluoromethyl)pyrazole (102.46 mg, 0.75 mmol, CAS: 20154-03-4), potassium phosphate (307.33 mg, 1.45 mmol), and N1,N2-bis(furan-2-ylmethyl)oxamide (43.13 mg, 0.17 mmol, CAS: 69010-90-8) in dimethyl sulfoxide (10 mL), and the mixture was subjected to nitrogen replacement 3 times and stirred at 130°C for 18 hours. The reaction solution was diluted with water (50 mL) and extracted 3 times with ethyl acetate (10 mL). The organic layers were combined, dried over anhydrous sodium sulfate, filtered, concentrated under reduced pressure, and separated by column chromatography (petroleum ether/ethyl acetate =3:1) to obtain the title compound as a yellow solid (172 mg, yield: 48%). LC-MS: m/z [M+H]⁺ = 614.

### 2) Synthesis of 8-chloro-7-(3-(trifluoromethyl)-1H-pyrazol-1-yl)-1,3-dihydro-2H-imidazo[4,5-b]quinolin-2-one

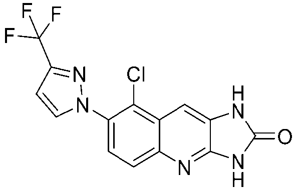

8-chloro-7-(3-(trifluoromethyl)-1H-pyrazol-1-yl)-1,3-bis((2-(trimethylsilyl)ethoxy)methyl)-1,3-dihydro-2H-imidazo[4,5-b]quinolin-2-one (152 mg, 0.25 mmol) was dissolved in trifluoroacetic acid (1 mL), trifluoromethanesulfonic acid (0.1 mL) was added, and the mixture was allowed to react at room temperature for 1 hour. The reaction solution was directly concentrated, the residue was dissolved in dichloromethane (10 mL) and ammonia water (1 mL), and the mixture was stirred at room temperature for 0.5 hour. The reaction solution was directly concentrated and purified by preparative liquid chromatography (36% to 66%(v/v) acetonitrile and water containing 0.1% formic acid) to obtain the title compound as a white solid (27.6 mg, yield: 31.5%). ¹H NMR (400 MHz, DMSO-d6) δ 11.96 (s, 1H), 11.42 (s, 1H), 8.47 (s, 1H), 7.95 (d, J = 8.8 Hz, 1H), 7.81 (s, 1H), 7.73 (d, J = 8.8 Hz, 1H), 7.07 (d, J = 2.4 Hz, 1H); LC-MS: m/z [M+H]⁺ = 354.

### Example 91 7-(6-methoxy-5-(trifluoromethyl)pyridin-3-yl)-1,3,5,6,7,8-hexahydro-2H-imidazo[4,5-b][1,6]naphthyridin-2-one

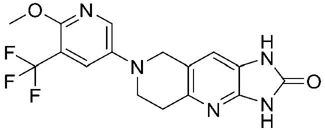

The title compound was synthesized by replacing bromobenzene with 2-methoxy-3-(trifluoromethyl)-5-bromopyridine (CAS: 1214377-42-0) using a method similar to that in Example 20. ¹H NMR (400 MHz, DMSO-d6) δ 11.16 (s, 1H), 10.74 (s, 1H), 8.17 (d, J = 2.6 Hz, 1H), 7.79 (d, J = 2.8 Hz, 1H), 7.07 (s, 1H), 4.37 (s, 2H), 3.90 (s, 3H), 3.59 (t, J = 5.9 Hz, 2H), 2.89 (t, J = 5.7 Hz, 2H); LC-MS: m/z [M+H]⁺ = 366.

### Example 92 5-chloro-6-(p-tolyl)-1,3-dihydro-2H-imidazo[4,5-b]quinoxalin-2-one

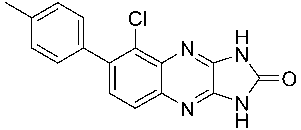

The title compound was synthesized by replacing 7-bromo-8-chloro-1,3-dihydro-2H-imidazo[4,5-b]quinolin-2-one (intermediate 1) with 6-bromo-5-chloro-1,3-dihydro-2H-imidazo[4,5-b]quinoxalin-2-one (intermediate 29) using a method similar to that in Example 1. ¹H NMR (400 MHz, DMSO-d6) δ 12.19 (s, 1H), 12.08 (s, 1H), 7.83 (d, J = 8.6 Hz, 1H), 7.52 (d, J = 8.5 Hz, 1H), 7.41 (d, J = 7.7 Hz, 2H), 7.31 (d, J = 7.7 Hz, 2H), 2.39 (s, 3H); LC-MS: m/z [M+H]⁺ = 311.

### Example 93 8-chloro-7-(4-fluorophenyl)-1-methyl-1,3-dihydro-2H-imidazo[4,5-b]quinolin-2-one

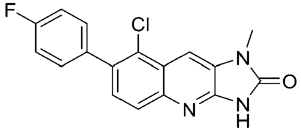

### 1) Synthesis of 8-chloro-7-(4-fluorophenyl)-1,3-bis((2-(trimethylsilyl)ethoxy)methyl)-1,3-dihydro-2H-imidazo[4,5-b] quinolin-2-one

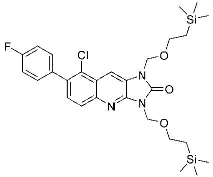

In a nitrogen atmosphere, [1,1'-bis(diphenylphosphino)ferrocene]dichloropalladium (60 mg, 0.08 mmol, CAS: 72287-26-4) was added into a mixed solution of 7-bromo-8-chloro-1,3-bis((2-(trimethylsilyl)ethoxy)methyl)-1,3-dihydro-2H-imidazo[4,5-b]quinolin-2-one (150 mg, 0.27 mmol, intermediate 1a), 4-fluorophenylboronic acid (40 mg, 0.27 mmol), potassium carbonate (150 mg, 1.08 mmol), water (0.4 mL), and 1,4-dioxane (4 mL), and the reaction mixture was allowed to react at 100°C for 3 hours. The reaction solution was filtered through Celite, and the filtrate was concentrated under reduced pressure and purified by column chromatography (petroleum ether/ethyl acetate =-10:1) to obtain the title compound as a white solid (110 mg, yield: 71%). LC-MS: m/z [M+H]⁺ = 574.

### 2) Synthesis of 8-chloro-7-(4-fluorophenyl)-3-((2-(trimethylsilyl)ethoxy)methyl)-1,3-dihydro-2H-imidazo[4,5-b]quinolin-2-one

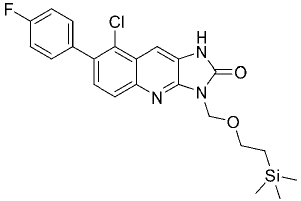

8-chloro-7-(4-fluorophenyl)-1,3-bis((2-(trimethylsilyl)ethoxy)methyl)-1,3-dihydro-2H-imidazo[4,5-b] quinolin-2-one (100 mg, 0.17 mmol) was added into a 1 N solution of tetrabutylammonium fluoride in tetrahydrofuran (2 mL), and the mixture was allowed to react at 80°C for 3 hours. The reaction solution was directly concentrated under reduced pressure and purified by column chromatography (petroleum ether/ethyl acetate = 5:1) to obtain the title compound as a yellow solid (6 mg, yield: 84%). LC-MS: m/z [M+H]⁺ = 444.

### 3) Synthesis of 8-chloro-7-(4-fluorophenyl)-1-methyl-3-((2-(trimethylsilyl)ethoxy)methyl)-1,3-dihydro-2H-imidazo[4,5-b] quinolin-2-one

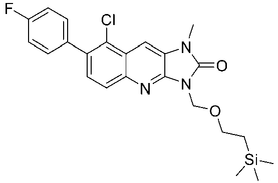

Iodomethane (50 mg, 0.35 mmol) was added into a solution of 8-chloro-7-(4-fluorophenyl)-3-((2-(trimethylsilyl)ethoxy)methyl)-1,3-dihydro-2H-imidazo[4,5-b]quinolin-2-one (65 mg, 0.15 mmol) and sodium hydride (50 mg, 1.25 mmol, 60%w/w in mineral oil) in tetrahydrofuran (3 mL), and the mixture was allowed to react at 20°C for 2 hours. The reaction solution was slowly poured into a saturated aqueous solution of ammonium chloride (15 mL) and extracted 2 times with ethyl acetate (10 mL). The organic phases were combined, concentrated under reduced pressure, and purified by column chromatography (petroleum ether/ethyl acetate = 6:1) to obtain the title compound as a yellow solid (60 mg, yield: 89%). LC-MS: m/z [M+H]⁺ = 458.

### 4) Synthesis of 8-chloro-7-(4-fluorophenyl)-1-methyl-1,3-dihydro-2H-imidazo[4,5-b]quinolin-2-one

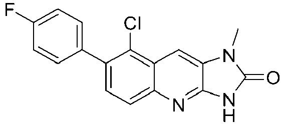

Trifluoromethanesulfonic acid (0.3 mL) was added into a mixed solution of 8-chloro-7-(4-fluorophenyl)-1-methyl-3-((2-(trimethylsilyl)ethoxy)methyl)-1,3-dihydro-2H-imidazo[4,5-b] quinolin-2-one (60 mg, 0.13 mmol) and trifluoroacetic acid (2 mL), and the mixture was allowed to react at 20°C for 1 hour. The reaction solution was directly concentrated under reduced pressure, then dichloromethane (1 mL), methanol (1 mL), and ammonia water (1 mL, 25.96 mmol) were added, and the mixture was allowed to react at 20°C for 1 hour. The mixture was concentrated under reduced pressure and purified by preparative liquid chromatography (acetonitrile and water containing 0.1% ammonium bicarbonate) to obtain the title compound as a white solid (1.3 mg, yield: 3%). ¹H NMR (400 MHz, DMSO-d6) δ 9.99 (s, 1H), 8.01 (s, 1H), 7.88 (d, *J =* 8.6 Hz, 1H), 7.56 (dd, *J =* 11.6, 6.4 Hz, 3H), 7.34 (t, J = 8.9 Hz, 2H), 3.43 (s, 3H); LC-MS: m/z [M+H]⁺ = 328.

### Example 94 8-chloro-7-(p-tolyl)oxazolo[4,5-b]quinolin-2(3H)-one

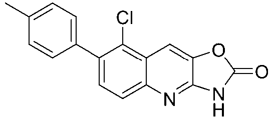

### 1) Synthesis of 2-(4-methoxybenzyl)oxy)acetonitrile

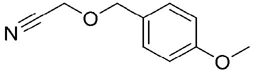

At -78°C and under nitrogen protection, a solution of 4-methoxybenzyl alcohol (4.50 mL, 36.19 mmol, CAS: 105-13-5, d = 1.113 g/mL) in tetrahydrofuran (10 mL) was added dropwise into a solution of sodium hydride (1.88 g, 47.04 mmol, 60% dispersed in mineral oil) in tetrahydrofuran (60 mL), the mixture was stirred at 0°C for 2 hours, then bromoacetonitrile (3.03 mL, 43.42 mmol, d = 1.722 g/mL) was added at 0°C, and the reaction mixture was heated to 25°C and stirred for 18 hours. A saturated aqueous solution of ammonium chloride (50 mL) was added into the reaction solution for quenching the reaction, the mixture was filtered through Celite, the filter cake was washed 3 times with ethyl acetate (30 mL), and the filtrate was extracted 3 times with ethyl acetate (30 mL). The organic phases were combined, washed 1 time with brine (10 mL), dried over anhydrous sodium sulfate, filtered, concentrated under reduced pressure, and purified by column chromatography (petroleum ether/ethyl acetate = 10:1-4:1) to obtain the title compound as a colorless oil (2.26 g, yield: 35%).

### 2) Synthesis of 6-bromo-5-chloro-3-((4-methoxybenzyl)oxy)quinolin-2-amine

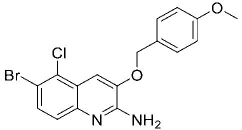

Potassium tert-butoxide (861.39 mg, 7.68 mmol) was slowly added into a solution of 2-(4-methoxybenzyl)oxy)acetonitrile (997.5 4mg, 5.63 mmol) and 6-amino-3-bromo-2-chlorobenzaldehyde (1200 mg, 5.12 mmol, synthesized according to a method provided in WO2019167000A1) in dimethyl sulfoxide (10 mL), and the mixture was subjected to nitrogen replacement 3 times and stirred at 25°C for 18 hours under nitrogen protection. The reaction solution was poured into water (100 mL) and extracted 3 times with ethyl acetate (50 mL). The organic phases were combined, washed 1 time with brine (20 mL), dried over anhydrous sodium sulfate, filtered, concentrated under reduced pressure, and purified by column chromatography (petroleum ether/ethyl acetate = 10:1-1:1) to obtain the title compound as a yellow solid (380 mg, yield: 19%). LC-MS: m/z [M+H]⁺ = 393/395.

### 3) Synthesis of 5-chloro-3-((4-methoxybenzyl)oxy)-6-(p-tolyl)quinolin-2-amine

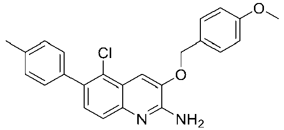

In a nitrogen atmosphere, [1,1'-bis(diphenylphosphino)ferrocene]dichloropalladium (92.01 mg, 0.13 mmol, CAS: 72287-26-4) was added into a mixed solution of 6-bromo-5-chloro-3-((4-methoxybenzyl)oxy)quinolin-2-amine (330 mg, 0.84 mmol), 4-methylphenylboronic acid (113.97 mg, 0.84 mmol), potassium carbonate (231.70 mg, 1.68 mmol), water (1 mL), and 1,4-dioxane (4 mL), and the reaction mixture was stirred at 100°C for 18 hours. The reaction solution was filtered through Celite, and the filtrate was concentrated under reduced pressure and purified by column chromatography (petroleum ether/ethyl acetate = 10:1-1:1) to obtain the title compound as a yellow solid (250 mg, yield: 74%). LC-MS: m/z [M+H]⁺ = 405.

### 4) Synthesis of 2-amino-5-chloro-6-(p-tolyl)quinolin-3-ol

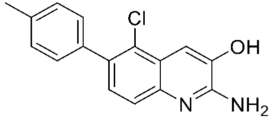

Trifluoroacetic acid (1 mL) was added into a solution of 5-chloro-3-((4-methoxybenzyl)oxy)-6-(p-tolyl)quinolin-2-amine (230 mg, 0.57 mmol) in dichloromethane (10 mL), and the mixture was stirred at 25°C for 18 hours. The reaction solution was directly concentrated to obtain the title compound (340 mg of a crude product), which was directly used in the next step. LC-MS: m/z [M+H]⁺ = 285.

### 5) Synthesis of 8-chloro-7-(p-tolyl)oxazolo[4,5-b]quinolin-2(3H)-one

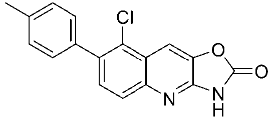

At room temperature, N,N'-carbonyldiimidazole (503.98 mg, 3.11 mmol) was added into a solution of 2-amino-5-chloro-6-(p-tolyl)quinolin-3-ol (295 mg, 1.04 mmol) and N,N-diisopropylethylamine (0.86 mL, 5.18 mmol, d = 0.742 g/mL) in tetrahydrofuran (10 mL), and the mixture was stirred at 70°C for 2 hours under nitrogen protection. The reaction solution was directly purified by preparative liquid chromatography (acetonitrile and water containing 0.1% formic acid) to obtain the title compound as a white solid (41 mg, yield: 13%). ¹H NMR (400 MHz, DMSO-d₆) δ 12.89 (s, 1H), 8.24 (s, 1H), 7.89 (d, J = 8.6 Hz, 1H), 7.62 (d, J = 8.6 Hz, 1H), 7.40 (d, J = 8.0 Hz, 2H), 7.32 (d, J = 7.9 Hz, 2H), 2.39 (s, 3H); LC-MS: m/z [M+H]⁺ = 311.

### Example 95 5-chloro-6-(p-tolyl)-1H-pyrrolo[2,3-b]quinolin-2,3-dione

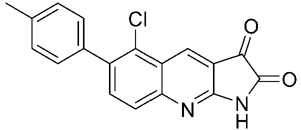

### 1) Synthesis of 5-chloro-1-(4-methoxybenzyl)-6-(p-tolyl)-1H-pyrrolo[2,3-b]quinolin-2,3-dione

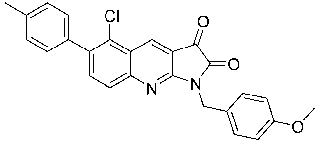

At room temperature, N-bromosuccinimide (45.65 mg, 0.26 mmol) was added into a solution of 5-chloro-1-(4-methoxybenzyl)-6-(p-tolyl)-1,3-dihydro-2H-pyrrolo[2,3-b]quinolin-2-one (110 mg, 0.26 mmol, a product of the third step in the preparation process of Example 2) in dimethyl sulfoxide (1 mL), and the mixture was stirred overnight at 100°C under nitrogen protection. The reaction solution was filtered, and the filtrate was purified by column chromatography using a C18 chromatographic column to obtain the title compound as a yellow solid (55 mg, yield: 48%). LC-MS: m/z [M+H]⁺ = 443.

### 2) Synthesis of 5-chloro-6-(p-tolyl)-1H-pyrrolo[2,3-b]quinolin-2,3-dione

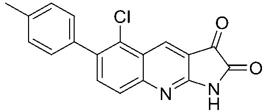

At room temperature, trifluoromethanesulfonic acid (0.11 mL) was added into a solution of 5-chloro-1-(4-methoxybenzyl)-6-(p-tolyl)-1H-pyrrolo[2,3-b]quinolin-2,3-dione (55 mg, 0.12 mmol) in trifluoroacetic acid (2 mL), and the mixture was stirred at 80°C for 1 hour. The reaction solution was concentrated under reduced pressure and purified by column chromatography using a C18 chromatographic column to obtain the title compound as a brown solid (4.9 mg, yield: 12%). ¹H NMR (400 MHz, DMSO-*d*₆) δ 12.04 (s, 1H), 8.66 (s, 1H), 7.88 - 7.76 (m, 2H), 7.41 (d, J = 8.0 Hz, 2H), 7.33 (d, J = 8.0 Hz, 2H), 2.40 (s, 3H); LC-MS: m/z [M+H]⁺ = 323.

### Example 96 7-(1-(difluoromethyl)-3-(methoxy-d3)-1H-pyrazol-4-yl)-1,3-dihydro-2H-imidazo[4,5-b]quinolin-2-one

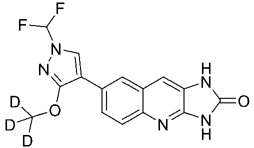

The title compound was synthesized by replacing 5-bromo-4-chloro-2-methoxypyridine (CAS: 851607-27-7) with 1-(difluoromethyl)-4-iodo-3-(methoxy-d3)-1H-pyrazole (intermediate 30) using a method similar to that in Example 8. ¹H NMR (400 MHz, DMSO-d6) δ 11.51 (s, 1H), 11.05 (s, 1H), 8.57 (s, 1H), 8.17 (d, J = 1.8 Hz, 1H), 7.84 - 7.78 (m, 2H), 7.78 - 7.51 (m, 2H); LC-MS: m/z [M+H]⁺ = 335.

### Biological activity test examples

### Test Example 1. SK-MEL-3 cell proliferation assay

1. Experimental objective: To determine inhibitory activities of compounds against proliferation of SK-MEL-3 cells.
2. Experimental materials:
   2.1 Drugs: CellTiter-Glo^{®} (Promega; G7573); McCoy's5A Medium (GIBCO; 16600-082); fetal bovine serum (ExCell Bio; FND500); and dimethyl sulfoxide (DMSO; Solarbio; D8371).
   2.2 Compounds: The compounds prepared in the Examples of the present application.
   2.3 Experimental instruments/materials: An incubator at 37°C (Thermo; HERA cell vios 160i); a clean bench (AIRTECH; VS-1300L-U); an inverted microscope (OPTEC; BDS400); a cell counter (Count star; IC1000); a microplate reader (Molecular Devices; SpectraMax Paradigm); and a 96-well plate (Biosahrp; BS-MP-96W).
3. Experimental method:
   The SK-MEL-3 cells were purchased from Nanjing Cobioer Biotechnology Co., Ltd. The cells were placed in a McCoys-5A(Gibco) culture medium (containing 10% fetal bovine serum; ExCell bio), into which 1% penicillin and streptomycin were added, and then cultured in the cell incubator at 37°C under 5% CO₂ and saturated humidity conditions.

After full growth, the cells were subjected to passage culture into the 96-well plate (5,000 cells/well). After culture overnight, the compounds at concentrations of 0.001 nM, 0.005 nM, 0.026 nM, 0.128 nM, 0.64 nM, 3.2 nM, 16 nM, 80 nM, 400 nM, and 2000 nM were added, respectively, where 0.1% DMSO and a culture medium were respectively set as a vehicle and a negative control, and 2 duplicate wells were set for each sample. The compounds were evenly mixed and then cultured in the incubator at 37°C under 5% CO₂ for 72 hours. After treatment, the 96-well plate was placed at room temperature for 30 minutes, 50 µL of Cell Titer-Glo^{®} (Promega) was added, and the cells were placed on a rotary oscillator for uniform shaking for 2 minutes and then placed in the dark for incubation at room temperature for 10 minutes. The 96-well plate was placed in the SpectraMax Paradigm (Molecular Devices) to test luminance signal values.

Cell activity inhibition rate (%)=100-(RLUcompound-RLUblank)/(RLUcontrol-RLUblank)*100%, wherein control refers to the 0.1 DMSO treatment group, and blank refers to the culture medium treatment group. IC₅₀ of the compounds was calculated and dose-response relationship curves were plotted by using the nonlinear regression function "Dose-Response-Inhibition" in Graphpad Prism 7.0.

### Test Example 2. A2058 cell proliferation assay

A2058 cells were purchased from Shanghai Fuheng Biology. The cells were resuspended in an RPMI160 culture medium containing 10% fetal bovine serum (Giboco) and then cultured in a cell incubator at 37°C under 5% CO₂ and saturated humidity conditions. When the A2058 cells reached a growth density of 80%-90%, the cells were subjected to digestion treatment and counted, and the counted cells were inoculated into a 96-well plate (2,000 cells/well). After 24 hours of cell culture, the compounds at concentrations of 2000 nM, 400 nM, 80 nM, 16 nM, 3.2 nM, 0.64 nM, 0.128 nM, 0.0256 nM, and 0.00512 nM were added, wherein the final concentration of DMSO was 0.2%, 2 duplicate wells were set for each sample, and 0.2% DMSO and a culture medium were set as controls. The compounds were evenly mixed and then placed in a cell incubator for culture for 96 hours. After treatment with the compounds, 50 µL of Cell Titer-Glo^{®} (Promega) was added into each well, and the cells were evenly shaken for 2 minutes and then placed in the dark for incubation at room temperature for 10 minutes. SpectraMax Paradigm (Molecular Devices) was used to test luminance signal values of the 96-well plate. Cell activity inhibition rate (%)=100-(RLUcompound-RLUblank)/(RLUcontrol-RLUblank)* 100%, wherein control refers to the 0.2% DMSO treatment group, and blank refers to the culture medium blank.

**Table 1: In vitro antiproliferative IC₅₀ values in SK-MEL-3 cells and A2058 cells in some Examples**

| **Example No.** | **Structure** | **SK-MEL-3 IC₅₀ (nM)** | **A2058 IC₅₀ (nM)** | **Example No.** | **Structure** | **SK-MEL-3 IC₅₀ (nM)** | **A2058 IC₅₀ (nM)** |
|---|---|---|---|---|---|---|---|
| **1** | 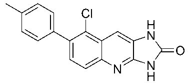 | 0.4 | - | **50** | 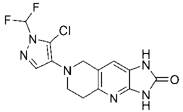 | 1.2 | 7.5 |
| **2** | 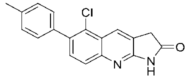 | 37.9 | - | **51** | 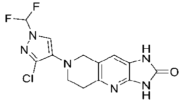 | 74.9 | - |
| **3** | 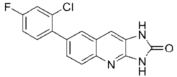 | 0.5 | 1.2 | **52** | 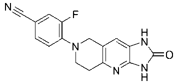 | 4.1 | - |
| **4** | 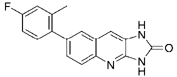 | 1.7 | - | **53** | 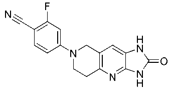 | 9.6 | - |
| **5** | 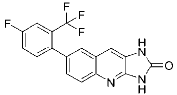 | 2.8 | - | **54** | 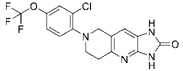 | 198.3 | - |
| **6** | 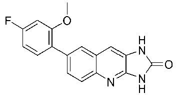 | 3.6 | - | **55** | 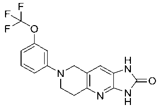 | 15.6 | - |
| **7** | 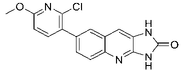 | 0.4 | - | **56** | 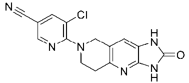 | 10.6 | - |
| **8** | 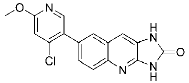 | 2.1 | - | **57** | 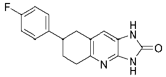 | - | 32.2 |
| **9** | 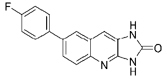 | 1.7 | - | **58** | 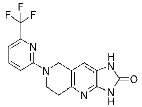 | 0.3 | 1.2 |
| **10** | 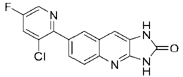 | 1.8 | - | **59** | 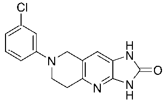 | 2.3 | - |
| **11** | 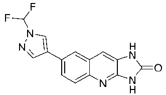 | 0.8 | - | **60** | 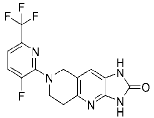 | - | 1.7 |
| **12** | 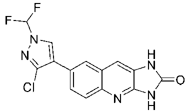 | 0.3 | - | **61** | 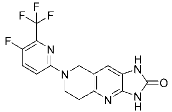 | - | 1.9 |
| **13** | 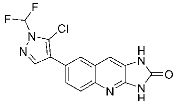 | 0.03 | 0.1 | **62** | 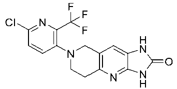 | - | 31.9 |
| **14** | 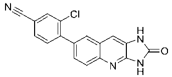 | 0.3 | 1.1 | **63** | 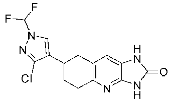 | - | 8.1 |
| **15** | 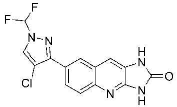 | 0.7 | 3.9 | **64** | 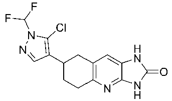 | 0.08 | 0.40 |
| **16** | 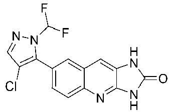 | 1.6 | - | **64-A** | 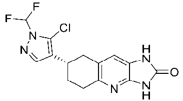 | 0.04 | 0.29 |
| **17** | 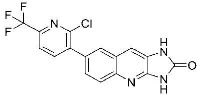 | 1.1 | 11.0 | **64-B** | 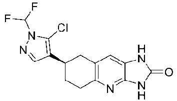 | 0.5 | 1.79 |
| **18** | 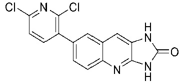 | 0.6 | - | **65** | 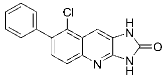 | 0.8 | - |
| **19** | 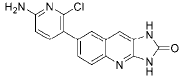 | 1.7 | - | **66** | 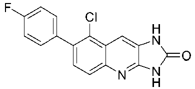 | 0.9 | - |
| **20** | 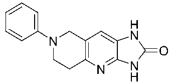 | 4.5 | - | **67** | 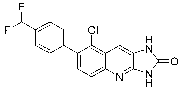 | 0.4 | - |
| **21** | 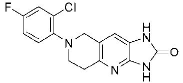 | 4.5 | 38.4 | **68** | 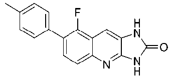 | 2.0 | - |
| **22** | 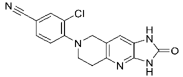 | 1.6 | 11.9 | **69** | 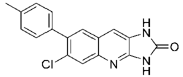 | 9.7 | - |
| **23** | 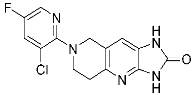 | 4.4 | 44.8 | **70** | 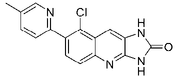 | 2.9 | - |
| **24** | 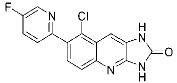 | 0.9 | 2.7 | **71** | 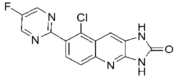 | 8.2 | - |
| **25** | 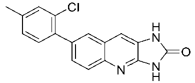 | 2.9 | - | **72** | 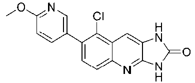 | 1.0 | - |
| **26** | 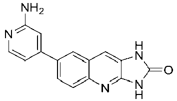 | 74.6 | - | **73** | 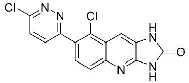 | 1.6 | - |
| **27** | 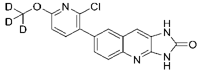 | 3.1 | 20.2 | **74** | 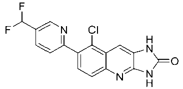 | 0.7 | - |
| **28** | 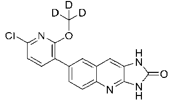 | 1.1 | 7.9 | **75** | 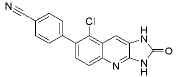 | 0.5 | - |
| **29** | 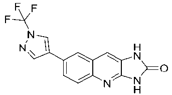 | 6.6 | - | **76** | 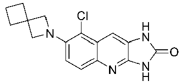 | 2.3 | - |
| **30** | 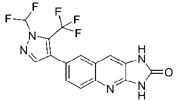 | 0.1 | 0.9 | **77** | 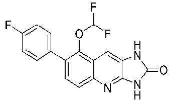 | 4.4 | - |
| **31** | 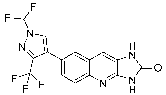 | 2.5 | - | **78** | 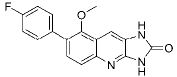 | 0.9 | - |
| **32** | 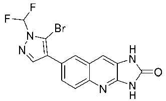 | 0.05 | 0.3 | **79** | 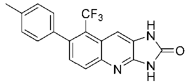 | 0.4 | - |
| **33** | 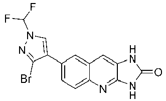 | - | - | **80** | 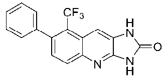 | 0.5 | - |
| **34** | 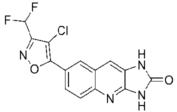 | 0.09 | 0.19 | **81** | 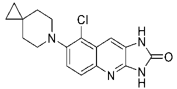 | 9.7 | - |
| **35** | 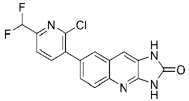 | 0.6 | 5.2 | **82** | 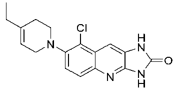 | 508.9 | - |
| **36** | 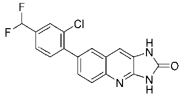 | 0.9 | 5.2 | **83** | 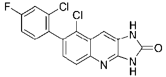 | 0.4 | - |
| **37** | 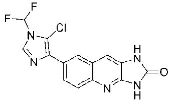 | - | 1.0 | **84** | 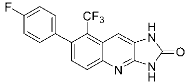 | 1.2 | - |
| **38** | 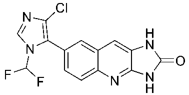 | 4.7 | - | **85** | 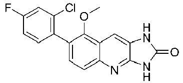 | 0.3 | - |
| **39** | 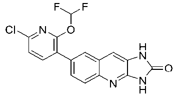 | - | 3.3 | **86** | 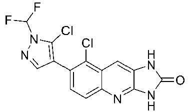 | 0.09 | 0.9 |
| **40** | 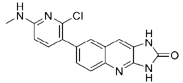 | - | 8.0 | **87** | 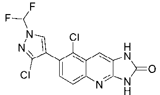 | - | 2.0 |
| **41** | 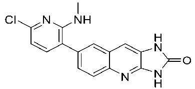 | - | 61.0 | **88** | 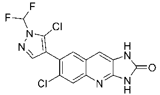 | - | 9.1 |
| **42** | 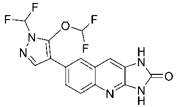 | - | 0.4 | **89** | 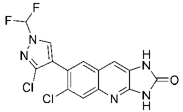 | - | 18.8 |
| **43** | 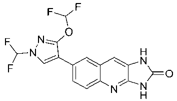 | - | - | **90** | 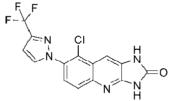 | 3.4 | - |
| **44** | 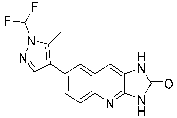 | 0.07 | 0.17 | **91** | 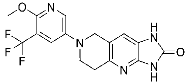 | 281.3 | - |
| **45** | 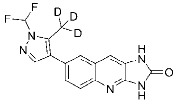 | - | 0.19 | **92** | 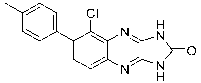 | 88.2 | - |
| **46** | 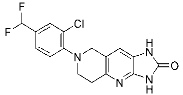 | 1.2 | - | **93** | 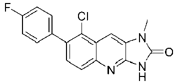 | 8.7 | - |
| **47** | 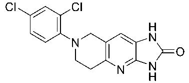 | 2.7 | - | **94** | 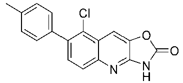 | 258.1 | - |
| **48** | 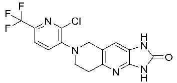 | 5.2 | 74.6 | **95** | 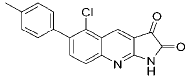 | 55.5 | - |
| **49** | 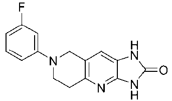 | 1.8 | - | **96** | 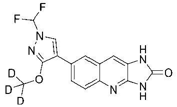 | - | - |

| | | | | | | | |
|---|---|---|---|---|---|---|---|
| Note: "-" indicates unmeasured. | | | | | | | |

### Test Example 3. PDE3A enzyme activity inhibition assay

The present invention used isotope-labeled [3H]cAMP as a substrate to test the inhibitory activities (IC50) of compounds against PDE3A. A stock solution of tritiated substrate cAMP was diluted 100 times with pure water to serve as a mother solution. Then, the substrate mother solution was diluted with an assay buffer (50 mM Tris. HCl, 10 mM MgCl₂, 0.5 mM DTT, pH of solution being 8.0) to achieve a cpm value of 20,000-30,000 on a liquid scintillation counter. 58 µL of the diluted substrate solution was added into a 1 mL EP tube, and 2 µL of a series of compound solutions at gradient concentrations (including 50 µM, 12.5 µM, 3.125 µM, 781.25 nM, 195.31 nM, 48.83 nM, 12.21 nM, 3.05 nM, 0.76 nM, and 0.19 nM) were slowly added and evenly mixed, wherein 2 µL of 0.1% DMSO was added in a control group. 40 µL of a PDE3A protein solution diluted to an appropriate concentration with an assay buffer was added, 40 µL of an assay buffer was added in a negative control group, and standing was allowed for a reaction at room temperature for 15 minutes. Then, 200 µL of 0.2 M ZnSO₄ and 200 µL of a 0.2 M BaOH₂ solution were sequentially added to terminate the reaction, and the mixture was evenly mixed by shaking and then centrifuged for 5 minutes. 430 µL of the supernatant was taken, transferred into a scintillation tube added with 2.5 mL of a scintillation solution and mixed thoroughly, and the radioactivity of the supernatant was measured with a liquid scintillation counter to determine the enzyme activity. Enzyme activity inhibition rate (%)=100-(RLUcompound-RLUblank)/(RLUcontrol-RLUblank)*100%, wherein control refers to the DMSO treatment group, and blank refers to the negative control group. IC₅₀ of the compounds was calculated and dose-response relationship curves were plotted by using the nonlinear regression function "Dose-Response-Inhibition" in Graphpad Prism 7.0

**Table 2: IC₅₀ values of enzyme activity inhibition against PDE3A**

| **Examp le No.** | **Structure** | **PDE3A IC50(nM )** | **Exampl e No.** | **Structure** | **PDE3A IC50(nM)** |
|---|---|---|---|---|---|
| **3** | 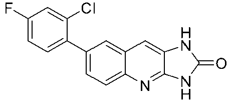 | 966.8 | **50** | 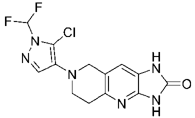 | 76.1 |
| **9** | 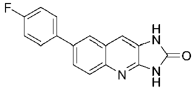 | 29.35 | **58** | 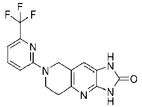 | 3.9 |
| **10** | 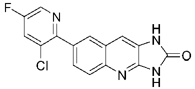 | 78.8 | **64-A** | 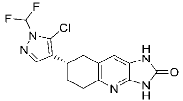 | 4.10 |
| **11** | 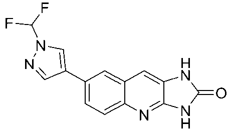 | 10.75 | **64-B** | 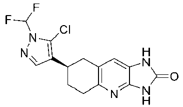 | 21.77 |
| **12** | 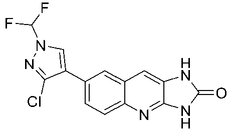 | 3.7 | **65** | 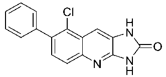 | 4.08 |
| **13** | 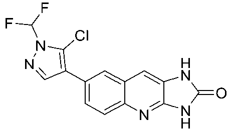 | 4.1 | **66** | 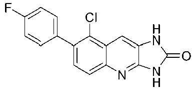 | 4.15 |
| **20** | 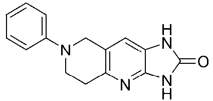 | 252.8 | **67** | 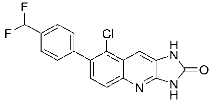 | 12.3 |
| **21** | 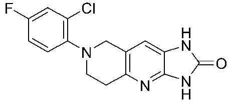 | 353 | **69** | 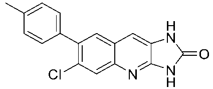 | 212.7 |
| **22** | 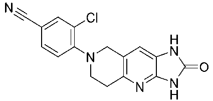 | 157.83 | **72** | 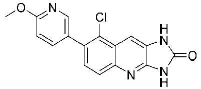 | 3.03 |
| **23** | 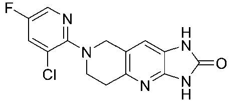 | 1125 | **74** | 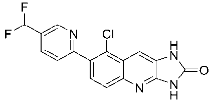 | 19.16 |
| **24** | 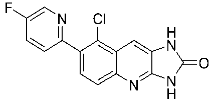 | 272.2 | **78** | 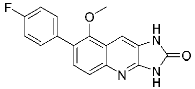 | 8.79 |
| **28** | 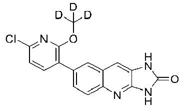 | 9.2 | **83** | 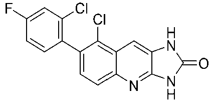 | 9.48 |
| **30** | 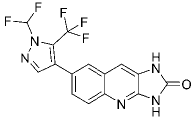 | 2.4 | **84** | 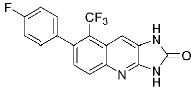 | 7.31 |
| **32** | 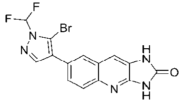 | 1.6 | **85** | 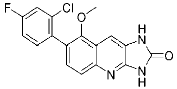 | 3.7 |
| **34** | 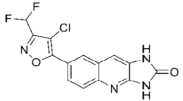 | 2.2 | **86** | 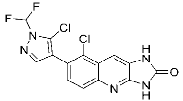 | 0.87 |
| **44** | 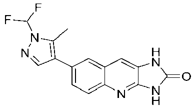 | 3.1 | | | |

It should be understood that the above detailed description and accompanying examples are only exemplary and should not be considered as limiting the scope of the present application, and the scope is defined only by the appended claims and equivalents thereof. Those skilled in the art can easily perceive various changes and modifications to the disclosed embodiments. Such changes and modifications may be made without departing from the spirit and the scope thereof, including but not limited to those related to the chemical structures, substituents, derivatives, intermediates, synthesis methods, preparations, and/or use methods of the present application. All publications, patents, and patent applications cited herein are incorporated herein by reference in their entirety for various purposes. These publications are provided only because they are disclosed earlier than the filing date of the present application. All declarations regarding the dates of these documents or statements of the contents of these documents are based on information available to the applicant and do not constitute any admission to the correctness of the dates of these documents or the contents of these documents. Moreover, any reference to these publications herein does not constitute an admission that the publications form a part of the common general knowledge in the art in any country.

## Claims

1. A compound of formula (I), a pharmaceutically acceptable salt thereof, a deuterated compound thereof, a stereoisomer thereof, a tautomer thereof, or a mixture thereof: 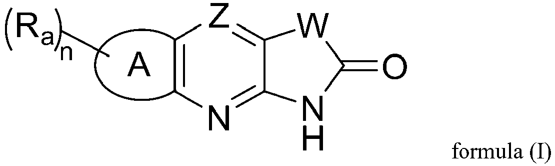 wherein
Z is selected from CR₁ or N;
W is selected from C=O, CR₂R₇, NR₃ or O;
R₁ is selected from H, halogen, hydroxyl, cyano, alkynyl, C₁-C₆ alkyl, C₁-C₆ haloalkyl, C₁-C₆ alkoxy, or C₃-C₆ cycloalkyl;
R₂ and R₇ are each independently selected from H, a deuterium atom, C₁-C₃ alkyl, or C₁-C₃ haloalkyl; or, R₂ and R₇, together with the carbon atoms to which they are attached, form C₃-C₆ cycloalkyl;
R₃ is selected from H, C₁-C₆ alkyl, C₁-C₆ haloalkyl, or C₃-C₆ cycloalkyl;
n is selected from any integer between 0-6;
Rₐ is each independently selected from H, halogen, cyano, alkynyl, optionally substituted C₁-C₆ alkoxy, C₁-C₆ haloalkyl, optionally substituted C₆-C₁₀ aryl, optionally substituted 5-10 membered heteroaryl, optionally substituted C₃-C₉ cycloalkyl, optionally substituted C₄-C₉ cycloalkenyl, or optionally substituted 3-10 membered heterocyclyl; and
ring A is selected from C₆-C₁₀ aryl, 5-10 membered heteroaryl, C₄-C₁₀ carbocyclyl, or 4-10 membered heterocyclyl.

2. The compound, the pharmaceutically acceptable salt thereof, the deuterated compound thereof, the stereoisomer thereof, the tautomer thereof, or the mixture thereof according to claim 1, wherein Rₐ is each independently selected from H, halogen, cyano, optionally substituted C₁-C₃ alkoxy, C₁-C₃ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl.

3. The compound, the pharmaceutically acceptable salt thereof, the deuterated compound thereof, the stereoisomer thereof, the tautomer thereof, or the mixture thereof according to claim 1 or 2, wherein the ring A is selected from a benzene ring, C₄-C₆ carbocyclyl, or 5-6 membered heterocyclyl; and
preferably, the ring A is selected from 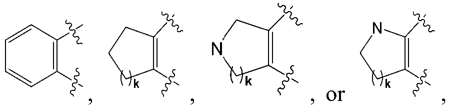 wherein k is selected from 1 or 2.

4. The compound, the pharmaceutically acceptable salt thereof, the deuterated compound thereof, the stereoisomer thereof, the tautomer thereof, or the mixture thereof according to any one of claims 1-3, wherein the compound has a structure represented by formula (II) or formula (II') or formula (II"): 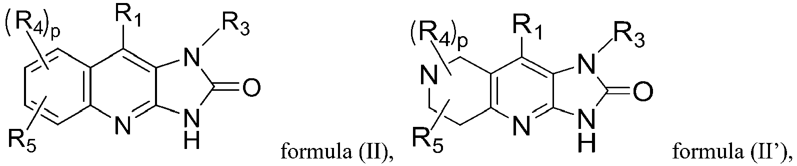 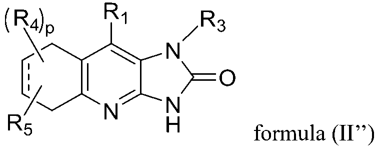 wherein
p is selected from 0, 1, 2, or 3;
R₁ is selected from H, halogen, hydroxyl, cyano, alkynyl, C₁-C₆ alkyl, C₁-C₆ haloalkyl, C₁-C₆ alkoxy, or C₃-C₆ cycloalkyl;
R₃ is selected from H, C₁-C₆ alkyl, C₁-C₆ haloalkyl, or C₃-C₆ cycloalkyl;
R₄ and R₅ are each independently selected from H, halogen, cyano, alkynyl, optionally substituted C₁-C₃ alkoxy, C₁-C₃ haloalkyl, optionally substituted C₆-C₁₀ aryl, optionally substituted 5-10 membered heteroaryl, optionally substituted C₃-C₆ cycloalkyl, optionally substituted C₄-C₆ cycloalkenyl, and optionally substituted 3-10 membered heterocyclyl; and 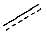 represents a single bond or a double bond.

5. The compound, the pharmaceutically acceptable salt thereof, the deuterated compound thereof, the stereoisomer thereof, the tautomer thereof, or the mixture thereof according to claim 4, wherein:
each R₄ is respectively and independently selected from H, halogen, cyano, C₁-C₃ haloalkyl, or optionally substituted C₁-C₃ alkoxy; and
R₅ is selected from optionally substituted C₆-C₁₀ aryl, optionally substituted 5-10 membered heteroaryl, cyano, optionally substituted C₃-C₆ cycloalkyl, and optionally substituted 3-10 membered heterocyclyl.

6. The compound, the pharmaceutically acceptable salt thereof, the deuterated compound thereof, the stereoisomer thereof, the tautomer thereof, or the mixture thereof according to claim 5, wherein
R₅ is selected from cyano, 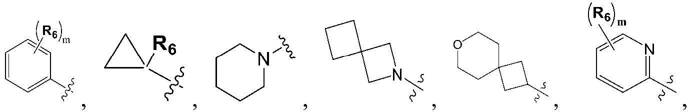 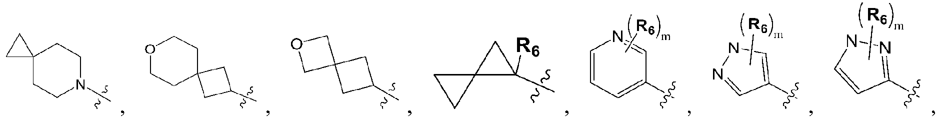 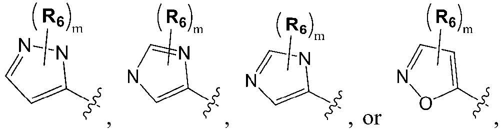 wherein m is selected from 0, 1, 2, or 3; R₆ is each independently selected from H, C₁-C₃ alkyl, C₁-C₃ haloalkyl, halogen, cyano, amino, or optionally substituted C₁-C₃ alkoxy; and the optionally substituted C₁-C₃ alkoxy is selected from -OCH₃, -OCD₃, -OCHD₂, -OCH₂D, ethoxy, -OCH₂F, -OCHF₂, -OCF₃, or -OCH₂CF₃.

7. The compound, the pharmaceutically acceptable salt thereof, the deuterated compound thereof, the stereoisomer thereof, the tautomer thereof, or the mixture thereof according to claim 1, wherein n is selected from any integer between 1-5, and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, optionally substituted C₃-C₆ cycloalkyl, or optionally substituted 3-10 membered heterocyclyl, wherein the optionally substituted C₁-C₆ alkoxy, aryl, heteroaryl, or heterocyclyl is unsubstituted or substituted with one or more substituents independently selected from deuterium, halogen, cyano, amino, C₁-C₆ alkyl, C₁-C₆ haloalkyl, C₃-C₆ cycloalkyl, 3-6 membered heterocyclyl, hydroxyl, C₁-C₆ alkoxy, C₁-C₆ haloalkoxy, C₁-C₆ alkylamino, C₆-C₁₀ aryl, 5-10 membered heteroaryl, and C₁-C₆ hydroxyalkyl;
preferably, n is selected from any integer between 1-3, and Rₐ is each independently selected from halogen, optionally substituted C₁-C₆ alkoxy, C₁-C₆ haloalkyl, optionally substituted C₆-C₁₀ aryl, optionally substituted 5-10 membered heteroaryl, or optionally substituted 3-10 membered heterocyclyl, wherein the optionally substituted C₁-C₆ alkoxy is unsubstituted or substituted with halogen, and the optionally substituted aryl, heteroaryl, or heterocyclyl is unsubstituted or substituted with one or more substituents independently selected from halogen, cyano, amino, C₁-C₆ alkyl, C₁-C₆ haloalkyl, C₁-C₆ alkoxy, C₁-C₆ haloalkoxy, and C₁-C₆ alkylamino; and
more preferably, n is 1 or 2, and Rₐ is each independently selected from F, Cl, Br, optionally substituted C₁-C₅ alkoxy, C₁-C₅ haloalkyl, optionally substituted C₆ aryl, optionally substituted 5-6 membered heteroaryl, or optionally substituted 6-8 membered heterocyclyl, wherein the optionally substituted C₁-C₆ alkoxy is unsubstituted or substituted with F, Cl, or Br, and the optionally substituted aryl, heteroaryl, or heterocyclyl is unsubstituted or substituted with one or more substituents independently selected from F, Cl, Br, cyano, amino, C₁-C₆ alkyl, C₁-C₆ haloalkyl, C₁-C₆ alkoxy, C₁-C₆ haloalkoxy, and C₁-C₆ alkylamino.

8. The compound, the pharmaceutically acceptable salt thereof, the deuterated compound thereof, the stereoisomer thereof, the tautomer thereof, or the mixture thereof according to claim 1 or 7, wherein the ring A is selected from C₆-C₁₀ aryl, 6-10 membered heteroaryl, C₅-C₈ carbocyclyl, or 5-8 membered heterocyclyl; and
preferably, the ring A is selected from C₆ aryl, 6-8 membered heteroaryl comprising 1-3 heteroatoms selected from N or O or S, C₅-C₇ carbocyclyl, or 5-7 membered heterocyclyl comprising 1-3 heteroatoms selected from N or O or S.

9. The compound, the pharmaceutically acceptable salt thereof, the deuterated compound thereof, the stereoisomer thereof, the tautomer thereof, or the mixture thereof according to any one of claims 1 and 7-8, wherein Z is selected from CR₁ or N, and R₁ is selected from H, halogen, hydroxyl, or C₁-C₃ alkyl; and
preferably, Z is selected from CH or N.

10. The compound, the pharmaceutically acceptable salt thereof, the deuterated compound thereof, the stereoisomer thereof, the tautomer thereof, or the mixture thereof according to any one of claims 1 and 7-9, wherein W is selected from C=O, CR₂R₇, NR₃ or O, R₂ and R₇ are each independently selected from H, a deuterium atom, or C₁-C₃ alkyl, and R₃ is selected from H, C₁-C₆ alkyl, or C₁-C₆ haloalkyl; and
preferably, W is selected from C=O, CH₂, NR₃ or O, and R₃ is selected from H or C₁-C₆ alkyl.

11. The compound, the pharmaceutically acceptable salt thereof, the deuterated compound thereof, the stereoisomer thereof, the tautomer thereof, or the mixture thereof according to claim 1, wherein the compound has a structure represented by formula (IV): 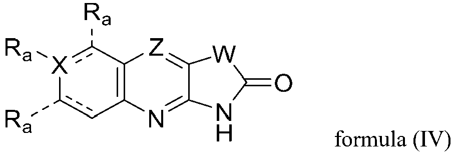 wherein X is selected from C, CH or N;
Rₐ is each independently selected from halogen, substituted or unsubstituted C₁-C₆ alkoxy, C₁-C₆ haloalkyl, substituted or unsubstituted C₆-C₁₀ aryl, substituted or unsubstituted 5-8 membered heteroaryl, and substituted or unsubstituted 5-10 membered heterocyclyl, wherein the C₁-C₆ alkoxy can be substituted with halogen, the aryl, heteroaryl, or heterocyclyl can be substituted with one or more substituents independently selected from deuterium, halogen, cyano, amino, C₁-C₆ alkyl, C₁-C₆ haloalkyl, C₁-C₆ alkoxy, C₁-C₆ haloalkoxy, and C₁-C₆ alkylamino, and the heteroaryl or heterocyclyl comprises 1-3 heteroatoms selected from N, O, or S;
Z is selected from CH or N, W is selected from C=O, CH₂, NR₃ or O, and R₃ is selected from H or C₁-C₆ alkyl;
represents a single bond or a double bond; and - - - represents that the bond is present or absent.

12. The compound, the pharmaceutically acceptable salt thereof, the deuterated compound thereof, the stereoisomer thereof, the tautomer thereof, or the mixture thereof according to claim 11, wherein the compound has a structure represented by formula (IV-1): 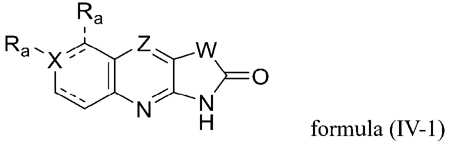 wherein X is C or CH;
Rₐ is selected from substituted or unsubstituted C₆ aryl, and substituted or unsubstituted 5-6 membered heteroaryl, wherein the aryl or heteroaryl can be substituted with 1-3 substituents independently selected from F, Cl, Br, C₁-C₆ alkyl, and C₁-C₆ haloalkyl, and the heteroaryl or heterocyclyl comprises 1-3 heteroatoms selected from N, O, or S;
Z is CH, and W is NH;
represents a single bond or a double bond; and - - - represents that the bond is present or absent.

13. The compound, the pharmaceutically acceptable salt thereof, the deuterated compound thereof, the stereoisomer thereof, the tautomer thereof, or the mixture thereof according to any one of claims 1-11, wherein the compound is selected from:

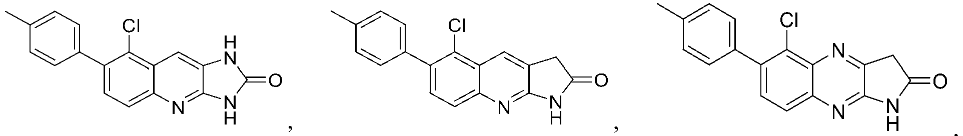

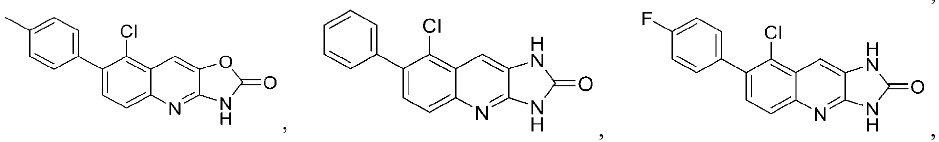

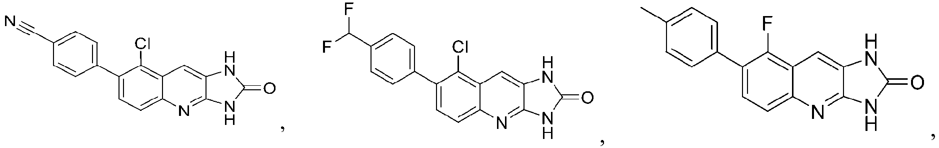

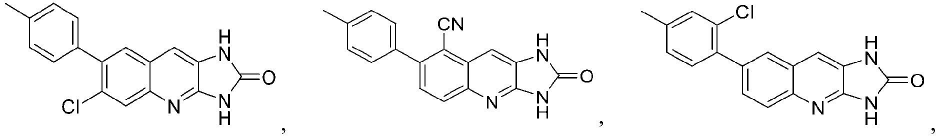

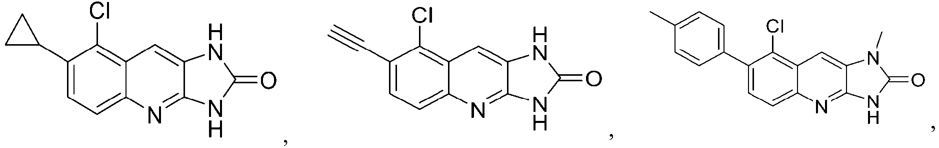

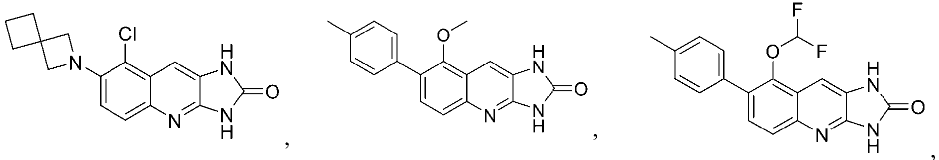

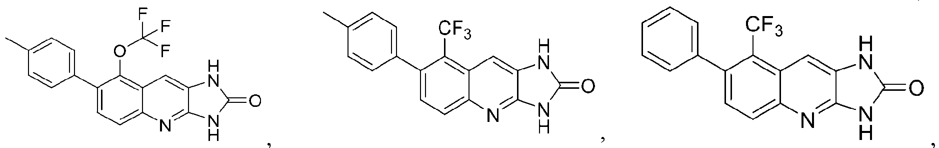

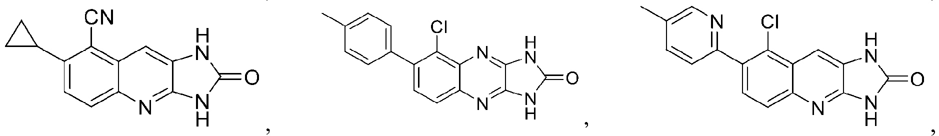

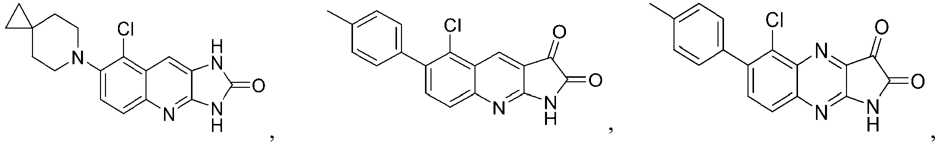

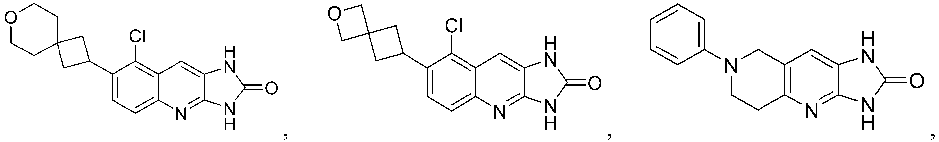

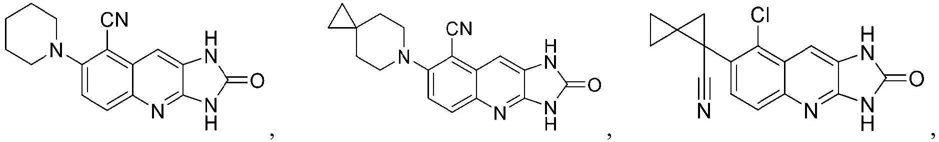

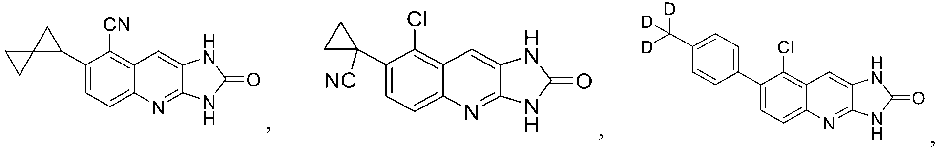

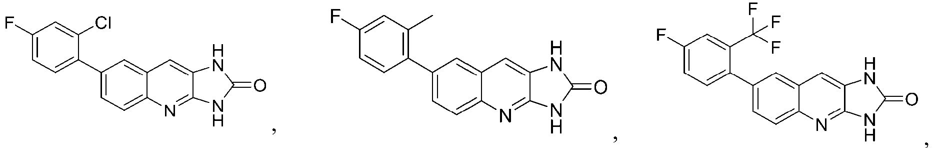

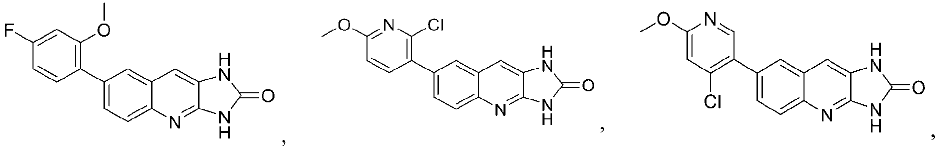

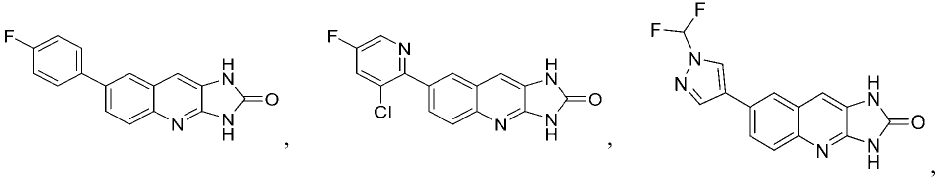

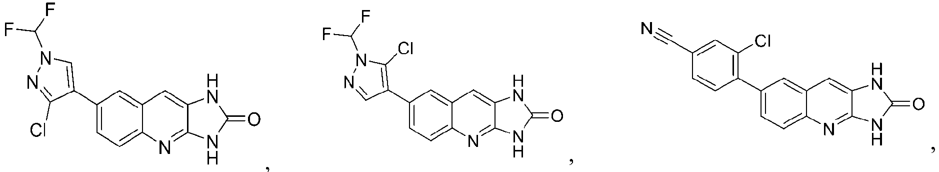

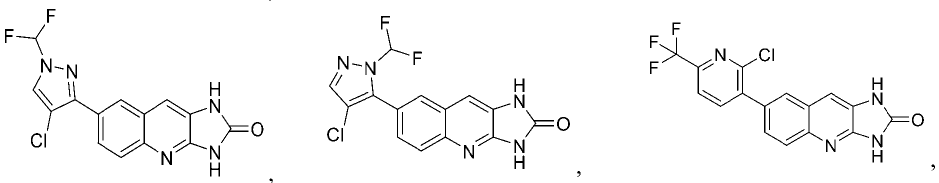

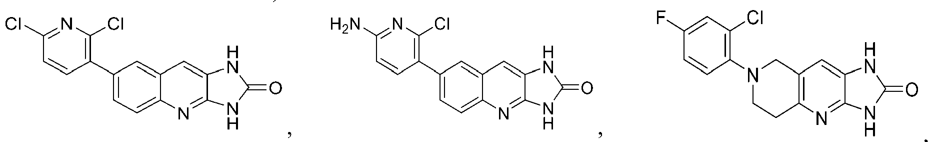

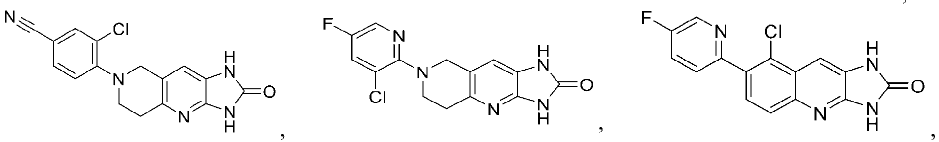

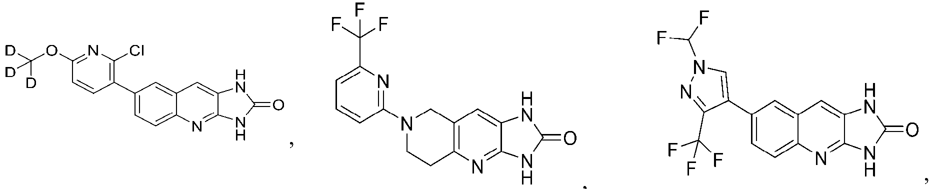

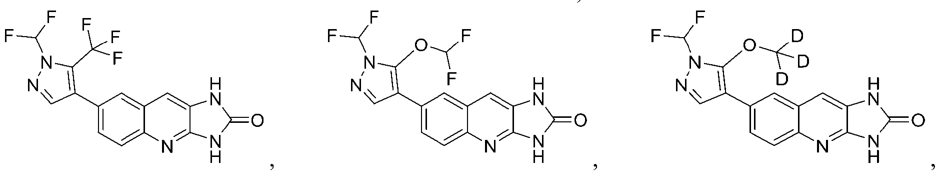

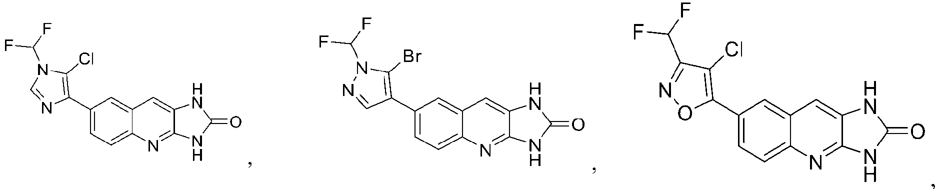

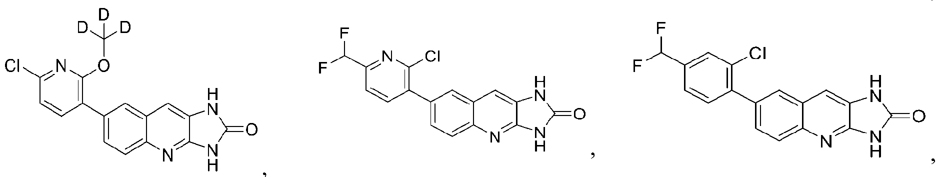

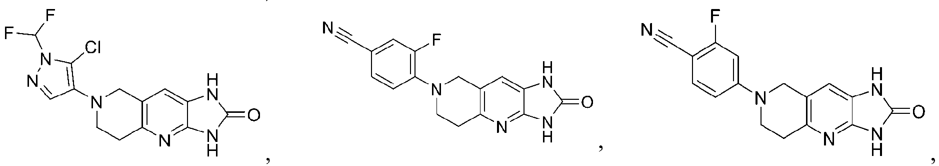

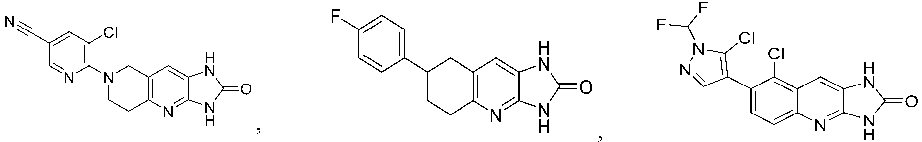

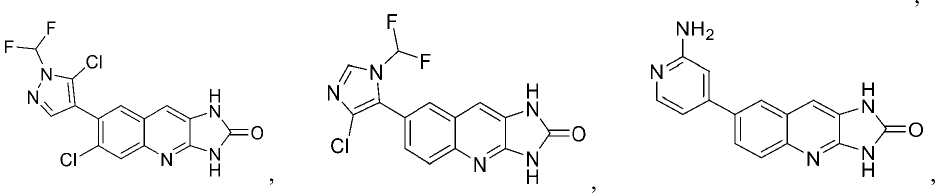

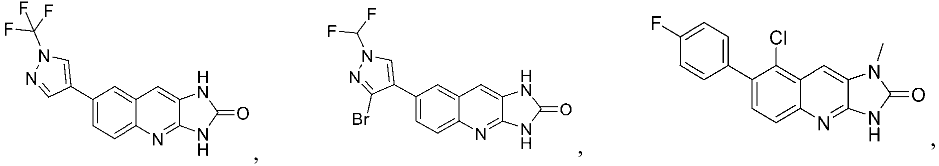

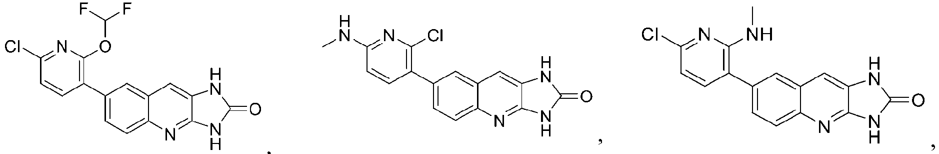

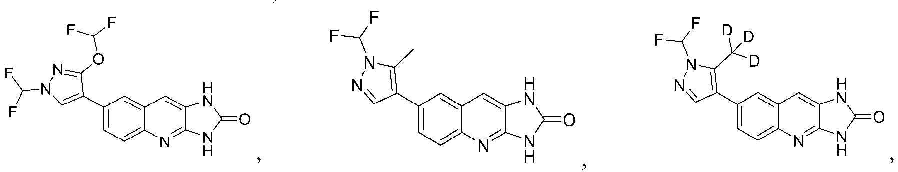

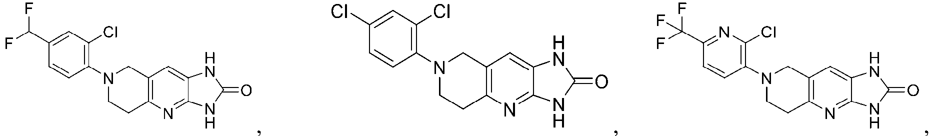

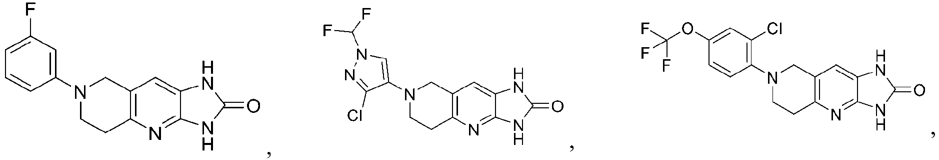

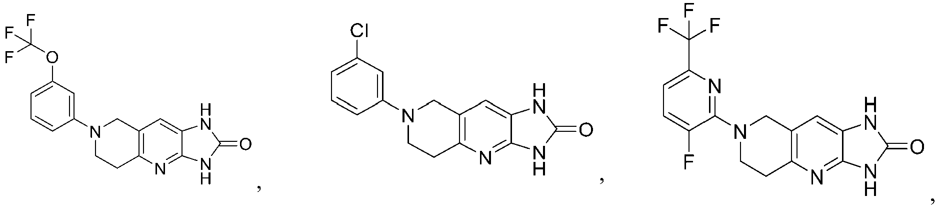

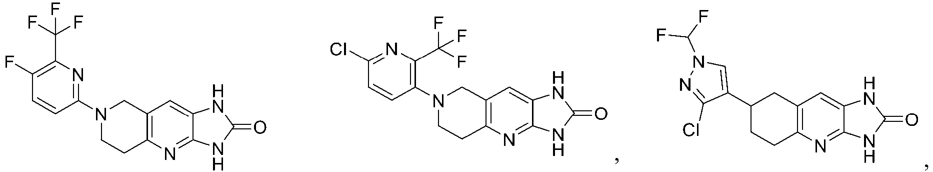

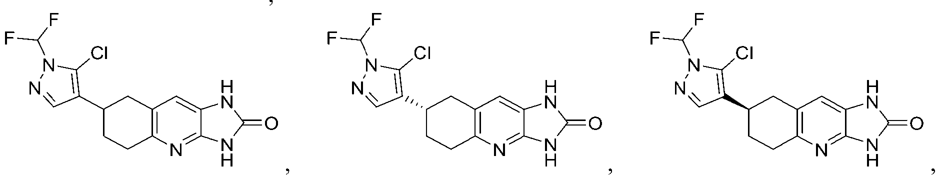

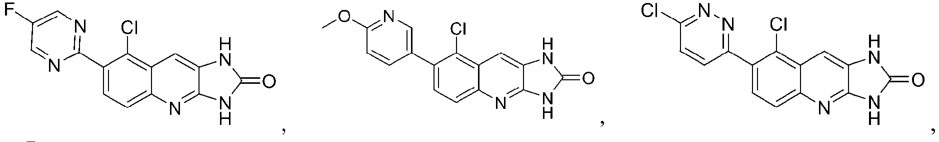

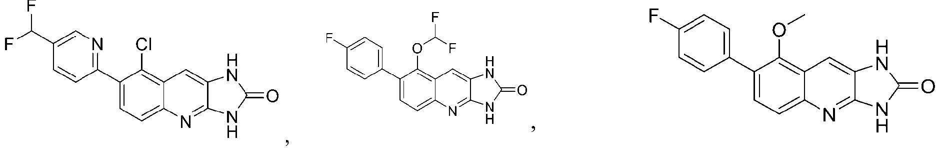

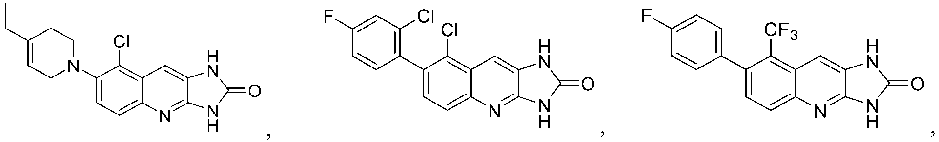

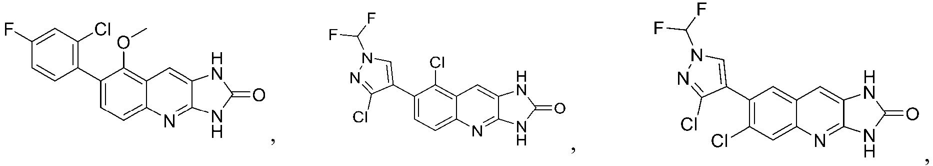

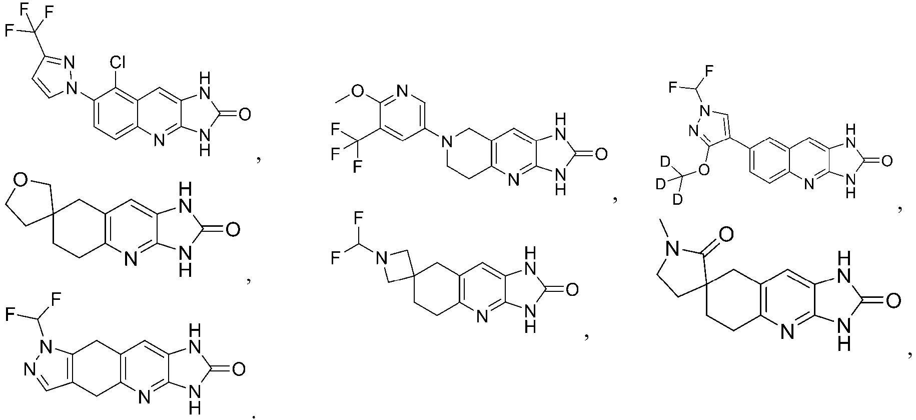

14. The compound, the pharmaceutically acceptable salt thereof, the deuterated compound thereof, the stereoisomer thereof, the tautomer thereof, or the mixture thereof according to any one of claims 1-13 for use as a drug for regulating the interaction between PDE3A and SLFN12, preferably, the drug is capable of enhancing and/or promoting the interaction between PDE3A and SLFN12.

15. The compound, the pharmaceutically acceptable salt thereof, the deuterated compound thereof, the stereoisomer thereof, the tautomer thereof, or the mixture thereof according to any one of claims 1-13 for use in treating and/or preventing a disease, wherein the disease is a hyperproliferative disease, preferably, the hyperproliferative disease is a cancer disease, and more preferably, the cancer disease comprises brain cancer, breast cancer, cervical cancer, acute myeloid leukemia (AML), lung cancer, skin cancer, esophageal cancer, ovarian cancer, pancreatic cancer, prostate cancer, and sarcoma.
